(19) Europäisches Patentamt
European Patent Office
Office européen des brevets

(11) Veröffentlichungsnummer: **0 267 147 B1**

# (12) EUROPÄISCHE PATENTSCHRIFT

(45) Veröffentlichungstag der Patentschrift: **07.08.91**

(51) Int. Cl.$^5$: **C07D 249/10**, A61K 31/41

(21) Anmeldenummer: **87810579.0**

(22) Anmeldetag: **07.10.87**

## (54) Aralkyl-4H-1,2,4-triazolderivate.

(30) Priorität: **09.10.86 CH 4035/86**

(43) Veröffentlichungstag der Anmeldung:
**11.05.88 Patentblatt 88/19**

(45) Bekanntmachung des Hinweises auf die Patenterteilung:
**07.08.91 Patentblatt 91/32**

(84) Benannte Vertragsstaaten:
**AT BE CH DE ES FR GB GR IT LI LU NL SE**

(56) Entgegenhaltungen:
**EP-A- 0 034 481**
**CH-A- 601 268**
**FR-A- 1 512 421**

(73) Patentinhaber: **CIBA-GEIGY AG**
**Klybeckstrasse 141**
**CH-4002 Basel(CH)**

(72) Erfinder: **Allgeier, Hans, Dr.**
**Lichsenweg 20**
**W-7850 Lörrach-Haagen(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft neue antikonvulsiv wirksame pharmazeutische Präparate, als Wirkstoff enthaltend ein Aralkyl-4H-1,2,4-triazol-derivat, insbesondere ein 5-Phenylniederalkyl-4H-1,2,4-triazol-3-carboxamid der Formel

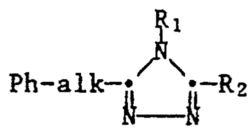

(I),

worin Ph durch Niederalkyl, Halogen und/oder Trifluormethyl substituiertes Phenyl bedeutet, alk Niederalkyliden darstellt, $R_1$ Wasserstoff oder Niederalkyl ist und $R_2$ für gegebenenfalls durch Niederalkyl oder Niederalkanoyl substituiertes Carbamyl steht, oder gegebenenfalls ein Tautomeres und/oder ein pharmazeutisch verwendbares Salz davon, die Verwendung der genannten Verbindungen der Formel I oder gegebenenfalls ihrer Tautomeren und/oder ihrer pharmazeutisch verwendbaren Salze zur Herstellung antikonvulsiv wirksamer pharmazeutischer Präparate, ein Verfahren zur Behandlung von Konvulsionen verschiedenartiger Genese, dadurch gekennzeichnet, dass man eine der genannten Verbindungen der Formel I oder gegebenenfalls ein Tautomeres und/oder ein pharmazeutisch verwendbares Salz davon verabreicht, sowie neue 5-Phenylniederalkyl-4H-1,2,4-triazol-3-carboxamide der Formel I, worin Ph durch Niederalkyl, Halogen und/oder Trifluormethyl substituiertes Phenyl bedeutet, alk Niederalkyliden darstellt, $R_1$ Wasserstoff oder Niederalkyl ist und $R_2$ für gegebenenfalls durch Niederalkyl oder Niederalkanoyl substituiertes Carbamyl steht, mit der Massgabe, dass in Verbindungen der Formel I, worin $R_1$ Methyl, $R_2$ N,N-Diäthylcarbamyl und alk Methylen bedeutet, Ph von in p-Stellung durch Chlor substiutiertem Phenyl verschieden ist, beispielsweise solche 5-Phenylniederalkyl-4H-1,2,4-triazol-3-carboxamide der Formel I, worin Ph von durch Halogen, namentlich durch Chlor, monosubstituiertem Phenyl verschieden ist, wenn $R_2$ N,N-Di-$C_1$-$C_4$-alkylcarbamyl, worin die beiden N-Alkylgruppen gleich oder verschieden sind, bedeutet, und jeweils ihre Salze sowie gegebenenfalls jeweils ihre Tautomeren und deren Salze sowie ein Verfahren zur Herstellung der letztgenannten, neuen Verbindungen der Formel I oder ihrer Tautomeren und/oder Salze.

Der Phenylrest Ph enthält beispielsweise bis und mit 3, insbesondere 1 oder 2, der genannten Substituenten, vorzugsweise 1 Niederalkyl-, Trifluormethyl- oder Halogensubstituenten oder 2 oder 3 Halogensubstituenten, ferner 1 Niederalkyl- und 1 Halogensubstituenten oder 1 Trifluormethyl- und 1 Halogensubstituenten, wobei vorzugsweise mindestens einer der Alkyl- und/oder Halogensubstituenten in einer o-Stellung oder ein Trifluormethylrest in einer m-Stellung gebunden ist. Als Beispiele seien genannt: o-Halogenphenyl, m-Trifluormethylphenyl, 2,6- und 2,5-, ferner 2,3- und 2,4-Dihalogenphenyl sowie o-Niederalkylphenyl.

Gegebenenfalls durch Niederalkyl oder Niederalkanoyl substituiertes Carbamyl $R_2$ ist beispielsweise Carbamyl oder in zweiter Linie N-Niederalkyl-, N,N-Diniederalkyl- oder N-Niederalkanoylcarbamyl.

Tautomere Formen von Verbindungen der Formel I können dann existieren, wenn $R_1$ Wasserstoff bedeutet. Die entsprechenden 4H-1,2,4-Triazol-Verbindungen der Formel

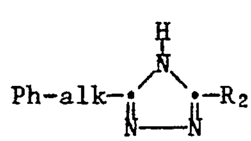

(Ia)

können dann im Gleichgewicht mit ihren 1H-1,2,4-Triazol-Tautomeren der Formeln

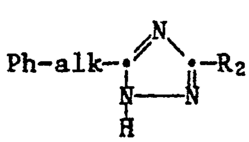

(Ib) und

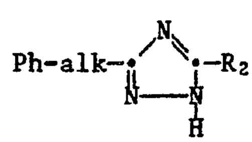

(Ic)

stehen, wobei entsprechende Verbindungen überwiegend, soweit bekannt, die Struktur Ia aufzuweisen

scheinen.

Die Verbindungen der Formel I und gegebenenfalls deren Tautomere können ferner in Form von Stereoisomeren vorliegen. Besitzt beispielsweise die Niederalkylidengruppe alk oder eine Niederalkylgruppe (als Substituent von Ph oder in einer substituierten Carbamylgruppe $R_2$) ein chirales Kohlenstoffatom (C-Atom), können die Verbindungen der Formel I als reine Enantiomere oder Enantiomerengemische, wie Racemate, oder, falls innerhalb der zuvor erwähnten Gruppen noch mindestens ein weiteres chirales Zentrum vorhanden ist, auch als Diastereomere, Diastereomerengemische oder Racematgemische vorliegen.

Salze von Verbindungen der Formel I oder gegebenenfalls deren Tautomeren sind insbesondere entsprechende Säureadditionssalze, vorzugsweise pharmazeutisch verwendbare Säureadditionssalze. Diese werden beispielsweise mit starken anorganischen Säuren, wie Mineralsäuren, z.B. Schwefelsäure, einer Phosphorsäure oder einen Halogenwasserstoffsäure, mit starken Carbonsäuren, wie Niederalkancarbonsäuren, z.B. Essigsäure, wie gegebenenfalls ungesättigten Dicarbonsäuren, z.B. Malon-, Malein- oder Fumarsäure, oder wie Hydroxycarbonsäuren, z.B. Wein- oder Zitronensäure, oder mit organischen Sulfonsäuren, wie Niederalkan- oder gegebenenfalls substituierten Benzolsulfonsäuren, z.B. Methan- oder p-Toluolsulfonsäure, gebildet. Umfasst sind ferner für pharmazeutische Verwendungen ungeeignete Salze, da diese beispielsweise für die Isolierung bzw. Reinigung freier Verbindungen der Formel I sowie gegebenenfalls ihrer Tautomeren und/oder pharmazeutisch verwendbaren Salze verwendet werden können.

Vor- und nachstehend sind unter mit "Nieder" bezeichneten organischen Gruppen und Verbindungen, sofern nicht abweichend definiert, vorzugsweise solche zu verstehen, die bis und mit 7, insbesondere bis und mit 4, C-Atome enthalten.

Niederalkyl ist beispielsweise $C_1$-$C_4$-Alkyl, d.h. Methyl, Aethyl, n-Propyl, Isopropyl, n-Butyl, Sekundärbutyl, Isobutyl oder Tertiärbutyl, kann aber ferner auch eine $C_5$-$C_7$-Alkylgruppe, d.h. Pentyl-, Hexyl- oder Heptylgruppe, sein.

Halogen ist Halogen mit einer Atomnummer bis und mit 35, d.h. Fluor, Chlor oder in zweiter Linie Brom.

Niederalkyliden ist $C_1$-$C_4$-Alkyliden, insbesondere 1,1-$C_1$-$C_4$-Alkyliden, wie Methylen oder Aethyliden, ferner 1,1-Propyliden oder 1,1-Butyliden, kann aber auch 2,2-$C_3$-$C_4$-Alkyliden, wie 2,2-Proyliden (Isopropyliden) oder 2,2-Butyliden sein.

N-Niederalkylcarbamyl ist N-$C_1$-$C_7$-Alkyl-, insbesondere N-$C_1$-$C_4$,-Alkylcarbamyl, wie N-Methyl-, N-Aethyl-, N-(n-Propyl)-, N-Isopropyl-, N-(n-Butyl)-, N-Isobutyl-, N-Sekundärbutyl-oder N-Tertiärbutylcarbamyl.

N,N-Diniederalkylcarbamyl ist beispielsweise N,N-Di-$C_1$-$C_4$-alkyl-carbamyl, wobei jeweils die beiden N-Alkylgruppen gleich oder verschieden sein können, wie N,N-Dimethyl-, N,N-Diäthyl-, N,N-Diisopropyl- oder N-Butyl-N-methyl-carbamyl.

N-Niederalkanoylcarbamyl ist beispielsweise N-$C_2$-$C_7$-Alkanoyl-, insbesondere N-$C_2$-$C_4$-Alkanoylcarbamyl, wie N-Acetyl-, N-Propionyl-oder N-Butyrylcarbamyl, kann aber auch N-Formylcarbamyl oder N-$C_5$-$C_7$-Alkanoylcarbamyl, wie N-Pivaloylcarbamyl, sein.

Die Verbindungen der Formel I und gegebenenfalls deren Tautomere und/oder ihre pharmazeutisch verwendbaren Salze besitzen wertvolle pharmakologische Eigenschaften, insbesondere eine ausgeprägte antikonvulsive Wirksamkeit, die sich beispielsweise an der Maus anhand eines deutlichen Metrazol-Antagonismus im Dosisbereich ab etwa 10 mg/kg p.o. sowie an Maus und Ratte anhand einer ausgeprägten Schutzwirkung gegen durch Elektroschock ausgelöste Konvulsionen im Dosisbereich ab etwa 4 mg/kg p.o. zeigen lässt. Die Verbindungen der Formel I und gegebenenfalls deren Tautomere und/oder ihre pharmazeutisch verwendbaren Salze sind dementsprechend vorzüglich geeignet zur Behandlung von Konvulsionen verschiedenartiger Genese, beispielsweise zur Behandlung der Epilepsie. Sie können dementsprechend als antikonvulsive, beispielsweise antiepileptische, Arzneimittelwirkstoffe verwendet werden. Dabei kann auch die gewerbsmässige Herrichtung der Wirksubstanzen eingeschlossen sein.

In der europäischen Patentanmeldung Nr. 34,481 werden unter anderem gegebenenfalls in 3-und/oder 4-Position durch Hydrocarbyl substituierte 4H-1,2,4-Triazol-5-carboxamidverbindungen mit peripher oder zentral beruhigender oder stimulierender Wirksamkeit generisch offenbart; jedoch werden dort keine konkreten Verbindungen, die unter den Umfang des vorliegenden Erfindungsgegenstandes fallen, erwähnt. In FR-1,512,421 werden unter anderem gegebenenfalls in 4-Position alkylsubstituierte 3-Halogenaralkyl-4H-1,2,4-triazol-5-carboxamidderivate mit Verwendbarkeit auf dem Gebiet der Photographie generisch offenbart; zudem wird dort bereits 3-(4-Chlorbenzyl)-4-methyl-4H-1,2,4-triazol-5-(N,N-diäthyl)carboxamid konkret offenbart. Diese in FR-1,512,421 konkret offenbarte Verbindung ist mittels Disclaimer aus dem Umfang des vorliegenden Erfindungsgegenstandes herausgenommen. Ansonsten stellt die vorliegende Erfindung teilweise eine Auswahl aus den Erfindungsgegenständen der beiden vorstehend erwähnten Offenbarungen dar.

Die Erfindung betrifft in erster Linie pharmazeutische, insbesondere antikonvulsiv wirksame, Präparate, die Herstellung derselben und ein Behandlungsverfahren, dadurch gekennzeichnet, dass man als Wirkstoff

eine Verbindung der Formel I, worin Ph durch Niederalkyl, Halogen und/oder Trifluormethyl mono-, di- oder trisubstituiertes Phenyl bedeutet, alk Niederalkyliden darstellt, $R_1$ Wasserstoff oder Niederalkyl ist und $R_2$ Carbamyl, N-Niederalkylcarbamyl, N,N-Diniederalkylcarbamyl oder N-Niederalkanoylcarbamyl bedeutet, vorzugsweise eine solche Verbindung der Formel I, worin Ph mindestens einen der Alkyl- und/oder Halogensubstituenten in einer o-Stellung oder einen Trifluormethylsubstituenten in einer m-Stellung trägt, oder gegebenenfalls ein Tautomeres und/oder ein pharmazeutisch verwendbares Salze davon wählt, sowie eine Verbindung der Formel I, worin Ph durch Niederalkyl, Halogen und/oder Trifluormethyl mono-, di- oder trisubstituiertes Phenyl bedeutet, alk Niederalkyliden darstellt, $R_1$ Wasserstoff oder Niederalkyl ist und $R_2$ Carbamyl, N-Niederalkylcarbamyl, N,N-Diniederalkylcarbamyl oder N-Niederalkanoylcarbamyl bedeutet, vorzugsweise eine solche Verbindung der Formel I, worin Ph mindestens einen der Alkyl- und/oder Halogensubstituenten in einer o-Stellung oder einen Trifluormethylsubstituenten in einer m-Stellung trägt, mit der Massgabe, dass in einer Verbindung der Formel I, worin $R_1$ Methyl, $R_2$ N,N-Diäthylcarbamyl und alk Methylen bedeutet, Ph von in p-Stellung durch Chlor substituiertem Phenyl verschieden ist, beispielsweise eine solche Verbindung der Formel I, worin Ph von durch Halogen, namentlich durch Chlor, monosubstituiertem Phenyl verschieden ist, wenn $R_2$ N,N-Di-$C_1$-$C_4$-alkylcarbamyl, worin die beiden N-Alkylgruppen gleich oder verschieden sind, bedeutet, und jeweils ihre Salze sowie gegebenenfalls jeweils ihre Tautomeren und deren Salze sowie ein Verfahren zur Herstellung der letztgenannten, neuen Verbindungen der Formel I oder ihrer Tautomeren und/oder Salze.

Die Erfindung betrifft vor allem pharmazeutische, insbesondere antikonvulsiv wirksame, Präparate, die Herstellung derselben und ein Behandlungsverfahren, dadurch gekennzeichnet, dass man als Wirkstoff eine Verbindung der Formel I, worin Ph durch $C_1$-$C_4$-Alkyl, Halogen oder Trifluormethyl monosubstituiertes oder durch Halogen, Halogen sowie $C_1$-$C_4$-Alkyl oder Halogen sowie Trifluormethyl disubstituiertes Phenyl bedeutet, wobei jeweils $C_1$-$C_4$-Alkyl z.B. für Methyl steht und Halogen eine Atomnummer bis und mit 35 aufweist und unabhängig voneinander z.B. für Fluor oder in zweiter Linie für Chlor steht, alk 1,1- oder 2,2-$C_1$-$C_4$-Alkyliden, wie Methylen oder Aethyliden, darstellt, $R_1$ Wasserstoff oder $C_1$-$C_4$-Alkyl, wie Methyl oder Aethyl, ist und $R_2$ Carbamyl, N-$C_1$-$C_4$-Alkylcarbamyl, wie N-Methyl- oder N-Aethylcarbamyl, N,N-Di-$C_1$-$C_4$-alkylcarbamyl, wie N,N-Dimethylcarbamyl, oder N-$C_2$-$C_7$-Alkanoylcarbamyl, wie N-Acetylcarbamyl, bedeutet, vorzugsweise eine solche Verbindung der Formel I, worin Ph mindestens einen der Alkyl- und/oder Halogensubstituenten in einer o-Stellung oder einen Trifluormethylsubstituenten in einer m-Stellung trägt, oder gegebenenfalls ein Tautomeres und/oder ein pharmazeutisch verwendbares Salz davon wählt, sowie eine Verbindung der Formel I, worin Ph durch $C_1$-$C_4$-Alkyl, Halogen oder Trifluormethyl monosubstituiertes oder durch Halogen, Halogen sowie $C_1$-$C_4$-Alkyl oder Halogen sowie Trifluormethyl disubstituiertes Phenyl bedeutet, wobei jeweils $C_1$-$C_4$-Alkyl z.B. für Methyl steht und Halogen eine Atomnummer bis und mit 35 aufweist und unabhängig voneinander z.B. für Fluor oder in zweiter Linie für Chlor steht, alk 1,1- oder 2,2-$C_1$-$C_4$-Alkyliden, wie Methylen oder Aethyliden, darstellt, $R_1$ Wasserstoff oder $C_1$-$C_4$-Alkyl, wie Methyl oder Aethyl, ist und $R_2$ Carbamyl, N-$C_1$-$C_4$-Alkylcarbamyl, wie N-Methyl- oder N-Aethylcarbamyl, N,N-Di-$C_1$-$C_4$-alkylcarbamyl, wie N,N-Dimethylcarbamyl, oder N-$C_2$-$C_7$-Alkanoylcarbamyl, wie N-Acetylcarbamyl, bedeutet, vorzugsweise eine solche Verbindung der Formel I, worin Ph mindestens einen der Alkyl- und/oder Halogensubstituenten in einer o-Stellung oder einen Trifluormethylsubstituenten in einer m-Stellung trägt, mit der Massgabe, dass in einer Verbindung der Formel I, worin $R_1$ Methyl, $R_2$ N,N-Diäthylcarbamyl und alk Methylen bedeutet, Ph von in p-Stellung durch Chlor substituiertem Phenyl verschieden ist, beispielsweise eine solche Verbindung der Formel I, worin Ph von durch Halogen, namentlich durch Chlor, monosubstituiertem Phenyl verschieden ist, wenn $R_2$ N,N-Di-$C_1$-$C_4$-alkylcarbamyl, worin die beiden N-Alkylgruppen gleich oder verschieden sind, bedeutet, und jeweils ihre Salze sowie gegebenenfalls jeweils ihre Tautomeren und deren Salze sowie ein Verfahren zur Herstellung der letztgenannten, neuen Verbindungen der Formel I oder ihrer Tautomeren und/oder Salze.

Die Erfindung betrifft vorzugsweise pharmazeutische, insbesondere antikonvulsiv wirksame, Präparate, die Herstellung derselben und ein Behandlungsverfahren, dadurch gekennzeichnet, dass man als Wirkstoff eine Verbindung der Formel I, worin Ph o-$C_1$-$C_4$-Alkylphenyl, m-Trifluormethylphenyl, o-Halogenphenyl oder 2,5- oder 2,6-, ferner 2,3- oder 2,4-Dihalogenphenyl bedeutet, wobei $C_1$-$C_4$-Alkyl z.B. für Methyl steht und Halogen jeweils eine Atomnummer bis und mit 35 aufweist und unabhängig voneinander z.B. für Fluor oder in zweiter Linie für Chlor steht, alk 1,1-$C_1$-$C_4$-Alkyliden, wie Methylen oder Aethyliden, darstellt, $R_1$ Wasserstoff oder $C_1$-$C_4$-Alkyl, wie Methyl oder Aethyl, ist und $R_2$ Carbamyl oder in zweiter Linie N-$C_1$-$C_4$-Alkyl-carbamyl, wie N-Methyl- oder N-Aethylcarbamyl, N,N-Di-$C_1$-$C_4$-alkyl-carbamyl, wie N,N-Dimethylcarbamyl, oder N-$C_2$-$C_7$-Alkanoylcarbamyl, wie N-Acetylcarbamyl, bedeutet, oder gegebenenfalls ein Tautomeres und/oder ein pharmazeutisch verwendbares Salz davon wählt, sowie eine Verbindung der Formel I, worin Ph o-$C_1$-$C_4$-Alkylphenyl, m-Trifluormethylphenyl, o-Halogenphenyl oder 2,5- oder 2,6-, ferner 2,3-oder 2,4-Dihalogenphenyl bedeutet, wobei $C_1$-$C_4$-Alkyl z.B. für Methyl steht und Halogen jeweils eine Atomnummer bis und mit 35 aufweist und unabhängig voneinander z.B. für Fluor oder in zweiter Linie für Chlor steht, alk 1,1,$C_1$-$C_4$-Alkyliden, wie Methylen oder Aethyliden, darstellt, $R_1$ Wasserstoff oder $C_1$-$C_4$-Alkyl, wie Methyl oder Aethyl, ist und $R_2$ Carbamyl oder in zweiter Linie N-$C_1$-$C_4$-Alkylcarbamyl, wie N-Methyl- oder N-Aethylcarbamyl, N,N-Di-$C_1$-$C_4$-alkylcarbamyl, wie N,N-Dimethylcarbamyl, oder N-$C_2$-$C_7$-Alkanoylcarbamyl, wie N-Acetylcarbamyl, bedeutet, mit der Massgabe, dass in einer Verbindung der Formel I, worin $R_1$ Methyl, $R_2$ N,N-Diäthylcarbamyl und alk Methylen bedeutet, Ph von in p-Stellung durch Chlor substituiertem Phenyl verschieden ist, beispielsweise eine solche Verbindung der Formel I, worin Ph von durch Halogen, namentlich durch Chlor, monosubstituiertem Phenyl verschieden ist, wenn $R_2$ N,N-Di-$C_1$-$C_4$-alkylcarbamyl, worin die beiden N-Alkylgruppen gleich oder verschieden sind, bedeutet, und jeweils ihre Salze sowie gegebenenfalls jeweils ihre Tautomeren und deren Salze sowie ein Verfahren zur Herstellung der letztgenannten, neuen Verbindungen der Formel I oder ihrer Tautomeren und/oder Salze.

Die Erfindung betrifft insbesondere pharmazeutische, insbesondere antikonvulsiv wirksame, Präparate, die Herstellung derselben und ein Behandlungsverfahren, dadurch gekennzeichnet, dass man als Wirkstoff eine Verbindung der Formel I, worin Ph m-Trifluormethylphenyl, o-Fluorphenyl oder 2,6-Difluorphenyl bedeutet, alk Methylen darstellt, $R_1$ $C_1$-$C_4$-Alkyl, wie Methyl oder Aethyl, ist und $R_2$ für Carbamyl steht, oder ein pharmazeutisch verwendbares Salz davon wählt, sowie eine Verbindung der Formel I, worin Ph m-Trifluormethylphenyl, o-Fluorphenyl oder 2,6-Difluorphenyl bedeutet, alk Methylen darstellt, $R_1$ $C_1$-$C_4$-Alkyl, wie Methyl oder Aethyl, ist und $R_2$ für Carbamyl steht, und ihre Salze sowie ein Verfahren zur Herstellung der letztgenannten, neuen Verbindungen der Formel I oder ihrer Salze.

Die Erfindung betrifft in allererster Linie pharmazeutische, insbesondere antikonvulsiv wirksame, Präparate, die Herstellung derselben und ein Behandlungsverfahren, dadurch gekennzeichnet, dass man als Wirkstoff eine Verbindung der Formel I, worin Ph m-Trifluormethylphenyl oder 2,6-Difluorphenyl bedeutet, alk Methylen darstellt, $R_1$ $C_1$-$C_4$-Alkyl, wie Methyl, ist und $R_2$ für Carbamyl steht, oder ein pharmazeutisch verwendbares Salz davon wählt, sowie eine Verbindung der Formel I, worin Ph m-Trifluormethylphenyl oder 2,6-Difluorphenyl bedeutet, alk Methylen darstellt, $R_1$ $C_1$-$C_4$-Alkyl, wie Methyl, ist und $R_2$ für Carbamyl steht, und ihre Salze sowie ein Verfahren zur Herstellung der letztgenannten, neuen Verbindungen der Formel I oder ihrer Salze.

Die Erfindung betrifft namentlich pharmazeutische, insbesondere antikonvulsiv wirksame, Präparate, die Herstellung derselben und ein Behandlungsverfahren, dadurch gekennzeichnet, dass man als Wirkstoff eine der in den Beispielen genannten neuen Verbindungen der Formel I oder gegebenenfalls ein Tautomeres und/oder ein pharmazeutisch verwendbares Salz davon wählt, sowie die in den Beispielen genannten neuen Verbindungen der Formel I und ihre Salze sowie gegebenenfalls ihre Tautomeren und deren Salze sowie ein Verfahren zur Herstellung der letztgenannten, neuen Verbindungen der Formel I oder ihrer Tautomeren und/oder Salze.

Das erfindungsgemässe Verfahren zur Herstellung der neuen Verbindungen der Formel I oder gegebenenfalls ihrer Tautomeren und/oder Salze beruht auf an sich bekannten Verfahrensweisen. Es ist z.B. dadurch gekennzeichnet, dass man

a) aus einer Verbindung der Formel

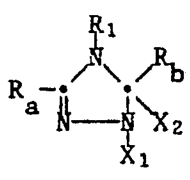 (II),

worin $X_1$ und $X_2$ unter Ausbildung einer zusätzlichen Bindung eliminierbare Gruppen bedeuten und einer der beiden Reste $R_a$ und $R_b$ eine Gruppe Ph-alk und der andere einen Rest $R_2$ darstellt, oder gegebenenfalls einem Tautomeren und/oder Salz davon $X_1$ und $X_2$ eliminiert oder

b) in einer Verbindung der Formel

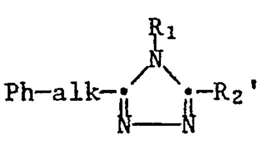 (III),

worin $R_2$' eine in einen Rest $R_2$ überführbare Gruppe bedeutet, oder gegebenenfalls einem Tautomeren und/oder Salz davon $R_2$' in $R_2$ überführt oder

c) zur Herstellung einer Verbindung der Formel I, worin $R_1$ von Wasserstoff verschieden ist und $R_2$ insbesondere N,N-Di-$C_1$-$C_4$-alkylcarbamyl ist, in eine Verbindung der Formel

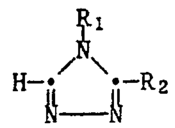
(IV)

oder ein Salz davon den Rest der Formel Ph-alk einführt oder

d) zur Herstellung einer Verbindung der Formel I, worin $R_1$ Wasserstoff ist, in einer Verbindung der Formel

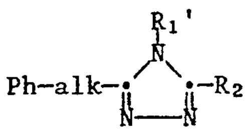
(V),

worin $R_1$' eine durch Wasserstoff ersetzbare Gruppe darstellt, oder einem Salz davon, $R_1$' durch Wasserstoff ersetzt oder

e) zur Herstellung einer Verbindung der Formel I, worin $R_2$ N,N-Di-$C_1$-$C_4$-alkylcarbamyl ist, in einer Verbindung der Formel

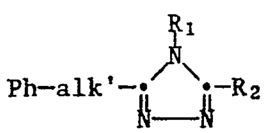
(VI),

worin alk' einen in eine Gruppe alk überführbaren Rest darstellt, oder gegebenenfalls einem Tautomeren und/oder Salz davon, alk' in alk überführt und jeweils ein gegebenenfalls verfahrensgemäss erhältliches Isomerengemisch in die Komponenten auftrennt und das Isomere der Formel I isoliert, gewünschtenfalls jeweils eine verfahrensgemäss oder auf andere Weise erhältliche Verbindung der Formel I in eine andere Verbindung der Formel I überführt, jeweils ein gegebenenfalls verfahrensgemäss erhältliches Stereoisomerengemisch in die Stereoisomeren auftrennt und das gewünschte Stereoisomere isoliert und/oder jeweils eine verfahrensgemäss erhältliche freie Verbindung der Formel I in ein Salz überführt oder ein verfahrensgemäss erhältliches Salz in die freie Verbindung der Formel I oder in ein anderes Salz umwandelt.

Die vor- und nachstehend in den Varianten beschriebenen Umsetzungen sowie die Herstellung neuer Ausgangsstoffe bzw. Zwischenprodukte erfolgen in Analogie zur Reaktions- und Bildungsweise bekannter Ausgangsstoffe bzw. Zwischenprodukte. Dabei wird, auch wenn nachstehend nicht ausdrücklich erwähnt, unter den jeweils üblichen Reaktions-, wie Temperatur- und Druckbedingungen, sowie unter Verwendung der jeweils üblichen Hilfsmittel, wie Katalysatoren, Kondensations- sowie Solvolysemittel und/oder geeigneter Lösungsoder Verdünnungsmittel oder eines Gemisches derselben, sowie gegebenenfalls unter Schutzgas, in einem geschlossenen Gefäss und/oder unter wasserfreien Bedingungen gearbeitet.

In den für die Verfahrensvariante a) zu verwendenden Verbindungen II bedeutet $X_1$ beispielsweise Wasserstoff und $X_2$ eine nucleofuge Abgangsgruppe. Nucleofuge Abgangsgruppen $X_2$ sind beispielsweise gegebenenfalls verätherte oder veresterte Hydroxy- oder Mercaptogruppen, ferner Amino-, Ammonium- und Sulfoniumgruppen. Veräthertes Hydroxy ist beispielsweise Niederalkoxy, wie Methoxy oder Aethoxy, oder gegebenenfalls substituiertes Phenylniederalkoxy, wie gegebenenfalls substituiertes Benzyloxy. Verestertes Hydroxy bedeutet insbesondere mit einer Mineralsäure oder einer organischen Sulfonsäure verestertes Hydroxy, vor allem Halogen, wie Chlor, Brom oder Iod, Sulfonyloxy, wie gegebenenfalls durch Halogen substituiertes Niederalkansulfonyloxy, z.B. Methan- oder Trifluormethansulfonyloxy, Cycloalkansulfonyloxy, z.B. Cyclohexansulfonyloxy, oder gegebenenfalls durch Niederalkyl oder Halogen substituiertes Benzolsulfonyloxy, z.B. Benzol-, p-Bromphenyl- oder p-Toluolsulfonyloxy, ferner Niederalkanoyloxy, z.B. Acetoxy oder

Pivaloyloxy. Veräthertes Mercapto ist z.B. Niederalkylthio, wie Methyl- oder Aethylthio, oder gegebenenfalls substituiertes Phenylthio, wie Phenylthio oder p-Tolylthio. Veresterte Mercaptogruppen sind beispielsweise Niederalkanoylthiogruppen, wie Acetylthio. Aminogruppen sind beispielsweise Amino-, N-Niederalkylamino-, N,N-Diniederalkylamino- oder N-Niederalkanoylaminogruppen, ferner Niederalkylen- bzw. Aza-, Oxa-oder Thianiederalkylenaminogruppen, z.B. Dimethylamino oder Diäthylamino, ferner Pyrrolidino, Piperidino, Morpholino oder Thiomorpholino, weiterhin Anilino. Ammoniumgruppen sind beispielsweise den vorstehend genannten Aminogruppen entsprechende tertiäre oder quaternäre Ammoniumgruppen, wie Triniederalkylammonio oder Pyridinio. Sulfoniumgruppen sind z.B. Diniederalkylsulfoniumgruppen, wie Dimethylsulfonium.

Für Salze und/oder Tautomere von Verbindungen II gilt in analoger Weise das vorstehend für Salze und/oder Tautomere von Verbindungen I Gesagte.

Die Eliminierung von $X_1$ und $X_2$ unter Ausbildung einer zusätzlichen Bindung (Abspaltung von $X_1$-$X_2$) erfolgt in üblicher Weise, beispielsweise durch Erwärmen auf etwa 40 bis etwa 200° C, und/oder im Falle von Verbindungen II, worin $X_2$ gegebenenfalls mit einer Mineralsäure oder organischen Sulfonsäure verestertes Hydroxy ist, durch Basebehandlung bzw. im Falle von Verbindungen II, worin $X_2$ gegebenenfalls veräthertes Hydroxy oder Mercapto bedeutet, durch Säurebehandlung. Dafür geeignete Basen bzw. Säuren sind beispielsweise die nachstehend genannten basischen bzw. sauren Mittel.

Die Verbindungen II werden vorzugsweise in situ hergestellt, beispielsweise indem man eine Verbindung der Formel

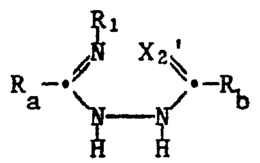 (IIa),

worin $X_2'$ gegebenenfalls funktionell abgewandeltes Oxo bedeutet, oder ein Tautomeres und/oder Salz davon cyclisiert, wobei die intermediär gebildete Verbindung II im allgemeinen ohne Isolierung erfindungsgemäss weiterreagiert.

Gegebenenfalls funktionell abgewandeltes Oxo $X_2'$ ist beispielsweise Oxo, Thioxo oder eine Gruppe $=N-R_1''$. Gruppen $=N-R_1''$ sind beispielsweise solche, in denen $R_1''$ eine Gruppe $R_1$ oder eine unter den Reaktionsbedingungen in $R_1$ überführbare Gruppe $R_1'$, z.B. die Acylgruppe, ist, z.B. Imino, N-Niederalkylimino, N-Niederalkanoylimino oder gegebenenfalls substituiertes N-Benzoylimino.

Tautomere von Verbindungen IIa sind beispielsweise solche, in denen die $R_a$-C($=NR_1$)-NH-Gruppierung in protomerer Form, d.h. als $R_a$-C($NHR_1$)$=$N-Gruppierung, vorliegt. Ebenso kann z.B. eine Verbindung IIa mit einer Oxo-, Thioxo- oder Iminogruppe $X_2'$ auch in ihrer tautomeren Form als Enol, En-Thiol oder Enamin der Formel

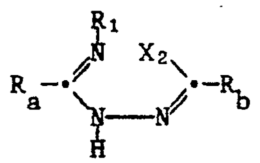 (IIb),

worin $X_2$ Hydroxy, Mercapto oder Amino bedeutet, vorliegen. Für Salze von Verbindungen IIa oder IIb gilt in analoger Weise das vorstehend für Salze von Verbindungen I Gesagte.

Die Cyclisierung von Verbindungen IIa oder deren Tautomeren und/oder Salzen und gegebenenfalls die nachfolgende in situ-Eliminierung von $X_1$-$X_2$ aus den gebildeten Verbindungen II oder gegebenenfalls deren Tautomeren und/oder Salzen erfolgen in üblicher Weise, d.h. unter neutralen, sauren oder basischen Bedingungen, erforderlichenfalls in Gegenwart eines sauren oder basischen Mittels, in Gegenwart eines inerten Lösungs- oder Verdünnungsmittels oder eines Gemisches derselben, bei Raumtemperatur oder vorzugsweise unter Erwärmen, z.B. im Temperaturbereich von etwa 40 bis etwa 200° C, bevorzugt von etwa 60 bis etwa 140° C, und/oder unter Inertgas, wie Stickstoff.

Als saure Mittel kommen beispielsweise Mineralsäuren oder deren Anhydride oder sauren Salze, z.B. Halogenwasserstoffsäuren, Schwefelsäure, Alkalimetallhydrogensulfate, Phosphorsäure, Polyphosphorsäure, Phosphorpentoxid, Phosphortrichlorid, Phosphoroxychlorid oder Phosphortribromid, organische Sulfonsäuren, wie p-Toluolsulfonsäure, oder Carbonsäuren oder deren Anhydride oder Halogenide, wie Niederalkansäuren oder deren Anhydride oder Halogenide, z.B. Essigsäure, Acetanhydrid oder Acetylchlorid, ferner gepufferte Säurelösungen, z.B. Phosphat- oder Acetatpuffer, oder Hydrohalogenide von Stickstoffbasen, z.B. Ammonium- oder Pyridiniumchlorid, in Betracht.

Basische Mittel sind beispielsweise Alkali- oder Erdalkalimetallhydroxide, -hydride, -amide, -niederalkanolate, -carbonate, -diniederalkylamide oder -niederalkylsilylamide, Niederalkylamine, basische Heterocyclen, Ammoniumhydroxide sowie carbocyclische Amine. Beispielhaft seien Natriumhydroxid, -hydrid, -amid, -methanolat, -äthanolat, -carbonat, Kaliumhydroxid, -tert.-butanolat, -carbonat, Lithiumdiisopropylamid, Kalium-bis-(trimethylsilyl)-amid, Calciumhydroxid, -hydrid, Di- oder Triäthylamin, Pyridin, Benzyltrimethylammoniumhydroxid, 1,5-Diazabicyclo[4.3.0]non-5-en (DBN) sowie 1,5-Diazabicyclo[5.4.0]undec-5-en (DBU) genannt.

Als inerte Lösungs- oder Verdünnungsmittel sind beispielsweise Halogenniederalkane, cyclische Aether, aromatische Kohlenwasserstoff, N,N-Diniederalkylniederalkansäureamide, Phosphorsäureniederalkylamide, Diniederalkylsulfoxide und cyclische Amine, ferner, insbesondere bei Durchführung der Umsetzung in Gegenwart eines Alkoholats, die dem Alkoholat entsprechenden Alkohole zu nennen, z.B. Di-, Tri- oder Tetrachlormethan, Tetrahydrofuran, Dioxan, Benzol, Toluol, Xylol, N,N-Dimethylformamid, Hexamethylphosphorsäuretriamid, Dimethylsulfoxid oder N-Methylmorpholin, ferner z.B. Methanol, Aethanol oder tert.-Butanol.

Die Verbindungen IIa oder deren Tautomere und/oder Salze können beispielsweise durch Umsetzung entsprechender Verbindungen der Formeln

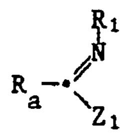 (IIc) und 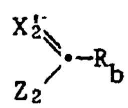 (IId),

worin $Z_1$ eine nucleofuge Abgangsgruppe $X_2$, $X_2'$ gegebenenfalls funktionell abgewandeltes Oxo und $Z_2$ Hydrazino darstellt, oder von Tautomeren und/oder Salzen derselben hergestellt und vorteilhaft in situ zu Verbindungen II bzw. I weiterumgesetzt werden.

So erhält man Verbindungen IIa, worin $R_a$ eine Gruppe Ph-alk, $R_b$ ein Rest $R_2$ und $X_2'$ gegebenenfalls funktionell abgewandeltes Oxo, vorzugsweise Oxo, ist, vorzugsweise, indem man entsprechende Verbindungen der Formeln IIc und IId, worin Z1 eine nucleofuge Abgangsgruppe $X_2$, vorzugsweise Niederalkoxy, $X_2'$ gegebenenfalls funktionell abgewandeltes Oxo, vorzugsweise Oxo, und $Z_2$ Hydrazino darstellt, oder gegebenenfalls deren Tautomere und/oder Salze miteinander umsetzt, wobei vorzugsweise in einem der zuvor beschriebenen inerten Lösungsmittel, unter Erwärmen, z.B. im Temperaturbereich von etwa 20 bis etwa 200 °C, insbesondere von etwa 40 bis etwa 140 °C, gegebenenfalls in Gegenwart eines der vorstehend erwähnten sauren oder basischen Mittel und/oder unter Inertgas, wie Stickstoff, gearbeitet wird. Die Verbindungen IIc ($R_a$ = Ph-alk, $Z_1$ = Niederalkoxy) erhält man z.B. durch Umsetzung eines Phenylalkansäureamids der Formel Ph-alk-$CONHR_1$ (IIe) mit einem geeigneten Niederalkylierungsmittel, vorzugsweise einem Triniederalkyl-, wie Triäthyloxoniumtetrafluoroborat, z.B. in Dichlormethan, oder durch Umsetzung eines Nitrils der Formel Ph-alk-CN (IIf) mit einem Alkohol in Gegenwart von trockenem Chlorwasserstoffgas und gegebenenfalls Umwandlung des erhaltenen Iminiumhydrochlorids in die freie Base durch Behandlung mit einer schwachen Base, wie Natriumhydrogencarbonat. Die Verbindungen IId ($R_b$ = $R_2$, $X_2'$ = Oxo, $Z_2$ = Hydrazino) sind bekannt oder in Analogie zu bekannten Methoden herstellbar.

Analog kann man Verbindungen IIa, worin $R_a$ eine Gruppe $R_2$, $R_b$ ein Rest Ph-alk- und $X_2'$ gegebenenfalls funktionell abgewandeltes Oxo, vorzugsweise Oxo, ist, vorzugsweise erhalten, indem man entsprechende Verbindungen IIc und IId, worin $Z_1$ eine nucleofuge Abgangsgruppe $X_2$, vorzugsweise Halogen, wie Chlor, $X_2'$ gegebenenfalls funktionell abgewandeltes Oxo, vorzugsweise Oxo, und $Z_2$ Hydrazino darstellt, oder gegebenenfalls deren Tautomere und/oder Salze miteinander umsetzt, wobei vorzugsweise z.B. unter Kühlung, beispielsweise im Temperaturbereich von etwa -20 bis etwa +15 °C, insbesondere von etwa -5 bis etwa +10 °C, in einem inerten Lösungsmittel, in Gegenwart eines basischen Mittels und/oder unter Inertgas, wie Stickstoff, gearbeitet wird. Die Verbindungen IIc ($R_a$ = $R_2$, $Z_1$ = Halogen) sind bekannt oder in Analogie zu bekannten Methoden erhältlich, z.B. durch Umsetzung von gegebenenfalls N-Niederalkyl-, N,N-Diniederalkyl- oder N-Niederalkanoyl-substituierten Oxalsäurediamiden mit Halogenierungsmitteln, wie Phosgen oder Phosphortribromid. Die Verbindungen IId ($R_b$ = Ph-alk, $X_2'$ = Oxo, $Z_2$ = Hydrazino) sind bekannt oder in Analogie zu bekannten Methoden herstellbar.

Verbindungen IIa, worin $R_a$ eine Gruppe $R_2$, $R_b$ ein Rest Ph-alk- und $X_2'$ gegebenenfalls funktionell abgewandeltes Oxo, vorzugsweise Oxo, ist, kann man auch dadurch erhalten, dass man zunächst eine Verbindung der Formel $R_1N=C(Z_1)-R_2'$ (II c'), worin $R_2'$ beispielsweise Niederalkoxy-, wie Aethoxycarbonyl, oder gegebenenfalls substituiertes Phenylniederalkoxycarbonyl und $Z_1$ eine nucleofuge Abgangsgruppe $X_2$, vorzugsweise Halogen, wie Chlor, ist, und eine Verbindung IId, worin $R_b$ einen Rest Ph-alk, $X_2'$ gegebenenfalls funktionell abgewandeltes Oxo, vorzugsweise Oxo, und $Z_2$ Hydrazino darstellt, oder gegebenenfalls jeweils ein Tautomeres und/oder Salz davon miteinander umsetzt, beispielsweise unter den zuvor angegebenen Reaktionsbedingungen, z.B. unter Kühlung und/oder in Gegenwart eines basischen Mittels, und anschliessend in der erhaltenen Verbindung der Formel $R_2'$ $C(=NR_1)-NHNH-C(=X_2')$alk-Ph (IIa') oder in einem gegebenenfalls erhaltenen Tautomeren und/oder Salz davon die Gruppe $R_2'$ zu einer Carbamylgruppe $R_2$ solvolysiert, beispielsweise durch Umsetzung mit Ammoniak, N-Mono oder N,N-Diniederalkyl- oder N-Niederalkanoylaminen, vorteilhaft unter Erwärmen, z.B. im Temperaturbereich von etwa 30 bis etwa 100° C, bevorzugt von etwa 40 bis etwa 80° C, in Gegenwart eines Dehydratisierungsmittels, wie N,N'-Dicyclohexylcarbodiimid, und/oder unter Inertgas, wie Stickstoff. Die Verbindungen IIc' sind bekannt oder in Analogie zu bekannten Methoden erhältlich, z.B. in analoger Weise wie zuvor beschrieben durch Umsetzung von Oxalsäuremonoalkylestermonoamiden mit Halogenierungsmitteln, wie Phosgen oder Phosphortribromid.

Analog kann man auch Verbindungen IIa, worin $R_a$ eine Gruppe Ph-alk, $R_b$ ein Rest $R_2$ und $X_2'$ gegebenenfalls funktionell abgewandeltes Oxo, vorzugsweise Oxo, ist, dadurch erhalten, das man zunächst eine Verbindung der Formel $H_2N-NH-C(=X_2')-R_2'$ (IId'), worin $R_2'$ beispielsweise Niederalkoxy-, wie Aethoxycarbonyl, oder gegebenenfalls substituiertes Phenylniederalkoxycarbonyl und $X_2'$ gegebenenfalls funktionell abgewandeltes Oxo, vorzugsweise Oxo, ist, und eine Verbindung IIc, worin $R_a$ ein Rest Ph-alk und $Z_1$ eine nucleofuge Abgangsgruppe $X_2$, vorzugsweise Niederalkoxy, ist, oder gegebenenfalls jeweils ein Tautomeres und/oder Salz davon miteinander umsetzt, beispielsweise unter den zuvor angegebenen Reaktionsbedingungen, z.B. unter Kühlung und/oder in Gegenwart eines basischen Mittels, und anschliessend in der erhaltenen Verbindung der Formel Ph-alk-$C(=NR_1)-NHNH-C(=X_2')-R_2'$ (IIa") oder in einem gegebenenfalls erhaltenen Tautomeren und/oder Salz davon die Gruppe $R_2'$ zu einer Carbamylgruppe $R_2$ solvolysiert, beispielsweise durch Umsetzung mit Ammoniak, N-Mono- oder N,N-Diniederalkyl- oder N-Niederalkanoylaminen unter den zuvor beschriebenen Reaktionsbedingungen. Die Verbindungen IId' sind bekannt oder in Analogie zu bekannten Methoden erhältlich.

Verbindungen IIa, worin einer der beiden Reste $R_a$ und $R_b$ eine Gruppe Ph-alk und der andere einen Rest $R_2$ darstellt und $X_2'$ gegebenenfalls funktionell abgewandeltes Oxo, vorzugsweise Oxo, ist, kann man auch erhalten, indem man eine Verbindung der Formel Ph-alk-$C(=X_2')NHNHC(=X_2'')-Z_3$ (IIg), worin $X_2'$ und $X_2''$ unabhängig voneinander gegebenenfalls funktionell abgewandeltes Oxo, vorzugsweise beide Oxo, und $Z_3$ entweder einen Rest $R_2$ oder eine in einen Rest $R_2$ überführbare Gruppe $R_2'$ , vorzugsweise eine Niederalkoxy-wie Aethoxycarbonylgruppe, oder eine gegebenenfalls substituierte Phenylniederalkoxycarbonylgruppe, bedeuten, bzw. gegebenenfalls ein Tautomeres und/oder Salz davon mit Ammoniak oder einem Amin $R_1NH_2$, vorteilhaft in Gegenwart eines basischen Mittels, wie Pyridin, umsetzt und gegebenenfalls anschliessend eine Gruppe $R_2'$ in den Rest $R_2$ überführt, z.B. durch Solvolyse, beispielsweise durch Umsetzung mit Ammoniak, N-Mono- oder N,N-Diniederalkyl- oder N-Niederalkanoylaminen unter den zuvor beschriebenen Reaktionbedingungen. Die Verbindungen IIg sind in Analogie zu bekannten Methoden, z.B. durch Umsetzung einer Verbindung der Formel $Z_3-C(=X_2'')-NHNH_2$ (IId") mit einer Verbindung der Formel Ph-alk-$C(=X_2')-Z_1$ (IIc"), worin $Z_1$ eine nucleofuge Abgangsgruppe $X_2$, vorzugsweise Niederalkoxy, bedeutet, erhältlich.

In einer besonders bevorzugten Ausführungsform der Verfahrensvariante a) wird eine Verbindung IIc ($R_a$ = Ph-alk, $Z_1$ = Niederalkoxy) bei etwa 80° bis etwa 120° C z.B. in N-Methylmorpholin oder N,N-Dimethylformamid mit einer Verbindung IId ($R_b$ = $R_2$, $X_2'$ = Oxo, $Z_2$ = Hydrazino) umgesetzt, wobei das Primärprodukt IIa ($R_a$ = Ph-alk-, $R_b$ = $R_2$, $X_2'$ = Oxo) unter intermediärer Bildung der entsprechenden Verbindung II ($R_a$ = Ph-alk-, $R_b$ = $R_2$, $X_1$ = Wasserstoff, $X_2$ = Hydroxy) in situ durch Eliminierung von Wasser zu der entsprechenden Zielverbindung der Formel I weiterreagiert.

Gemäss der Verfahrensvariante b) in gegebenenfalls N-mono- oder N,N-diniederalkylierte oder N-niederalkanoylierte Carbamylreste $R_2$ überführbare Gruppen $R_2'$ sind entweder zu Resten $R_2$ solvolysierbare Gruppen $R_2'$ oder oxidativ in Reste $R_2$ überführbare Gruppen $R_2'$.

Zu Resten $R_2$ solvolysierbare Gruppen $R_2'$ sind vorzugsweise z.B. freie, veresterte oder in einer Salz- oder Anhydridform vorliegende Carboxygruppen, die Cyanogruppe oder gegebenenfalls niederalkylierte oder niederalkanoylierte Amidinogruppen.

Veresterte Carboxygruppen sind beispielsweise mit einem Niederalkanol oder einem Niederalkylmercaptan veresterte Carboxygruppen, d.h. Niederalkoxy- oder Niederalkylthiocarbonylgruppen, können aber auch mit einem anderen Alkohol oder Mercaptan, z.B. mit einem gegebenenfalls substituierten Phenol,

verestert sein.

In Salzform vorliegende Carboxygruppen sind beispielsweise in einer von Ammoniak oder einem Mono- oder Diniederalkylamin abgeleiteten Ammoniumsalzform vorliegende Carboxygruppen, ferner in Metall-, z.B. Alkali- oder Erdalkalimetallsalzform, vorliegende Carboxygruppen.

In Anhydridform vorliegende Carboxygruppen sind beispielsweise in einer Halogenidform vorliegende Carboxygruppen, wie Chlorcarbonyl, können aber auch mit einer reaktiven Carbonsäure anhydridisiert sein und beispielsweise Alkoxycarbonyloxycarbonyl oder Trifluoracetoxycarbonyl bedeuten.

Gegebenenfalls niederalkylierte oder niederalkanoylierte Amidinogruppen sind beispielsweise unsubstituierte oder N-niederalkylierte, N,N-diniederalkylierte oder N-niederalkanoylierte Amidinogruppen.

Für Salze und/oder Tautomere von Verbindungen III gilt in analoger Weise das vorstehend für Salze und/oder Tautomere von Verbindungen I Gesagte.

Die Ueberführung der genannten Gruppen $R_2'$ in gegebenenfalls wie angegeben substituiertes Carbamyl erfolgt in üblicher Weise, z.B. durch Solvolyse, d.h. Hydrolyse oder Ammono- oder Aminolyse (Umsetzung mit Wasser oder Ammoniak oder einem Mono- oder Diniederalkylamin).

Durch Hydrolyse kann man beispielsweise unsubstituierte oder N-mono-oder N,N-diniederalkylierte oder N-niederalkanoylierte Amidinogruppen $R_2'$ in gegebenenfalls niederalkylierte oder niederalkanoylierte Carbamylgruppen oder Cyano $R_2'$ in Carbamyl überführen. Die Hydrolyse der genannten Amidinogruppen erfolgt beispielsweise unter sauren Bedingungen, z.B. in Gegenwart von Mineralsäuren, z.B. von Salz- oder Schwefelsäure. Die Hydrolyse von Cyano wird beispielsweise in Gegenwart eines basischen Hydrolysemittels, wie eines Alkalimetallhydroxides, z.B. von Natronlauge oder Kalilauge, durchgeführt. Sie kann durch Peroxyverbindungen, z.B. Wasserstoffperoxid, erleichtert werden und erfolgt beispielsweise in einem Niederalkanol, z.B. in Aethanol, als Verdünnungsmittel.

Durch Ammono- oder Aminolyse kann man beispielsweise freie oder veresterte oder in einer Salz- oder Anhydridform vorliegende Carboxygruppen $R_2'$ in unsubstituiertes oder niederalkyliertes Carbamyl überführen. Dabei arbeitet man erforderlichenfalls in Gegenwart eines Kondensationsmittels, vorteilhaft in einem inerten Lösungsmittel. Kondensationsmittel sind beispielsweise basische Kondensationsmittel, vor allem Ammoniak oder für die Aminolyse verwendete Amine im Ueberschuss, ausgehend von in Anhydridform vorliegendem Carboxy ferner Alkalimetallhydroxide oder -carbonate, oder tertiäre organische Stickstoffbasen, wie Triniederalkylamine oder tertiäre heteroaromatische Stickstoffbasen, z.B. Triäthylamin oder Pyridin. Als inertes Lösungsmitel ist beispielsweise Toluol, bei der Solvolyse von veresterten Carboxygruppen ferner Aethanol besonders geeignet. Freie Carboxygruppen können unter Dehydratisierung der intermediär gebildeten Ammoniumsalze, z.B. durch Erhitzen oder Einwirkung wasserentziehender Mittel, wie von Säureanhydriden, z.B. Phosphorpentoxid, Acetylchlorid und dergleichen, oder von Carbodiimiden, z.B. N,N'-Dicyclohexylcarbodiimid, in gegebenenfalls niederalkyliertes Carbamyl überführt werden.

Als oxidativ in eine gegebenenfalls wie angegeben substituierte Carbamylgruppe $R_2$ überführbare Gruppe $R_2'$ kommt beispielsweise die Formylgruppe in Betracht. Die oxidative Ueberführung derselben erfolgt nach üblichen Methoden. So können die Aldehyde der Formel

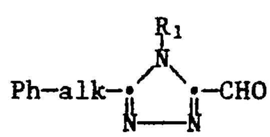

(IIIb)

durch Behandlung mit Ammoniak oder einem N-Mono- oder N,N-Diniederalkylamin unter oxidierenden Bedingungen, beispielsweise in Gegenwart oxidierender Schwermetallverbindungen, wie Mangan-IV-, Eisen-VI- oder Nickel-IV-verbindungen, oder Schwermetallperoxiden, z.B. Nickelperoxid, in entsprechende Verbindungen I überführt werden. Dabei behandelt man die Verbindungen IIIb beispielsweise mit getrocknetem Ammoniakgas unter Kühlung, etwa im Temperaturbereich von etwa -60 bis etwa ± 0 °C, insbesondere von etwa -30 bis etwa -15 °C, und in Gegenwart von Nickelperoxid oder setzt die Aldehyde IIIb mit Mangandioxid und Natriumcyanid in Gegenwart von Ammoniak oder einen N-Niederalkyl- oder N,N-Diniederalkylamin um, wobei beispielsweise in Isopropanol als Lösungsmittel und unter Kühlung, z.B. im Temperaturbereich von etwa -20 bis etwa +10, insbesondere von etwa -5 bis etwa +5 °C, gearbeitet wird.

Die Ausgangsstoffe der Formel III, worin $R_2'$ einen der beschriebenen in $R_2$ überführbaren Reste bedeutet, lassen sich nach mehreren Methoden erhalten, z.B. indem man aus einer Verbindung der Formel

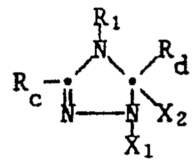 (II'),

worin $X_1$ und $X_2$ unter Ausbildung einer zusätzlichen Bindung eliminierbare Gruppen bedeuten und einer der Reste $R_c$ und $R_d$ eine Gruppe Ph-alk und der andere einen Rest $R_2'$ darstellt, oder gegebenenfalls einem Tautormeren und/oder Salz davon $X_1$ und $X_2$ eliminiert, beispielsweise analog wie unter der Verfahrensvariante a) beschrieben.

Die Verbindungen II' werden vorzugsweise in situ hergestellt, beispielsweise durch Cyclisierung einer Verbindung der Formel

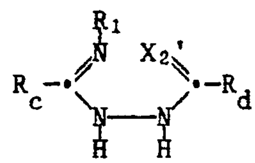 (II'a),

worin $X_2'$ gegebenenfalls funktionell abgewandeltes Oxo bedeutet, oder eines Tautomeren und/oder Salzes davon, wobei analog wie unter der Verfahrensvariante a) beschrieben vorgegangen wird. Die Verbindungen II'a lassen sich z.B. in analoger Weise wie unter der Verfahrensvariante a) für Verbindungen IIa' oder IIa" beschrieben, herstellen.

Eine weitere Methode zur Herstellung der Ausgangsstoffe der Formel III, worin $R_2'$ einen der beschriebenen zu $R_2$ solvolysierbaren Reste bedeutet, beruht auf der Einführung des Restes Ph-alk in eine Verbindung der Formel

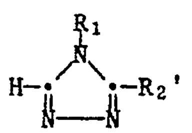 (IIIa)

oder ein Salz davon, wobei in analoger Weise wie unter der Verfahrensvariante c) angegeben verfahren wird.

Verfahrensgemäss erhältliche Verbindungen III, worin $R_2'$ verestertes Carboxy oder Cyano bedeutet, können nachfolgend, z.B. durch Behandeln mit Natrium- oder Kaliumhydroxid in wässrig-äthanolischer Lösung zu den entsprechenden Carbonsäuren (III; $R_2'$ = Carboxy) hydrolysiert werden, die in einem weiteren Reaktionsschritt, z.B. mittels Thionylchlorid in Toluol/Pyridin oder Phosphorpentachlorid in Tetrahydrofuran oder mittels Phosphoroxychlorid, in die Säurechloride (III; $R_2'$ = Chlorcarbonyl) überführt werden können. Primär erhaltene Nitrile (III; $R_2'$ = Cyano) können auch durch Umsetzung mit einer Mineralsäure, z.B. mit Chlor- oder Bromwasserstoffsäure oder Schwefelsäure, und einem Alkohol, z.B. einem Niederalkanol oder Benzylalkohol, und Weiterumsetzung des gebildeten Iminoäthers mit Ammoniak oder einem Mono- oder Diniederalkylamin in die entsprechenden Amidine (III; $R_2'$ = gegebenenfalls niederalkyliertes Amidino) umgewandelt werden.

In einer bevorzugten Ausführungsform zur Herstellung von Verbindungen III, worin $R_2'$ verestertes Carboxy ist, werden Verbindungen der Formeln

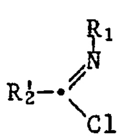 (IIId) und 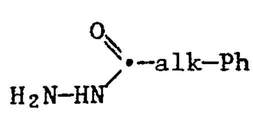 (IIIe)

in Toluol miteinander umgesetzt und das gebildete Primärprodukt IIa' ($X_2$' = Oxo) ohne zusätzliche Reinigung durch mehrstündiges Erhitzen in siedendem Xylol unter intermediärer Bildung der entsprechenden Verbindung II' ($R_c$ = verestertes Carboxy $R_2$', $R_d$ = Ph-alk, $X_1$ = Wasserstoff, $X_2$ = Hydroxy) in situ durch Eliminierung von Wasser in das gewünschte Zwischenprodukt III ($R_2$' = verestertes Carboxy) überführt.

Ausgangsstoffe III, worin $R_2$' Formyl bedeutet, d.h. Aldehyde IIIb, sind auch durch Oxidation entsprechender primärer Alkohole der Formel III ($R_2$' = Hydroxymethyl) zugänglich, wobei die Umsetzung unter den üblichen Reaktionsbedingungen und unter Verwendung dazu gebräuchlicher Oxidationssysteme, wie Dimethylsulfoxid/Oxalylchlorid, Chromtrioxid, Natriumdichromat/Wasser, Pyridiniumchlorochromat, Kaliumpermanganat, Mangandioxid, Bismuttrioxid, Nickelperoxid, Cerammoniumnitrat, Bleitetraacetat/Pyridin oder N-Chlorsuccinimid, oder unter Verwendung von Sauerstoff, beispielsweise in Gegenwart geeigneter Katalysatoren, wie Metalloxiden, z.B. Aluminiumoxid, Siliciumdioxid oder Eisen-(III)-oxid, oder mittels Dehydrogenierungs-Katalysatoren, wie Kupferoxid oder Kupferchromit, ausgeführt wird. Verwendet man Nickelperoxid oder Mangandioxid als Oxidationsmittel, braucht die Aldehydstufe nicht isoliert zu werden, sondern kann in situ mit Ammoniak oder dem entsprechenden Amin weiter umgesetzt werden. Die entsprechenden primären Alkohole (III; $R_2'$ = Hydroxymethyl) können beispielsweise durch Reaktion von Triazolen der Formel

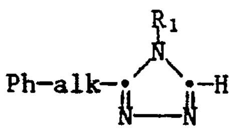

(IIIc)

mit Formaldehyd oder Formaldehyd-Aequivalenten, wie Paraformaldehyd, in Gegenwart eines basischen Mittels erhalten werden. Als basische Mittel kommen insbesondere metallorganische Basen, wie Alkalimetallniederalkyl- oder -phenylverbindungen, z.B. Methyl- oder Butyllithium oder Phenylnatrium, in Betracht, die unter Kühlung, z.B. im Temperaturbereich von etwa -100 bis etwa +10 °C, insbesondere von etwa -80 bis etwa -20 °C, unter Inertgas, wie Stickstoff, und/oder in Gegenwart eines inerten Lösungsmittels, wie Tetrahydrofuran oder 1,2-Dimethoxyäthan, zur Anwendung gelangen. In einer bevorzugten Anwendungsform wird dabei gasförmiger Formaldehyd in das basische Reaktionsgemisch eingeleitet. Ebenso können die primären Alkohole (III; $R_2'$ = Hydroxymethyl) aus entsprechenden Verbindungen II' hergestellt werden.

In einer alternativen Verfahrensweise kann das Triazol IIIc wie beschrieben deprotoniert und durch Umsetzung mit N,N-Diniederalkylformamiden, z.B. N,N-Dimethylformamid, direkt in den Aldehyd der Formel IIIb überführt werden.

Die Triazole IIIc kann man z.B. herstellen, indem man eine Verbindung der Formel HalC(=$NR_1$)H (IIIf), worin Hal für Halogen, wie Chlor, steht, in analoger Weise wie unter der Verfahrensvariante a) für Verbindungen IIc beschrieben, mit einer Verbindung IId ($R_b$ = Ph-alk, $X_2'$ = Oxo, $Z_2$ = Hydrazino) umsetzt.

Die Einführung des Restes der Formel Ph-alk in eine Verbindung der Formel IV, für deren Salze in analoger Weise das vorstehend für Salze von Verbindungen I Gesagte gilt, gemäss Verfahrensvariante c) erfolgt beispielsweise durch Umsetzung mit einem reaktionsfähigen Ester eines entsprechenden im Phenylteil substituierten Phenylniederalkanols.

Reaktionsfähige Ester von im Phenylteil substituierten Phenylniederalkanolen haben beispielsweise die Formel Ph-alk-$Z_4$ (IVa), wobei als reaktionsfähiges verestertes Hydroxy $Z_4$ vorzugsweise mit einer Mineralsäure oder einer organischen Sulfonsäure, wie mit einer Halogenwasserstoffsäure oder einer aliphatischen oder aromatischen Sulfonsäure verestertes Hydroxy, z.B. Chlor, Brom oder Iod oder Methan-, Aethan-, Benzol- oder p-Toluolsulfonyloxy, in Betracht kommt.

Die Umsetzung erfolgt beispielsweise unter basischen Bedingungen, wie in Gegenwart eines basischen Kondensationsmittels, z.B. der in der Verfahrensvariante a) angegebenen Art, vorzugsweise in einem der ebenda angegebenen Lösungs- oder Verdünnungsmittel, erforderlichenfalls unter Erwärmen und/oder unter Inertgas, wie Stickstoff.

Die Verbindungen der Formel IV sind bekannt oder können in Analogie zu bekannten Methoden, beispielsweise durch Umsetzung von Formylhydrazin mit einer Verbindung IIc, worin $R_a$ einen Rest $R_2$ und $Z_1$ eine nucleofuge Abgangsgruppe $X_2$, vorzugsweise Halogen, wie Chlor, darstellt, hergestellt werden, wobei die üblichen Reaktionsbedingungen, beispielsweise wie in der Verfahrensvariante a) beschrieben, angewendet werden.

Gemäss der Verfahrensvariante d) durch Wasserstoff ersetzbare Gruppen $R_1'$ sind beispielsweise Acylgruppen, z.B. von einer organischen Carbon- oder Sulfonsäure abgeleitete Acylgruppen.

Von einer organischen Carbonsäure abgeleitetes Acyl ist beispielsweise der Rest einer aliphatischen oder monocyclisch-aromatischen Carbonsäure, wie Niederalkanoyl oder gegebenenfalls substituiertes Benzoyl, ferner Pyridoyl. Von einer organischen Sulfonsäure abgeleitetes Acyl ist beispielsweise Niederalkansulfonyl.

Für Salze von Verbindungen V gilt in analoger Weise das vorstehend für Salze von Verbindungen I Gesagte.

Für den Austausch der Acylgruppe $R_1'$ gegen Wasserstoff kommen die üblichen Reduktionssysteme und Reaktionsbedingungen in Betracht, z.B. Diboran, Lithiumaluminiumhydrid, Natriumborhydrid/Cobalt-(II)-chlorid, Natriumborhydrid/Trifluoressigsäure oder Trihalogensilane, z.B. Trichlorsilan.

Die Ausgangsstoffe V lassen sich beispielsweise in Analogie zu dem in der Verfahrensvariante a) beschriebenen Eliminierungsverfahren erhalten. So können z.B. eine Verbindung der Formel

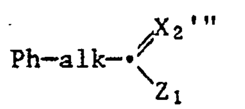

(Va),

worin $X_2'''$ eine Gruppe $=N-R_1'$ ($R_1'$ = Acyl) darstellt und $Z_1$ für eine nucleofuge Abgangsgruppe $X_2$, beispielsweise wie unter der Verfahrensvariante a) beschrieben, steht, und eine Verbindung IId, worin $R_b$ einen Rest $R_2$, $X_2'$ gegebenenfalls funktionell abgewandeltes Oxo, vorzugsweise Oxo, und $Z_2$ Hydrazino darstellt, oder gegebenenfalls jeweils ein Tautomeres und/oder Salz davon miteinander umgesetzt werden, wobei das Primärprodukt der Formel Ph-alk-C(=NR$_1$')-NHNHC(=O)R$_2$ (Vb) unter intermediärer Bildung einer Verbindung der Formel

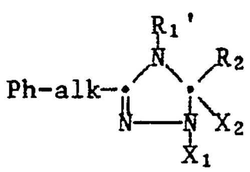

(Vc),

worin $X_1$ Wasserstoff und $X_2$ Hydroxy bedeutet, in situ durch Eliminierung von Wasser zu der entsprechenden Zielverbindung der Formel V weiterreagiert.

Die Ausgangsstoffe der Formel Va können z.B. durch Umsetzung von entsprechend N-substituierten Phenylalkansäureamiden der Formel Ph-alk-C(=O)NHR$_1$' (Vd) mit einem Triniederalkyl-, wie Triäthyloxoniumtetrafluoroborat, analog zu der unter der Variante a) beschriebenen Verfahrensweise, erhalten werden.

In den gemäss Verfahrensvariante e) zu verwendenden Verbindungen der Formel VI, für deren Salze und/oder Tautomere in analoger Weise das vorstehend für Salze und/oder Tautomere von Verbindungen I Gesagte gilt, ist ein in eine Gruppe alk überführbarer Rest alk' insbesondere ein α-Hydroxyniederalkylidenrest -alk"(OH)-, worin alk" eine Niederalkylidengruppe darstellt, welche die für die Gruppe alk in den Verbindungen der Formel I definierten Bedeutungen haben kann, und zusätzlich an dem die beiden Ringsysteme verknüpfenden C-Atom durch Hydroxy substituiert ist. Die entsprechenden Verbindungen der Formel

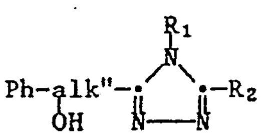

(VIa),

worin $R_2$ N,N-Di-$C_1$-$C_4$-alkyl-, wie N,N-Dimethylcarbamyl, bedeutet, werden durch Reduktion der Hydroxyniederalkylidengruppe zu erfindungsgemässen Verbindungen der Formel I reduziert. Zur Reduktion werden die üblichen Reaktionsbedingungen und Reduktionssysteme angewendet, beispielsweise Kupferchromit, Palladium auf Kohle, Lithiumaluminiumhydrid/Aluminiumchlorid, Iod/Wasser/roter Phosphor oder Wasserstoff in Gegenwart von Katalysatoren, wie Platin. Auch zweistufige Reduktionsverfahren sind üblich, z.B. Ueberführung des Alkohols in ein Sulfonat, z.B. p-Toluolsulfonat, und Reduktion des Sulfonats in situ, z.B. mit Lithiumaluminiumhydrid in Tetrahydrofuran.

Die Ausgangsstoffe VIa werden z.B. erhalten, indem man eine Verbindung IV, worin $R_2$ N,N-Di-$C_1$-$C_4$-alkyl-, wie N,N-Dimethylcarbamyl, bedeutet, in Gegenwart eines basischen Mittels, z.B. der in der Verfahrensvariante a) angegebenen Art, mit einem Aldehyd der Formel Ph-CHO (VIb) umsetzt, wobei die üblichen Reaktionsparameter gewählt werden.

Verfahrensgemäss oder anderweitig erhältliche Verbindungen der Formel I können in andere Verbindungen der Formel I überführt werden, indem man eine oder mehrere Variablen der allgemeinen Formel I in andere überführt.

So kann man in Verbindungen I unsubstituiertes Carbamyl $R_2$ in N-Mono- oder N,N-Diniederalkylcarbamyl, ebenso N-Mononiederalkylcarbamyl $R_2$ in N,N-Diniederalkylcarbamyl überführen, beispielsweise durch Behandeln mit einem reaktiven Ester eines Niederalkanols, wie einem Niederalkylhalogenid, z.B. -bromid oder -iodid, Niederalkylsulfonat, z.B. -methansulfonat oder -p-toluolsulfonat, oder einem Diniederalkylsulfat, z.B. Dimethylsulfat, vorzugsweise unter basischen Bedingungen, wie in Gegenwart von Natriumhydrid oder von Natronlauge oder Kalilauge und eines Phasentransfer-Katalysators, wie Tetrabutylammoniumbromid oder Benzyltrimethylammoniumchlorid.

Ebenso können Verbindungen I, in denen $R_1$ Wasserstoff bedeutet, in analoger Weise zu Verbindungen I, worin $R_1$ Niederalkyl darstellt, N-niederalkyliert werden. Die N-Alkylierung kann auch in der Weise durchgeführt werden, dass wenn man von Verbindungen I ausgeht, worin $R_1$ Wasserstoff bedeutet und $R_2$ unsubstituiertes Carbamyl darstellt, unter den entsprechenden Reaktionsbedingungen neben der Einführung der N-Niederalkylgruppe $R_1$ im gleichen Reaktionsschritt auch eine N-Alkylierung zu N-Mono- oder N,N-Diniederalkylcarbamyl $R_2$ erreicht. Analog kann verfahren werden, wenn man von Verbindungen I ausgeht, worin $R_1$ Wasserstoff bedeutet und $R_2$ N-mononiederalkylsubstituiertes Carbamyl ist. Gemäss dieser Methode der N-Alkylierung erhält man jeweils Verbindungen I, in denen alle im selben Reaktionsschritt eingeführten N-Niederalkylgruppen gleichartig sind.

Weiterhin kann man unsubstituiertes Carbamyl $R_2$ in N-Niederalkanoylcarbamyl überführen, beispielsweise durch Umsetzung mit einer Niederalkansäure, wie Ameisen-, Essig- oder Propionsäure, oder einem Derivat einer solchen Säure, z.B. einem Säurehalogenid, wie Säurechlorid, Ester oder insbesondere einem Anhydrid, z.B. Acetylchlorid oder Acetanhydrid, wobei indes stärker basische Kondensationsmittel, wie Alkalimetallamide oder -alkoholate, z.B. Natriumamid oder Natriummethanolat, oder auch saure Kondensationsmittel, wie Mineralsäuren, z.B. Schwefelsäure, erforderlich sein können. Verbindungen, in denen $R_1$ Wasserstoff ist, werden dabei auch am Atom $N^4$ N-niederalkanoyliert, so dass nach erfolgter Umsetzung in einem zusätzlichen Schritt, z.B. wie unter der Verfahrensvariante d) beschrieben, die Niederalkanoylgruppe wieder vom Atom $N^4$ abgespalten werden muss.

Salze von Verbindungen der Formel I oder gegebenenfalls von deren Tautomeren können in an sich bekannter Weise hergestellt werden. So erhält man beispielsweise Säureadditionssalze von Verbindungen der Formel I durch Behandeln mit einer Säure oder einem geeigneten Ionenaustauscherreagens. Die Säureadditionssalze können in üblicher Weise in die freien Verbindungen überführt werden, z.B. durch Behandeln mit einem geeigneten basischen Mittel.

Je nach Verfahrensweise und Reaktionsbedingungen können die Verbindungen der Formel I mit salzbildenden, insbesondere basischen, Eigenschaften in freier Form oder in Form von Salzen erhalten werden, ebenso gegebenenfalls deren Tautomere.

Infolge der engen Beziehung zwischen den Verbindungen der Formel I in freier Form und in Form ihrer Salze sind im vorausgegangenen und nachfolgend unter der freien Verbindung oder ihren Salzen sinn- und zweckgemäss gegebenenfalls auch die entsprechenden Salze oder die freie Verbindung zu verstehen.

Die neuen Verbindungen der Formel I einschliesslich ihrer Salze von salzbildenden Verbindungen können auch in Form ihrer Hydrate erhalten werden oder andere, beispielsweise zur Kristallisation von in fester Form vorliegenden Verbindungen verwendete, Lösungsmittel einschliessen.

Die neuen Verbindungen der Formel I können, je nach Wahl der Ausgangsstoffe und Arbeitsweisen, in Form eines der möglichen Isomeren oder als Gemisch derselben vorliegen. Dabei sind, in Abhängigkeit von der Molekülsymmetrie, z.B. je nach Anzahl, absoluter und relativer Konfiguration der Chiralitätszentren, wie asymmetrischen C-Atome, als reine Isomere z.B. reine Enantiomere und/oder reine Diastereomere, wie reine cis/trans-Isomere, erhältlich. Entsprechend können als Isomerengemische z.B. Enantiomerengemische, wie Racemate, Diastereomerengemische oder Racematgemische vorliegen.

Erhaltene Diastereomerengemische und Racematgemische können auf Grund der unterschiedlichen physikalischen Eigenschaften der Komponenten nach üblichen physikalischen Trennverfahren, beispielsweise durch Destillation, fraktionierte Kristallisation und/oder Chromatographie, in die Komponenten aufgetrennt

werden.

Erhaltene Enantiomerengemische, z.B. Racemate, lassen sich nach bekannten Methoden in die Enantiomeren zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenäther, wobei nur ein Enantiomeres komplexiert wird, oder durch Ueberführung in diastereomere Salze, z.B. durch Umsetzung eines basischen Endstoffracemats mit einer optisch aktiven Säure, wie Carbonsäure, z.B. Wein- oder Aepfelsäure, oder Sulfonsäure, z.B. Camphersulfonsäure, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man jeweils das wirksamere Stereoisomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates oder Salzes und/oder seiner Racemate bzw. Enantiomeren verwendet oder insbesondere unter den Reaktionsbedingungen bildet.

Neue Ausgangsstoffe, die speziell für die Herstellung der verfahrensgemäss erhältlichen Verbindungen entwickelt wurden, insbesondere die vorzugsweise verwendete zu den eingangs als bevorzugt gekennzeichneten Verbindungen der Formel I führende Ausgangsstoffauswahl, die Verfahren zu ihrer Herstellung sowie ihre Verwendung als Zwischenprodukte bilden ebenfalls einen Gegenstand der Erfindung.

Die Erfindung betrifft ebenfalls die Verwendung von Verbindungen der Formel I sowie gegebenenfalls ihrer Tautomeren und/oder pharmazeutisch verwendbaren Salze, insbesondere als pharmakologische, in erster Linie antikonvulsiv wirksame, Wirksubstanzen. Dabei kann man sie, vorzugsweise in Form pharmazeutischer Präparate, in einem Verfahren zur prophylaktischen und/oder therapeutischen Behandlung des tierischen oder menschlichen Körpers, insbesondere als Antikonvulsiva, z.B. zur Behandlung von Konvulsionen verschiedenartiger Genese, beispielsweise zur Behandlung der Epilepsie, anwenden.

Die Erfindung betrifft gleichfalls pharmazeutische Präparate, die eine Verbindung der Formel I oder gegebenenfalls ein Tautomeres und/oder pharmazeutisch verwendbares Salz davon als Wirkstoff enthalten, sowie Verfahren zu ihrer Herstellung.

Bei diesen pharmazeutischen Präparaten handelt es sich um solche, die eine therapeutisch wirksame Menge des Wirkstoffs, gegebenenfalls zusammen mit anorganischen oder organischen, festen oder flüssigen, pharmazeutisch verwendbaren Hilfsstoffen enthalten, und die sich zur enteralen, z.B. oralen, oder parenteralen Verabreichung an Warmblüter eignen. So verwendet man vorzugsweise pharmazeutische Präparate in Dosiseinheitsformen, wie Dragées, Tabletten, Kapseln oder Suppositorien, ferner Ampullen, welche den Wirkstoff zusammen mit Verdünnungsmitteln, z.B. Laktose, Dextrose, Saccharose, Mannitol, Sorbitol, Cellulose und/oder Glycin, und/oder Schmiermitteln, z.B. Kieselerde, Talk, Stearinsäure oder Salze davon, wie Magnesium- oder Calciumstearat, und/oder Polyäthylenglykol, aufweisen. Tabletten können ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxylmethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, Adsorptionsmittel, Farbstoffe, Geschmacksstoffe und/oder Süssmittel enthalten. Ferner kann man die Verbindungen der Formel I in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden könne. Die pharmazeutischen Präparate können sterilisiert sein und/oder Hilfsstoffe, z.B. Konservier-, Stabilisier-, Netz und/oder Emulgiermittel, Löslichkeitsvermittler, Salze zur Regulierung des osmotischen Druckes und/oder Puffer, enthalten. Die neuen pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs-oder Lyophilisierungsverfahren, hergestellt und enthalten von etwa 0.1 % bis etwa 100 %, insbesondere von etwa 1 % bis etwa 50 %, Lyophilisate bis zu 100 %, des Aktivstoffes.

Die Dosierung des Wirkstoffs kann von verschiedenen Faktoren, wie Applikationsweise, Warmblüter-Spezies, Alter und/oder individuellem Zustand abhängen. Die täglich zu verabreichenden Dosen liegen im Normfall bei oraler Applikation zwischen etwa 5 und etwa 50 mg/kg und für Warmblüter mit einem Gewicht von etwa 70 kg vorzugsweise zwischen etwa 0,5 g und etwa 5,0 g, wobei zur Erreichung der Tagesdosis vorteilhaft mehrere gleiche Teildosen verabreicht werden können.

Die folgenden Beispiele dienen zur Illustration der Erfindung; sie sollen jedoch diese in ihrem Umfang in keiner Weise einschränken. Temperaturen werden in Celsiusgraden angegeben.

Beispiel 1:

320 mg (1,5 mmol) 1-Aethoxy-2-(2,6-difluorphenyl)-1-(N-methylimino)-äthan und 155 mg (1,5 mmol) Oxalsäuremonoamidmonohydrazid werden unter Stickstoffatmosphäre in 5 ml N-Methylmorpholin gelöst und 41 Stunden unter Rückfluss erhitzt. Nach dem Abkühlen wird das Reaktionsgemisch mit 20 ml Dichlormethan verdünnt und nacheinander zweimal mit Wasser, zweimal mit 2N-Salzsäure und nochmals mit Wasser ausgeschüttelt. Die drei zuletzt erhaltenen sauren wässrigen Phasen werden vereinigt, mit konzentrierter Ammoniaklösung alkalisch gestellt und dreimal mit Dichlormethan extrahiert. Die vereinigten organischen Phasen werden über Magnesiumsulfat getrocknet und geeignet. Nach Umkristallisation des Rohprodukts aus Aethanol erhält man 102 mg (27 % d. Th.) des gewünschten 5-(2,6-Difluorbenzyl)-4-methyl-4H-1,2,4-triazol-3-carboxamids, das bei 189-190° schmilzt.

Das 1-Aethoxy-2-(2,6-difluorphenyl)-1-(N-methylimino)-äthan kann man z.B. auf folgende Weise herstellen:

75,3 g (0,44 mol) 2,6-Difluorphenylessigsäure werden in 96 ml (156,5 g; 1,32 mol) Thionylchlorid gelöst, 1 Stunde bei 60° gerührt und anschliessend 2 Stunden unter Rückfluss erhitzt. Danach wird das überschüssige Thionylchlorid abdestilliert und der Rückstand fraktioniert, wobei man 79,5 g (95 % d. Th.) 2,6-Difluorphenylacetylchlorid erhält.

25 ml Methylaminlösung (33 %ig in Aethanol) werden mit 90 ml Dichlormethan verdünnt. Unter Kühlung werden 9,52 g (50 mmol) 2,6-Difluorphenylacetylchlorid, gelöst in 10 ml Dichlormethan, so zugetropft, dass die Temperatur des Reaktionsgemisches 10° nicht übersteigt. Man rührt noch eine weitere Stunde bei 10° nach und extrahiert dann das Reaktionsgemisch dreimal mit Wasser. Die organische Phase wird über Magnesiumsulfat getrocknet und eingeengt. Das Rohprodukt wird aus Dichlormethan/Petroläther umkristallisiert und liefert 8,57 g (92,6 % d. Th.) 2,6-Difluorphenylessigsäuremethylamid vom Smp. 163-164°.

5,55 g (30 mmol) 2,6-Difluorphenylessigsäuremethylamid werden in 70 ml Dichlormethan gelöst, mit 7,4 g (39 mmol) Triäthyloxoniumtetrafluoroborat versetzt und 25 Stunden bei Raumtemperatur gerührt. Anschliessend wird das Reaktionsgemisch nacheinander mit kalter 2N-Natriumcarbonatlösung, Eiswasser und kalter gesättigter Natriumchloridlösung ausgeschüttelt, über Magnesiumsulfat getrocknet und eingeengt. Der Rückstand wird mit einem Gemisch aus Aether und Hexan versetzt, wobei das nicht umgesetzte Edukt ungelöst bleibt und abfiltriert wird. Das Filtrat wird eingeengt und der Kugelrohrdestillation unterworfen. Man erhält 4 g (62,6 % d. Th.) 1-Aethoxy-2-(2,6-difluorphenyl)-1-(N-methylimino)-äthan, das bei 70° (0,02 mm) siedet.

Beispiel 2:

Analog wie in Beispiel 1 beschrieben kann die Umsetzung von 320 mg (1,5 mmol) 1-Aethoxy-2-(2,6-difluorphenyl)-1-(N-methylimino)-äthan und 155 mg (1,5 mmol) Oxalsäuremonoamidmonohydrazid auch in 5 ml N,N-Dimethylformamid als Lösungsmittel durchgeführt werden. Man erhält ebenfalls 102 mg (27 % d. Th.) 5-(2,6-Difluorbenzyl)-4-methyl-4H-1,2,4-triazol-3-carboxamid vom Smp. 186-189°.

Beispiel 3:

In analoger Weise wie in den Beispielen 1 und 2 beschrieben kann man durch Umsetzung von 1-Aethoxy-2-(o-chlorphenyl)-1-(N-methylimino)-äthan [Sdp.: 90° (0,02 mm)] mit Oxalsäuremonoamidmonohydrazid auch das 5-(o-Chlorbenzyl)-4-methyl-4H-1,2,4-triazol-3-carboxamid, das bei 187-187,5° (aus Aethanol) schmilzt, herstellen.

Beispiel 4:

Ein Gemisch aus 1,85 g (14 mmol) Oxalsäuremonoäthylamidmonohydrazid und 3 g (14 mmol) 1-Aethoxy-2-(2,6-difluorphenyl)-1-(N-methylimino)-äthan werden unter Rühren 45 Minuten auf 200° (Badtemperatur) erhitzt. Nach dem Abkühlen wird das Reaktionsgemisch in Dichlormethan gelöst. Nacheinander schüttelt man einmal mit halbkonzentrierter Salzsäure (1 Teil konzentrierte Salszäure/1 Teil Wasser) und zweimal mit 2N-Salzsäure aus. Die vereinigten wässrigen Phasen werden zweimal mit Dichlormethan extrahiert, mit Aktivkohle versetzt und über Hyflo filtriert. Das Filtrat wird mit konzentrierter Ammoniaklösung alkalisch gestellt und dreimal mit Dichlormethan extrahiert. Die vereinigten organischen Phasen werden zweimal mit Wasser gewaschen, über Magnesiumsulfat getrocknet, eingeengt und aus Dichlormethan/Aether kristallisiert. Nach Umkristallisation aus Essigesster/Aether erhält man das 5-(2,6-Difluorbenzyl)-4-methyl-4H-1,2,4-triazol-3-(N-äthyl)carboxamid vom Smp. 144-145°.

Das Oxalsäuremonoäthylamidmonohydrazid lässt sich z.B. folgendermassen herstellen:
7,25 g (50 mmol) Oxalsäuremonoäthylamidmonoäthylester werden in 70 ml Aethanol gelöst. Unter Kühlung werden 2,5 ml (2,6 g; 52 mmol) Hydrazinhydrat, gelöst in 10 ml Aethanol, so zugetropft, dass die Temperatur des Reaktionsgemisches nicht über 10° ansteigt. Man rührt anschiessend eine Stunde bei Raumtemperatur nach und filtriert das ausgefallene Oxalsäuremonoäthylamidmonohydrazid, das bei 161-164° schmilzt, ab.

Beispiel 5:

10 g (36 mmol) 5-(2,6-Difluorbenzyl)-4-methyl-4H-1,2,4-triazol-3-carbonsäureäthylester werden in einem Bombenrohr in 100 ml 4,4 N-ammoniakalischem Aethanol 2 Stunden bei 70° gerührt. Anschliessend wird das Reaktionsgemisch auf das halbe Volumen eingeengt. Die ausgeschiedenen Kristalle werden abfiltriert, aus Aethanol umkristallisiert und liefern das 5-(2,6-Difluorbenzyl)-4-methyl-4H-1,2,4-triazol-3-carboxamid vom Smp. 189-191°.

Der 5-(2,6-Difluorbenzyl)-4-methyl-4H-1,2,4-triazol-3-carbonsäureäthylester kann beispielsweise folgendermassen hergestellt werden:
70,4 g (0,41 mmol) 2,6-Difluorphenylessigsäure, 700 ml Aethanol und 35 ml konzentrierte Schwefelsäure werden 3 Stunden unter Rückfluss erhitzt. Anschliessend wird das Reaktionsgemisch eingedampft, in Dichlormethan aufgenommen und die Lösung nacheinander mit Wasser, 2N-Natriumcarbonatlösung und nochmals mit Wasser ausgeschüttelt. Nach Trocknung über Magnesiumsulfat wird die organische Phase eingedampft. Den Rückstand löst man in 560 ml Aethanol. Nach Zugabe von 30 ml (31 g; 0,62 mol) Hydrazinhydrat wird 4 Tage unter Rückfluss erhitzt. Nach dem Abkühlen filtriert man die ausgefallenen Kristalle ab und erhält so 56,4 g (74 % d. Th.) 2,6-Difluorphenylessigsäurehydrazid, das bei 177-178° schmilzt.

18,6 g (142 mmol) Oxalsäuremonomethylamidmonoäthylester werden unter Stickstoffatmosphäre in 63 ml Trichlormethan gelöst und mit 59 ml einer 20 %igen Lösung von Phosgen in Toluol versetzt. Bei einer Temperatur von 5-10° werden 9,2 ml Pyridin zugetropft. Man rührt anschiessend 2 Stunden bei 0° nach, wobei während der zweiten Stunde Stickstoff durch das Reaktionsgemisch geblasen wird. Anschliessend werden 17,6 g (95 mmol) 2,6-Difluorphenylessigsäurehydrazid zugegeben. Das Reaktionsgemisch wird 40 Minuten bei Raumtemperatur nachgerührt, dann auf Eiswasser gegossen, mit konzentrierter Salzsäure angesäuert und dreimal mit Aether extrahiert. Die saure wässrige Phase wird mit konzentrierter Ammoniaklösung alkalisch gestellt und dreimal mit Dichlormethan ausgeschüttelt. Die vereinigten Dichlormethan-Phasen werden zweimal mit Wasser gewaschen, über Magnesiumsulfat getrocknet und eingedampft. Der Rückstand wird in 160 ml Xylol aufgenommen und 9 Stunden unter Rückfluss gerührt. Anschliessend wird das Reaktionsgemisch eingeengt und an Kieselgel mit Essigester als Laufmittel chromatographiert. Das Eluat wird eingedampft und der kristalline Rückstand aus Essigester/Aether umkristallisiert. Man erhält den 5-(2,6-Difluorbenzyl)-4-methyl-4H-1,2,4-triazol-3-carbonsäureäthylester vom Smp. 127-128°.

Beispiel 6:

In analoger Weise wie in Beispiel 5 beschrieben kann man herstellen:
ausgehend von o-Fluorphenylessigsäurehydrazid (Smp.: 139-143°), Phosgen und Oxalsäuremonomethylamidmonoäthylester den 5-(o-Fluorbenzyl)-4-methyl-4H-1,2,4-triazol-3-carbonsäureäthylester (Smp.: 83-84°) und hieraus das 5-(o-Fluorbenzyl)-4-methyl-4H-1,2,4-triazol-3-carboxamid vom Smp. 165-166° und
ausgehend von m-Trifluormethylphenylessigsäurehydrazid (Smp.: 102-104°), Phosgen und Oxalsäuremonomethylamidmonoäthylester den 4-Methyl-5-(m-trifluormethylbenzyl)-4H-1,2,4-triazol-3-carbonsäureäthylester (Smp.: 64-65°) und hieraus das 4-Methyl-5-(m-trifluormethylbenzyl)-4H-1,2,4-triazol-3-carboxamid vom Smp. 167-168°.

Beispiel 7:

In analoger Weise wie in Beispiel 5 beschrieben kann man, ausgehend von 2,6-Difluorphenylessigsäurehydrazid (Smp.: 177-178°), Phosgen und Oxalsäuremonoäthylamidmonoäthylester, den 4-Aethyl-5-(2,6-difluorbenzyl)-4H-1,2,4-triazol-3-carbonsäureäthylester (Smp.: 120-121°) und hieraus das 4-Aethyl-5-(2,6-difluorbenzyl)-4H-1,2,4-triazol-3-carboxamid vom Smp. 222-224° herstellen.

Beispiel 8:

In analoger Weise wie in Beispiel 5 beschrieben kann man, ausgehend von 5-(2,6-Difluorbenzyl)-4-methyl-4H-1,2,4-triazol-3-carbonsäureäthylester (Smp. 127-128°), auch herstellen:
unter Verwendung von Methylamin anstelle von Ammoniak das 5-(2,6-Difluorbenzyl)-4-methyl-4H-1,2,4-triazol-3-(N-methyl)carboxamid vom Smp. 179-180° und
unter Verwendung von Dimethylamin anstelle von Ammoniak das 5-(2,6-Difluorbenzyl)-4-methyl-4H-1,2,4-triazol-3-(N,N-dimethyl)carboxamid vom Smp. 124-126°.

Beispiel 9:

In analoger Weise wie in Beispiel 5 beschrieben kann man, ausgehend von 5-(o-Chlorbenzyl)-4-methyl-4H-1,2,4-triazol-3-carbonsäureäthylester, das 5-(o-Chlorbenzyl)-4-methyl-4H-1,2,4-triazol-3-carboxamid vom Smp. 187-187,5° herstellen.

Der 5-(o-Chlorbenzyl)-4-methyl-4H-1,2,4-triazol-3-carbonsäureäthylester lässt sich z.B. auf folgende Weise herstellen:
14,8 g (80 mmol) o-Chlorphenylessigsäurehydrazid werden in 230 ml Dichlormethan suspendiert. Bei 5° tropft man 12 g (80 mmol) 2-Chlor-2-(N-methylimino)-essigsäureäthylester [Tetrahedron Lett. 30, 2827 (1979)] zu. Man lässt 2 Stunden unter Eiskühlung und dann über Nacht bei Raumtemperatur nachrühren. Das Reaktionsgemisch wird auf Eis gegossen, mit 50 ml 2N-Salzsäure versetzt und zweimal mit Dichlormethan extrahiert. Die vereinigten Dichlormethan-Phasen werden zweimal mit Wasser gewaschen. Alle sauren wässrigen Phasen werden vereinigt, mit festem Natriumcarbonat alkalisch gestellt und dreimal mit Dichlormethan ausgeschüttelt. Diese drei organischen Phasen werden vereinigt, nacheinander mit Wasser und gesättigter Natriumchloridlösung gewaschen, über Magnesiumsulfat getrocknet und eingedampft. Der Rückstand wird in 150 ml Xylol aufgenommen und 44 Stunden unter Rückfluss gerührt. Danach wird das Reaktionsgemisch eingeengt und an Kieselgel mit Essigester als Laufmittel chromatographiert. Das Eluat wird eingedampft und aus Aether umkristallisiert. Man erhält den 5-(o-Chlorbenzyl)-4-methyl-4H-1,2,4-triazol-3-carbonsäureäthylester vom Smp. 87-88° in einer Ausbeute von 12,4 g (55 % d. Th.).

Beispiel 10:

1,43 g (6 mmol) 5-(2,6-Difluorbenzyl)-3-hydroxymethyl-4-methyl-4H-1,2,4-triazol und 5,22 g (60 mmol) Mangandioxid werden in 40 ml Toluol 3 Std. am Wasserabscheider unter Rückfluss erhitzt. Das Reaktionsgemisch wird sodann abgekühlt, über Hyflo filtriert und das Filtrat eingedampft. Den Rückstand gibt man zu 90 ml mit Ammoniak gesättigtem Isopropanol. Bei 0° werden 1,47 g (30 mmol) Natriumcyanid und 10,44 g (120 mmol) Mangandioxid zugegeben. Man lässt 4 Std. bei 0° rühren und filtriert das Reaktionsgemisch über Hyflo. Das Filtrat wird eingedampft. Nach Umkristallisation des Rückstandes aus Aethanol erhält man das reine 5-(2,6-Difluorbenzyl)-4-methyl-4H-1,2,4-triazol-3-carboxamid.

Das 5-(2,6-Difluorbenzyl)-3-hydroxymethyl-4-methyl-4H-1,2,4-triazol kann man z.B. in analoger Weise wie in Beispiel 1 oder 4 beschrieben durch Umsetzung von 2,13 g (10 mmol) 1-Aethoxy-2-(2,6-difluorphenyl)-1-(N-methylimino)-äthan mit 0,9 g (10 mmol) Hydroxyessigsäurehydrazid herstellen.

Beispiel 11:

In analoger Weise wie in den Beispielen 1 bis 10 beschrieben kann man ebenfalls herstellen:
das 5-(o-Chlorbenzyl)-1,2,4-triazol-3-carboxamid vom. Smp. 208-214°,
das 5-[1-(2,6-Difluorphenyl)äthyl]-4-methyl-4H-1,2,4-triazol-3-carboxamid und
das 5-(2,6-Difluorbenzyl)-4-methyl-4H-1,2,4-triazol-3-(N-acetyl)carboxamid.

Beispiel 12:

Tabletten, enthaltend je 50 mg des Wirkstoffs, z.B. 5-(2,6-Difluorbenzyl)-4-methyl-4H-1,2,4-triazol-3-carboxamid, können z.B. wie folgt hergestellt werden:

Zusammensetzung (für 10000 Tabletten):
Wirkstoff 500.0 g
Lactose 500.0 g
Kartoffelstärke 352.0 g
Gelatine 8.0 g
Talk 60.0 g

Magnesiumstearat 10.0 g
Siliciumdioxid (hochdispers) 20.0 g
Aethanol q.s.

Der Wirkstoff wird mit der Lactose und 292 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesiumstearat und das hochdisperse Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 13:

Lacktabletten, enthaltend je 100 mg des Wirkstoffs, z.B. 5-(2,6-Difluorbenzyl)-4-methyl-4H-1,2,4-triazol-3-carboxamid, können z.B. wie folgt hergestellt werden:

Zusammensetzung (für 1000 Tabletten):
Wirkstoff 100.00 g
Lactose 100.00 g
Maisstärke 70.00 g
Talk 8.50 g
Calciumstearat 1.50 g
Hydroxypropylmethylcellulose 2.36 g
Schellack 0.64 g
Wasser q.s.
Dichlormethan q.s.

Der Wirkstoff, die Lactose und 40 g der Maisstärke werden gemischt und mit einer Kleister, hergestellt aus 15 g Maisstärke und Wasser (unter Erwärmen), befeuchtet und granuliert. Das Granulat wird getrocknet, der Rest der Maisstärke, der Talk und das Calciumstearat werden zugegeben und mit dem Granulat vermischt. Das Gemisch wird zu Tabletten (Gewicht: 280 mg) verpresst und diese mit einer Lösung der Hydroxypropylmethylcellulose und des Schellacks in Dichlormethan lackiert; Endgewicht der Lacktablette: 283 mg.

Beispiel 14:

In analoger Weise wie in den Beispielen 12 und 13 beschrieben können auch pharmazeutische Präparate, enthaltend eine andere Verbindung der Formel I oder gegebenenfalls ein Tautomeres und/oder ein pharmazeutisch verwendbares Salz davon, beispielsweise gemäss den Beispielen 1 bis 11, hergestellt werden.

**Patentansprüche**
**Patentansprüche für folgende Vertragsstaaten : AT, BE, CH, DE, FR, GB, IT, LI, LU, NL, SE**

**1.** Eine Verbindung der Formel

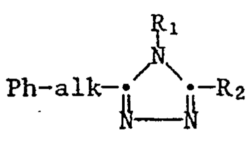 (I),

worin Ph durch $C_1$-$C_7$-Alkyl, Halogen mit einer Atomnummer bis und mit 35 und/oder Trifluormethyl substituiertes Phenyl bedeutet alk $C_1$-$C_4$-Alkyliden darstellt, $R_1$ Wasserstoff oder $C_1$-$C_7$-Alkyl ist und $R_2$ für gegebenenfalls durch $C_1$-$C_7$-Alkyl oder $C_1$-$C_7$-Alkanoyl substituiertes Carbamyl steht, mit der Massgabe, dass in einer Verbindung der Formel I, worin $R_1$ Methyl, $R_2$ N,N-Diäthylcarbamyl und alk Methylen bedeutet, Ph von in p-Stellung durch Chlor substituiertem Phenyl verschieden ist, oder gegebenenfalls ein Tautomeres und/oder Salz davon.

2. Eine Verbindung der Formel I gemäss Anspruch 1, worin Ph durch $C_1$-$C_7$-Alkyl, Halogen mit einer Atomnummer bis und mit 35 und/oder Trifluormethyl mono-, di- oder trisubstituiertes Phenyl bedeutet, alk $C_1$-$C_4$-Alkyliden darstellt, $R_1$ Wasserstoff oder $C_1$-$C_7$-Alkyl ist und $R_2$ Carbamyl, N-$C_1$-$C_7$-Alkylcarbamyl, N,N-Di-$C_1$-$C_4$-alkylcarbamyl oder N-$C_1$-$C_7$-Alkanoylcarbamyl bedeutet, oder gegebenenfalls ein Tautomeres und/oder Salz davon.

3. Eine Verbindung der Formel I gemäss Anspruch 1, worin Ph durch $C_1$-$C_4$-Alkyl, Halogen oder Trifluormethyl monosubstituiertes oder durch Halogen, Halogen sowie $C_1$-$C_4$-Alkyl oder Halogen sowie Trifluormethyl disubstituiertes Phenyl bedeutet, wobei Halogen jeweils eine Atomnummer bis und mit 35 aufweist, alk 1,1- oder 2,2-$C_1$-$C_4$-Alkyliden darstellt, $R_1$ Wasserstoff oder $C_1$-$C_4$-Alkyl ist und $R_2$ Carbamyl, N-$C_1$-$C_4$-Alkylcarbamyl, N,N-Di-$C_1$-$C_4$-alkylcarbamyl oder N-$C_2$-$C_7$-Alkanoylcarbamyl bedeutet, oder gegebenenfalls ein Tautomeres und/oder Salz davon.

4. Eine Verbindung der Formel I gemäss einem der Ansprüche 1 bis 3, worin Ph mindestens einen der Alkyl- und/oder Halogensubstituenten in einer o-Stellung oder einen Trifluormethylsubstituenten in einer m-Stellung trägt, oder gegebenenfalls ein Tautomeres und/oder Salz davon.

5. Eine Verbindung der Formel I gemäss Anspruch 1, worin Ph o-$C_1$-$C_4$-Alkylphenyl, m-Trifluormethylphenyl, o-Halogenphenyl oder 2,3-, 2,4-, 2,5- oder 2,6-Dihalogenphenyl bedeutet, wobei Halogen jeweils eine Atomnummer bis und mit 35 aufweist, alk 1,1-$C_1$-$C_4$-Alkyliden darstellt, $R_1$ Wasserstoff oder $C_1$-$C_4$-Alkyl ist und $R_2$ Carbamyl oder N-$C_1$-$C_4$-Alkylcarbamyl, N,N-Di-$C_1$-$C_4$-alkylcarbamyl oder N-$C_2$-$C_7$-Alkanoylcarbamyl bedeutet, oder gegebenenfalls ein Tautomeres und/oder Salz davon.

6. Eine Verbindung der Formel I gemäss einem der Ansprüche 1 bis 5 mit der Massgabe, dass Ph von durch Halogen mit einer Atomnummer bis und mit 35 monosubstituiertem Phenyl verschieden ist, wenn $R_2$ N,N-Di-$C_1$-$C_4$-alkylcarbamyl, worin die beiden N-Alkylgruppen gleich oder verschieden sind, bedeutet, oder gegebenenfalls ein Tautomeres und/oder Salz davon.

7. Eine Verbindung der Formel I gemäss Anspruch 1, worin Ph m-Trifluormethylphenyl, o-Fluorphenyl oder 2,6-Difluorphenyl bedeutet, alk Methylen darstellt, $R_1$ $C_1$-$C_4$-Alkyl ist und $R_2$ für Carbamyl steht, oder ein Salz davon.

8. Eine Verbindung der Formel I gemäss Anspruch 1, worin Ph m-Trifluormethylphenyl oder 2,6-Difluorphenyl bedeutet, alk Methylen darstellt, $R_1$ $C_1$-$C_4$-Alkyl ist und $R_2$ für Carbamyl steht, oder ein Salz davon.

9. 5-(2,6-Difluorbenzyl)-4-methyl-4H-1,2,4-triazol-3-carboxamid oder ein Salz davon.

10. 5-(o-Chlorbenzyl)-4-methyl-4H-1,2,4-triazol-3-carboxamid, 4-Methyl-5-(m-trifluormethylbenzyl)-4H-1,2,4-triazol-3-carboxamid, 4-Aethyl-5-(2,6-difluorbenzyl)-4H-1,2,4-triazol-3-carboxamid oder 5-(2,6-Difluorbenzyl)-4-methyl-4H-1,2,4-triazol-3-(N-methyl)carboxamid oder jeweils ein Salz davon.

11. 5-(2,6-Difluorbenzyl)-4-methyl-4H-1,2,4-triazol-3-(N-äthyl)carboxamid, 5-(o-Fluorbenzyl)-4-methyl-4H-1,2,4-triazol-3-carboxamid, 5-(2,6-Difluorbenzyl)-4-methyl-4H-1,2,4-triazol-3-(N,N-dimethyl)carboxamid, 5-(o-Chlorbenzyl)-1,2,4-triazol-3-carboxamid, 5-[1-(2,6-Difluorphenyl)äthyl]-4-methyl-4H-1,2,4-triazol-3-carboxamid oder 5-(2,6-Difluorbenzyl)-4-methyl-4H-1,2,4-triazol-3-(N-acetyl)carboxamid oder jeweils ein Salz davon.

12. Eine Verbindung gemäss einem der Ansprüche 1 bis 5 und 9 bis 11 in freier Form.

13. Eine Verbindung der Formel

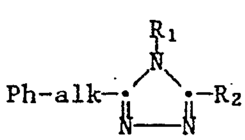

(I),

worin Ph durch $C_1$-$C_7$-Alkyl, Halogen mit einer Atomnummer bis und mit 35 und/oder Trifluormethyl substituiertes Phenyl bedeutet, alk $C_1$-$C_4$-Alkyliden darstellt, $R_1$ Wasserstoff oder $C_1$-$C_7$-Alkyl ist und $R_2$ für gegebenenfalls durch $C_1$-$C_7$-Alkyl oder $C_1$-$C_7$-Alkanoyl substituiertes Carbamyl steht, oder gegebenenfalls ein Tautomeres und/oder ein pharmazeutisch verwendbares Salz davon zur Anwendung in einem Verfahren zur prophylaktischen und/oder therapeutischen Behandlung des tierischen oder menschlichen Körpers.

**14.** Eine Verbindung gemäss Anspruch 13 in freier Form zur Anwendung in einem Verfahren zur prophylaktischen und/oder therapeutischen Behandlung des tierischen oder menschlichen Körpers.

**15.** Eine Verbindung gemäss einem der Ansprüche 1 bis 12 oder gegebenenfalls ein Tautomeres und/oder ein pharmazeutisch verwendbares Salz davon zur Anwendung in einem Verfahren zur prophylaktischen und/oder therapeutischen Behandlung des tierischen oder menschlichen Körpers.

**16.** Eine Verbindung gemäss Anspruch 12 in freier Form zur Anwendung in einem Verfahren zur prophylaktischen und/oder therapeutischen Behandlung des tierischen oder menschlichen Körpers.

**17.** Eine Verbindung der Formel I gemäss Anspruch 13, worin Ph p-Chlorphenyl bedeutet,alk Methylen darstellt, $R_1$ Methyl ist und $R_2$, für N,N-Diäthylcarbamyl steht, oder ein pharmazeutisch verwendbares Salz davon zur Anwendung in einem Verfahren zur prophylaktischen und/oder therapeutischen Behandlung des tierischen oder menschlichen Körpers.

**18.** Eine Verbindung der Formel I gemäss Anspruch 13, worin Ph durch Halogen mit einer Atomnummer bis und mit 35 monosubstituiertes Phenyl bedeutet, alk $C_1$-$C_4$-Alkyliden darstellt, $R_1$ Wasserstoff oder $C_1$-$C_7$-Alkyl ist und $R_2$ N,N-Di-$C_1$-$C_4$-alkylcarbamyl, worin die beiden N-Alkylgruppen gleich oder verschieden sind, bedeutet, oder gegebenenfalls ein Tautomeres und/oder ein pharmazeutisch verwendbares Salz davon zur Anwendung in einem Verfahren zur prophylaktischen und/oder therapeutischen Behandlung des tierischen oder menschlichen Körpers.

**19.** Eine Verbindung gemäss Anspruch 17 oder 18 in freier Form zur Anwendung in einem Verfahren zur prophylaktischen und/oder therapeutischen Behandlung des tierischen oder menschlichen Körpers.

**20.** Ein pharmazeutische Präparat, als Wirkstoff enthaltend eine Verbindung gemäss einem der Ansprüche 1 bis 19 oder gegebenenfalls ein Tautomeres und/oder ein pharmazeutisch verwendbares Salz davon gegebenenfalls neben üblichen pharmazeutischen Hilfsstoffen.

**21.** Ein pharmazeutisches Präparat, als Wirkstoff enthaltend eine Verbindung gemäss einem der Ansprüche 12, 14, 16 und 19 in freier Form gegebenenfalls neben üblichen pharmazeutischen Hilfsstoffen.

**22.** Verfahren zur Herstellung einer Verbindung der Formel

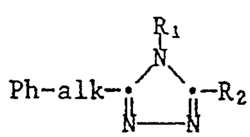

(I),

worin Ph durch $C_1$-$C_7$-Alkyl, Halogen mit einer Atomnummer bis und mit 35 und/oder Trifluormethyl substituiertes Phenyl bedeutet, alk $C_1$-$C_4$-Alkyliden darstellt, $R_1$ Wasserstoff oder $C_1$-$C_7$-Alkyl ist und $R_2$ für gegebenenfalls durch $C_1$-$C_7$-Alkyl oder $C_1$-$C_7$-Alkanoyl substituiertes Carbamyl steht, mit der Massgabe, dass in einer Verbindung der Formel I, worin $R_1$ Methyl, $R_2$ N,N-Diäthylcarbamyl und alk Methylen bedeutet, Ph von in p-Stellung durch Chlor substituierten Phenyl verschieden ist, oder gegebenenfalls eines Tautomeren und/oder Salzes davon, dadurch gekennzeichnet, dass man

a) aus einer Verbindung der Formel

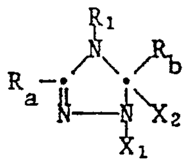

(II),

worin $X_1$ und $X_2$ unter Ausbildung einer zusätzlichen Bindung eliminierbare Gruppen bedeuten und einer der beiden Reste $R_a$ und $R_b$ eine Gruppe Ph-alk und der andere einen Rest $R_2$ darstellt, oder gegebenenfalls einem Tautomeren und/oder Salz davon $X_1$ und $X_2$ eliminiert oder

b) in einer Verbindung der Formel

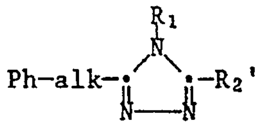

(III),

worin $R_2$' eine in einen Rest $R_2$ überführbare Gruppe bedeutet, oder gegebenenfalls einem Tautomeren und/oder Salz davon $R_2$' in $R_2$ überführt oder

c) zur Herstellung einer Verbindung der Formel I, worin $R_1$ von Wasserstoff verschieden ist und $R_2$ insbesondere N,N-Di-$C_1$-$C_4$-alkylcarbamyl ist, in eine Verbindung der Formel

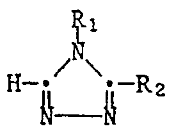

(IV)

oder ein Salz davon den Rest der Formel Ph-alk einführt oder

d) zur Herstellung einer Verbindung der Formel I, worin $R_1$ Wasserstoff ist, in einer Verbindung der Formel

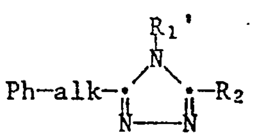

(V),

worin $R_1$' eine durch Wasserstoff ersetzbare Gruppe darstellt, oder einem Salz davon $R_1$' durch Wasserstoff ersetzt oder

e) zur Herstellung einer Verbindung der Formel I, worin $R_2$ N,N-Di-$C_1$-$C_4$-alkylcarbamyl ist, in einer Verbindung der Formel

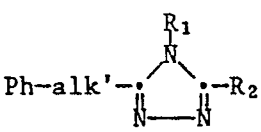

(VI),

worin alk' einen in eine Gruppe alk überführbaren Rest darstellt, oder gegebenenfalls einem Tautomeren und/oder Salz davon alk' in alk überführt und jeweils ein gegebenenfalls verfahrensgemäss erhältliches Isomerengemisch in die Komponenten auftrennt und das Isomere der Formel I isoliert, gewünschtenfalls jeweils eine verfahrensgemäss oder auf andere Weise erhältliche Verbindung der Formel I in eine andere Verbindung der Formel I überführt, jeweils ein gegebenenfalls verfahrensgemäss erhältliches Stereoisomerengemisch in die Stereoisomeren auftrennt und das gewünschte Stereoisomere isoliert und/oder jeweils eine verfahrensgemäss erhältliche freie Verbindung der Formel I in ein Salz überführt oder ein verfahrensgemäss erhältliches Salz in die freie

Verbindung der Formel I oder in ein anderes Salz umwandelt.

**23.** Verfahren gemäss Anspruch 22 zur Herstellung einer Verbindung der Formel I, worin Ph durch $C_1$-$C_7$-Alkyl, Halogen mit einer Atomnummer bis und mit 35 und/oder Trifluormethyl mono-, di- oder trisubstituiertes Phenyl bedeutet, alk $C_1$-$C_4$-Alkyliden darstellt, $R_1$ Wasserstoff oder $C_1$-$C_7$-Alkyl ist und $R_2$ Carbamyl, N-$C_1$-$C_7$-Alkylcarbamyl, N,N-Di-$C_1$-$C_4$-alkylcarbamyl oder N-$C_1$-$C_7$-Alkanoylcarbamyl bedeutet, oder gegebenenfalls eines Tautomeren und/oder Salzes davon.

**24.** Verfahren gemäss Anspruch 22 zur Herstellung einer Verbindung der Formel I, worin Ph durch $C_1$-$C_4$-Alkyl, Halogen oder Trifluormethyl monosubstituiertes oder durch Halogen, Halogen sowie $C_1$-$C_4$-Alkyl oder Halogen sowie Trifluormethyl disubstituiertes Phenyl bedeutet, wobei Halogen jeweils eine Atomnummer bis und mit 35 aufweist, alk 1,1- oder 2,2-$C_1$-$C_4$-Alkyliden darstellt, $R_1$ Wasserstoff oder $C_1$-$C_4$-Alkyl ist und $R_2$ Carbamyl, N-$C_1$-$C_4$-Alkylcarbamyl, N,N-Di-$C_1$-$C_4$-alkylcarbamyl oder N-$C_2$-$C_7$-Alkanoylcarbamyl bedeutet, oder gegebenenfalls eines Tautomeren und/oder Salzes davon.

**25.** Verfahren gemäss Anspruch 22 zur Herstellung einer Verbindung der Formel I, worin Ph, alk, $R_1$ und $R_2$ jeweils eine der in einem der Ansprüche 22 bis 24 beschriebenen Bedeutungen haben und worin Ph mindestens einen der Alkyl- und/oder Halogensubstituenten in einer o-Stellung oder einen Trifluormethylsubstituenten in einer m-Stellung trägt, oder gegebenenfalls eines Tautomeren und/oder Salzes davon.

**26.** Verfahren gemäss Anspruch 22 zur Herstellung einer Verbindung der Formel I, worin Ph o-$C_1$-$C_4$-Alkylphenyl, m-Trifluormethylphenyl, o-Halogenphenyl oder 2,3-, 2,4-, 2,5- oder 2,6-Dihalogenphenyl bedeutet, wobei Halogen jeweils eine Atomnummer bis und mit 35 aufweist, alk 1,1-$C_1$-$C_4$-Alkyliden darstellt, $R_1$ Wasserstoff oder $C_1$-$C_4$-Alkyl ist und $R_2$ Carbamyl oder N-$C_1$-$C_4$-Alkylcarbamyl, N,N-Di-$C_1$-$C_4$-alkylcarbamyl oder N-$C_2$-$C_7$-Alkanoylcarbamyl bedeutet, oder gegebenenfalls eines Tautomeren und/oder Salzes davon.

**27.** Verfahren gemäss Anspruch 22 zur Herstellung einer Verbindung der Formel I, worin Ph, alk, $R_1$ und $R_2$ jeweils eine der in einem der Ansprüche 22 bis 26 beschriebenen Bedeutungen haben, mit der Massgabe, dass Ph von durch Halogen mit einer Atomnummer bis und mit 35 monosubstituiertem Phenyl verschieden ist, wenn $R_2$ N,N-Di-$C_1$-$C_4$-alkylcarbamyl, worin die beiden N-Alkylgruppen gleich oder verschieden sind, bedeutet, oder gegebenenfalls eines Tautomeren und/oder Salzes davon.

**28.** Verfahren gemäss Anspruch 22 zur Herstellung einer Verbindung der Formel I, worin Ph m-Trifluormethylphenyl, o-Fluorphenyl oder 2,6-Difluorphenyl bedeutet, alk Methylen darstellt, $R_1$ $C_1$-$C_4$-Alkyl ist und $R_2$ für Carbamyl steht, oder eines Salzes davon.

**29.** Verfahren gemäss Anspruch 22 zur Herstellung einer Verbindung der Formel I, worin Ph m-Trifluormethylphenyl oder 2,6-Difluorphenyl bedeutet, alk Methylen darstellt, $R_1$ $C_1$-$C_4$-Alkyl ist und $R_2$ für Carbamyl steht, oder eines Salzes davon.

**30.** Verfahren gemäss Anspruch 22 zur Herstellung von 5-(2,6-Difluorbenzyl)-4-methyl-4H-1,2,4-triazol-3-carboxamid oder eines Salzes davon.

**31.** Verfahren gemäss Anspruch 22 zur Herstellung von 5-(o-Chlorbenzyl)-4-methyl-4H-1,2,4-triazol-3-carboxamid, 4-Methyl-5-(m-trifluormethylbenzyl)-4H-1,2,4-triazol-3-carboxamid, 4-Aethyl-5-(2,6-difluorbenzyl)-4H-1,2,4-triazol-3-carboxamid oder 5-(2,6-Difluorbenzyl)-4-methyl-4H-1,2,4-triazol-3-(N-methyl)-carboxamid oder jeweils eines Salzes davon.

**32.** Verfahren gemäss Anspruch 22 zur Herstellung von 5-(2,6-Difluorbenzyl)-4-methyl-4H-1,2,4-triazol-3-(N-äthyl)carboxamid, 5-(o-Fluorbenzyl)-4-methyl-4H-1,2,4-triazol-3-carboxamid, 5-(2,6-Difluorbenzyl)-4-methyl-4H-1,2,4-triazol-3-(N,N-dimethyl)carboxamid, 5-(o-Chlorbenzyl)-1,2,4-triazol-3-carboxamid, 5-[1-(2,6-Difluorphenyl)äthyl]-4-methyl-4H-1,2,4-triazol-3-carboxamid oder 5-(2,6-Difluorbenzyl)-4-methyl-4H-1,2,4-triazol-3-(N-acetyl)carboxamid oder jeweils eines Salzes davon.

**33.** Verfahren gemäss Anspruch 22 zur Herstellung einer Verbindung der Formel I, worin Ph, alk, $R_1$ und $R_2$ jeweils eine der in einem der Ansprüche 22 bis 26 beschriebenen Bedeutungen haben, in freier

Form bzw. Verfahren gemäss Anspruch 22 zur Herstellung einer der in einem der Ansprüche 30 bis 32 beschriebenen Verbindungen in freier Form.

**34.** Verfahren zur Herstellung eines pharmazeutischen Präparates, dadurch gekennzeichnet, dass man
a) aus einer Verbindung der Formel

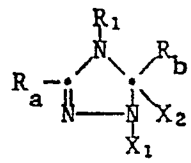

(II),

worin $X_1$ und $X_2$ unter Ausbildung einer zusätzlichen Bindung eliminierbare Gruppen bedeuten und einer der beiden Reste $R_a$ und $R_b$ eine Gruppe Ph-alk und der andere einen Rest $R_2$ darstellt, oder gegebenenfalls einem Tautomeren und/oder Salz davon $X_1$ und $X_2$ eliminiert oder
b) in einer Verbindung der Formel

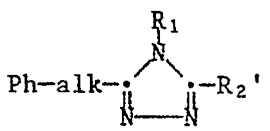

(III),

worin $R_2$' eine in einen Rest $R_2$ überführbare Gruppe bedeutet, oder gegebenenfalls einem Tautomeren und/oder Salz davon $R_2$' in $R_2$ überführt oder
c) zur Herstellung einer Verbindung der Formel I, worin $R_1$ von Wasserstoff verschieden ist und $R_2$, insbesondere N,N-Di-$C_1$-$C_4$-alkylcarbamyl ist, in eine Verbindung der Formel

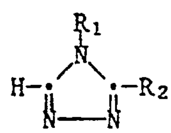

(IV)

oder ein Salz davon den Rest der Formel Ph-alk einführt oder
d) zur Herstellung einer Verbindung der Formel I, worin $R_1$ Wasserstoff ist, in einer Verbindung der Formel

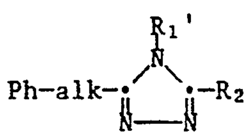

(V),

worin $R_1$' eine durch Wasserstoff ersetzbare Gruppe darstellt, oder einem Salz davon $R_1$' durch Wasserstoff ersetzt oder
e) zur Herstellung einer Verbindung der Formel I, worin $R_2$ N,N-Di-$C_1$-$C_4$-alkylcarbamyl ist, in einer Verbindung der Formel

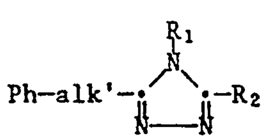

(VI),

worin alk' einen in eine Gruppe alk überführbaren Rest darstellt, oder gegebenenfalls einem

Tautomeren und/oder Salz davon alk' in alk überführt und jeweils ein gegebenenfalls verfahrensgemäss erhältliches Isomerengemisch in die Komponenten auftrennt und das Isomere der Formel I isoliert, gewünschtenfalls jeweils eine verfahrensgemäss oder auf andere Weise erhältliche Verbindung der Formel I in eine andere Verbindung der Formel I überführt, jeweils ein gegebenenfalls verfahrensgemäss erhältliches Stereoisomerengemisch in die Stereoisomeren auftrennt und das gewünschte Stereoisomere isoliert und/oder jeweils eine verfahrensgemäss erhältliche freie Verbindung der Formel I in ein Salz überführt oder ein verfahrensgemäss erhältliches Salz in die freie Verbindung der Formel I oder in ein anderes Salz umwandelt und eine jeweils erhaltene Verbindung der Formel

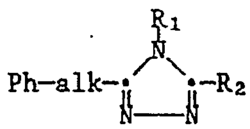 (I),

worin Ph durch $C_1$-$C_7$-Alkyl, Halogen mit einer Atomnummer bis und mit 35 und/oder Trifluormethyl substituiertes Phenyl bedeutet, alk $C_1$-$C_4$-Alkyliden darstellt, $R_1$ Wasserstoff oder $C_1$-$C_7$-Alkyl ist und $R_2$ für gegebenenfalls durch $C_1$-$C_7$-Alkyl oder $C_1$-$C_7$-Alkanoyl substituiertes Carbamyl steht, oder ein gegebenenfalls erhaltenes Tautomeres und/oder ein jeweils erhaltenes pharmazeutisch verwendbares Salz davon mit üblichen pharmazeutischen Hilfsstoffen vermischt.

**35.** Verfahren gemäss Anspruch 34 zur Herstellung eines pharmazeutischen Präparates, dadurch gekennzeichnet, dass man eine gemäss dem in Anspruch 34 beschriebenen Verfahren erhaltene Verbindung der Formel I, worin Ph durch $C_1$-$C_7$-Alkyl, Halogen mit einer Atomnummer bis und mit 35 und/oder Trifluormethyl substituiertes Phenyl bedeutet, alk $C_1$-$C_4$-Alkyliden darstellt, $R_1$ Wasserstoff oder $C_1$-$C_7$-Alkyl ist und $R_2$ für gegebenenfalls durch $C_1$-$C_7$-Alkyl oder $C_1$-$C_7$-Alkanoyl substituiertes Carbamyl steht, in freier Form mit üblichen pharmazeutischen Hilfsstoffen vermischt.

**36.** Verfahren zur Herstellung eines pharmazeutischen Präparates, dadurch gekennzeichnet, dass man eine Verbindung, erhältlich gemäss einem der Ansprüche 22 bis 33, oder gegebenenfalls ein Tautomeres und/oder ein pharmazeutisch verwendbares Salz davon mit üblichen pharmazeutischen Hilfsstoffen vermischt.

**37.** Verfahren gemäss Anspruch 36 zur Herstellung eines pharmazeutischen Präparates, dadurch gekennzeichnet, dass man eine Verbindung, erhältlich gemäss Anspruch 33, in freier Form mit üblichen pharmazeutischen Hilfsstoffen vermischt.

**38.** Verfahren gemäss Anspruch 34 zur Herstellung eines pharmazeutischen Präparates, dadurch gekennzeichnet, dass man eine gemäss dem in Anspruch 34 beschriebenen Verfahren erhaltene Verbindung der Formel I, worin Ph p-Chlorphenyl bedeutet, alk Methylen darstellt, $R_1$ Methyl ist und $R_2$ für N,N-Diäthylcarbamyl steht, oder ein pharmazeutisch verwendbares Salz davon mit üblichen pharmazeutischen Hilfsstoffen vermischt.

**39.** Verfahren gemäss Anspruch 34 zur Herstellung eines pharmazeutischen Präparates, dadurch gekennzeichnet, dass man eine gemäss dem in Anspruch 34 beschriebenen Verfahren erhaltene Verbindung der Formel I, worin Ph durch Halogen mit einer Atomnummer bis und mit 35 monosubstituiertes Phenyl bedeutet, alk $C_1$-$C_4$-Alkyliden darstellt, $R_1$ Wasserstoff oder $C_1$-$C_7$-Alkyl ist und $R_2$ N,N-Di-$C_1$-$C_4$-alkylcarbamyl,worin die beiden N-Alkylgruppen gleich oder verschieden sind, bedeutet, oder gegebenenfalls ein Tautomeres und/oder ein pharmazeutisch verwendbares Salz davon mit üblichen pharmazeutischen Hilfsstoffen vermischt.

**40.** Verfahren gemäss Anspruch 34 zur Herstellung eines pharmazeutischen Präparates, dadurch gekennzeichnet, dass man eine der in Anspruch 38 oder 39 beschriebenen Verbindungen der Formel I in freier Form mit üblichen pharmazeutischen Hilfsstoffen vermischt.

**41.** Verwendung einer Verbindung der Formel I, erhältlich gemäss einem der Ansprüche 22 bis 33, bzw. Verwendung einer der in einem der Ansprüche 38 bis 40 beschriebenen Verbindungen der Formel I

bzw. Verwendung einer Verbindung der Formel I gemäß einem der Ansprüche 1 bis 19 oder jeweils gegebenenfalls eines Tautomeren und/oder eines pharmazeutisch verwendbaren Salzes davon zur Herstellung eines pharmazeutischen Präparates, z.B. eines Antikonvulsivums.

**Patentansprüche für folgende Vertragsstaaten : ES, GR**

1. Verfahren zur Herstellung einer Verbindung der Formel

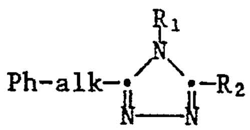

(I),

worin Ph durch $C_1$-$C_7$-Alkyl, Halogen mit einer Atomnummer bis und mit 35 und/oder Trifluormethyl substituiertes Phenyl bedeutet, alk $C_1$-$C_4$-Alkyliden darstellt, $R_1$ Wasserstoff oder $C_1$-$C_7$-Alkyl ist und $R_2$ für gegebenenfalls durch $C_1$-$C_7$-Alkyl oder $C_1$-$C_7$-Alkanoyl substituiertes Carbamyl steht, mit der Massgabe, dass in einer Verbindung der Formel I, worin $R_1$ Methyl, $R_2$ N,N-Diäthylcarbamyl und alk Methylen bedeutet, Ph von in p-Stellung durch Chlor substituiertem Phenyl verschieden ist, oder gegebenenfalls eines Tautomeren und/oder Salzes davon, dadurch gekennzeichnet, dass man

a) aus einer Verbindung der Formel

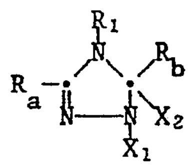

(II),

worin $X_1$ und $X_2$ unter Ausbildung einer zusätzlichen Bindung eliminierbare Gruppen bedeuten und einer der beiden Reste $R_a$ und $R_b$ eine Gruppe Ph-alk und der andere einen Rest $R_2$ darstellt, oder gegebenenfalls einem Tautomeren und/oder Salz davon $X_1$ und $X_2$ eliminiert oder

b) in einer Verbindung der Formel

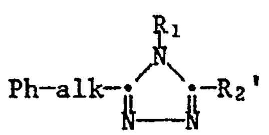

(III),

worin $R_2$' eine in einen Rest $R_2$ überführbare Gruppe bedeutet, oder gegebenenfalls einem Tautomeren und/oder Salz davon $R_2$' in $R_2$ überführt oder

c) zur Herstellung einer Verbindung der Formel I, worin $R_1$ von Wasserstoff verschieden ist und $R_2$ insbesondere N,N-Di-$C_1$-$C_4$-alkylcarbamyl ist, in eine Verbindung der Formel

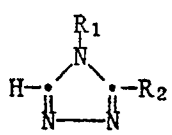

(IV)

oder ein Salz davon den Rest der Formel Ph-alk einführt oder

d) zur Herstellung einer Verbindung der Formel I, worin $R_1$ Wasserstoff ist, in einer Verbindung der Formel

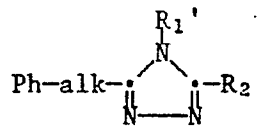

(V),

worin $R_1$' eine durch Wasserstoff ersetzbare Gruppe darstellt, oder einem Salz davon $R_1$' durch Wasserstoff ersetzt oder

e) zur Herstellung einer Verbindung der Formel I, worin $R_2$ N,N-Di-$C_1$-$C_4$-alkylcarbamyl ist, in einer Verbindung der Formel

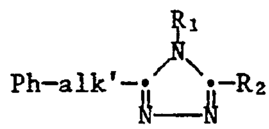

(VI),

worin alk' einen in eine Gruppe alk überführbaren Rest darstellt, oder gegebenenfalls einem Tautomeren und/oder Salz davon alk' in alk überführt und jeweils ein gegebenenfalls verfahrensgemäss erhältliches Isomerengemisch in die Komponenten auftrennt und das Isomere der Formel I isoliert, gewünschtenfalls jeweils ein verfahrensgemäss oder auf andere Weise erhältliche Verbindung der Formel I in eine andere Verbindung der Formel I überführt, jeweils ein gegebenenfalls verfahrensgemäss erhältliches Stereoisomerengemisch in die Stereoisomeren auftrennt und das gewünschte Stereoisomere isoliert und/oder jeweils eine verfahrensgemäss erhältliche freie Verbindung der Formel I in ein Salz überführt oder ein verfahrensgemäss erhältliches Salz in die freie Verbindung der Formel I oder in ein anderes Salz umwandelt.

2. Verfahren gemäss Anspruch 1 zur Herstellung einer Verbindung der Formel I, worin Ph durch $C_1$-$C_7$-Alkyl, Halogen mit einer Atomnummer bis und mit 35 und/oder Trifluormethyl mono-, di- oder trisubstituiertes Phenyl bedeutet, alk $C_1$-$C_4$-Alkyliden darstellt, $R_1$ Wasserstoff oder $C_1$-$C_7$-Alkyl ist und $R_2$ Carbamyl, N-$C_1$-$C_7$-Alkylcarbamyl, N,N-Di-$C_1$-$C_4$-alkylcarbamyl oder N-$C_1$-$C_7$-Alkanoylcarbamyl bedeutet, oder gegebenenfalls eines Tautomeren und/oder Salzes davon.

3. Verfahren gemäss Anspruch 1 zur Herstellung einer Verbindung der Formel I, worin Ph durch $C_1$-$C_4$-Alkyl, Halogen oder Trifluormethyl monosubstituiertes oder durch Halogen, Halogen sowie $C_1$-$C_4$-Alkyl oder Halogen sowie Trifluormethyl disubstituiertes Phenyl bedeutet, wobei Halogen jeweils eine Atomnummer bis und mit 35 aufweist, alk 1,1- oder 2,2-$C_1$-$C_4$-Alkyliden darstellt, $R_1$ Wasserstoff oder $C_1$-$C_4$-Alkyl ist und $R_2$ Carbamyl, N-$C_1$-$C_4$-Alkylcarbamyl, N,N-Di-$C_1$-$C_4$-alkylcarbamyl oder N-$C_2$-$C_7$-Alkanoylcarbamyl bedeutet, oder gegebenenfalls eines Tautomeren und/oder Salzes davon.

4. Verfahren gemäss Anspruch 1 zur Herstellung einer Verbindung der Formel I, worin Ph, alk, $R_1$ und $R_2$ jeweils eine der in einem der Ansprüche 1 bis 3 beschriebenen Bedeutungen haben und worin Ph mindestens einen der Alkyl- und/oder Halogensubstituenten in einer o-Stellung oder einen Trifluormethylsubstituenten in einer m-Stellung trägt, oder gegebenenfalls eines Tautomeren und/oder Salzes davon.

5. Verfahren gemäss Anspruch 1 zur Herstellung einer Verbindung der Formel I, worin Ph o-$C_1$-$C_4$-Alkylphenyl, m-Trifluormethylphenyl, o-Halogenphenyl oder 2,3-, 2,4-, 2,5- oder 2,6-Dihalogenphenyl bedeutet, wobei Halogen jeweils eine Atomnummer bis und mit 35 aufweist, alk 1,1-$C_1$-$C_4$-Alkyliden darstellt, $R_1$ Wasserstoff oder $C_1$-$C_4$-Alkyl ist und $R_2$ Carbamyl oder N-$C_1$-$C_4$-Alkylcarbamyl, N,N-Di-$C_1$-$C_4$-alkylcarbamyl oder N-$C_2$-$C_7$-Alkanoylcarbamyl bedeutet, oder gegebenenfalls eines Tautomeren und/oder Salzes davon.

6. Verfahren gemäss Anspruch 1 zur Herstellung einer Verbindung der Formel I, worin Ph, alk, $R_1$ und $R_2$ jeweils eine der in einem der Ansprüche 1 bis 5 beschriebenen Bedeutungen haben, mit der Massgabe, dass Ph von durch Halogen mit einer Atomnummer bis und mit 35 monosubstituiertem Phenyl verschieden ist, wenn $R_2$ N,N-Di-$C_1$-$C_4$-alkylcarbamyl, worin die beiden N-Alkylgruppen gleich oder verschieden sind, bedeutet, oder gegebenenfalls eines Tautomeren und/oder Salzes davon.

7. Verfahren gemäss Anspruch 1 zur Herstellung einer Verbindung der Formel I, worin Ph m-Trifluormethylphenyl, o-Fluorphenyl oder 2,6-Difluorphenyl bedeutet, alk Methylen darstellt, $R_1$ $C_1$-$C_4$-Alkyl ist und $R_2$ für Carbamyl steht, oder eines Salzes davon.

8. Verfahren gemäss Anspruch 1 zur Herstellung einer Verbindung der Formel I, worin Ph m-Trifluormethylphenyl oder 2,6-Difluorphenyl bedeutet, alk Methylen darstellt, $R_1$ $C_1$-$C_4$-Alkyl ist und $R_2$ für Carbamyl steht, oder eines Salzes davon.

9. Verfahren gemäss Anspruch 1 zur Herstellung von 5-(2,6-Difluorbenzyl)-4-methyl-4H-1,2,4-triazol-3-carboxamid oder eines Salzes davon.

10. Verfahren gemäss Anspruch 1 zur Herstellung von 5-(o-Chlorbenzyl)-4-methyl-4H-1,2,4-triazol-3-carboxamid, 4-Methyl-5-(m-trifluormethylbenzyl)-4H-1,2,4-triazol-3-carboxamid, 4-Aethyl-5-(2,6-difluorbenzyl)-4H-1,2,4-triazol-3-carboxamid oder 5-(2,6-Difluorbenzyl)-4-methyl-4H-1,2,4-triazol-3-(N-methyl)-carboxamid oder jeweils eines Salzes davon.

11. Verfahren gemäss Anspruch 1 zur Herstellung von 5-(2,6-Difluorbenzyl)-4-methyl-4H-1,2,4-triazol-3-(N-äthyl)carboxamid, 5-(o-Fluorbenzyl)-4-methyl-4H-1,2,4-triazol-3-carboxamid, 5-(2,6-Difluorbenzyl)-4-methyl-4H-1,2,4-triazol-3-(N,N-dimethyl)carboxamid, 5-(o-Chlorbenzyl)-1,2,4-triazol-3-carboxamid, 5-[1-(2,6-Difluorphenyl)äthyl]-4-methyl-4H-1,2,4-triazol-3-carboxamid oder 5-(2,6-Difluorbenzyl)-4-methyl-4H-1,2,4-triazol-3-(N-acetyl)carboxamid oder jeweils eines Salzes davon.

12. Verfahren gemäss Anspruch 1 zur Herstellung einer Verbindung der Formel I, worin Ph, alk, $R_1$ und $R_2$ jeweils eine der in einem der Ansprüche 1 bis 8 beschriebenen Bedeutungen haben, in freier Form bzw. Verfahren gemäss Anspruch 1 zur Herstellung einer der in einem der Ansprüche 9 bis 11 beschriebenen Verbindungen in freier Form.

13. Verfahren gemäss Anspruch 1 zur Herstellung einer Verbindung der Formel I, worin Ph, alk, $R_1$ und $R_2$ jeweils eine der in einem der Ansprüche 1 bis 5 beschriebenen Bedeutungen haben, in freier Form bzw. Verfahren gemäss Anspruch 1 zur Herstellung einer der in einem der Ansprüche 9 bis 11 beschriebenen Verbindungen in freier Form.

14. Verfahren zur Herstellung eines pharmazeutischen Präparates, dadurch gekennzeichnet, dass man
a) aus einer Verbindung der Formel

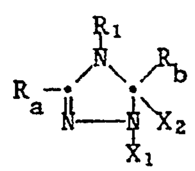

(II),

worin $X_1$ und $X_2$ unter Ausbildung einer zusätzlichen Bindung eliminierbare Gruppen bedeuten und einer der beiden Reste $R_a$ und $R_b$ eine Gruppe Ph-alk und der andere einen Rest $R_2$ darstellt, oder gegebenenfalls einem Tautomeren und/oder Salz davon $X_1$ und $X_2$ eliminiert oder
b) in einer Verbindung der Formel

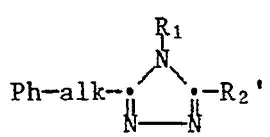

(III),

worin $R_2$' eine in einen Rest $R_2$, überführbare Gruppe bedeutet, oder gegebenenfalls einem Tautomeren und/oder Salz davon $R_2$' in $R_2$ überführt oder
c) zur Herstellung einer Verbindung der Formel I, worin $R_1$ von Wasserstoff verschieden ist und $R_2$, insbesondere N,N-Di-$C_1$-$C_4$-alkylcarbamyl ist, in eine Verbindung der Formel

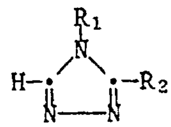 (IV)

oder ein Salz davon den Rest der Formel Ph-alk einführt oder

d) zur Herstellung einer Verbindung der Formel I, worin $R_1$ Wasserstoff ist, in einer Verbindung der Formel

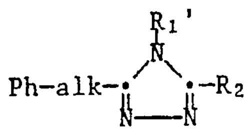 (V),

worin $R_1'$ eine durch Wasserstoff ersetzbare Gruppe darstellt, oder einem Salz davon $R_1'$ durch Wasserstoff ersetzt oder

e) zur Herstellung einer Verbindung der Formel I, worin $R_2$ N,N-Di-$C_1$-$C_4$-alkylcarbamyl ist, in einer Verbindung der Formel

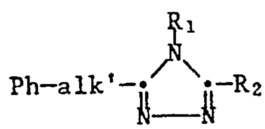 (VI),

worin alk' einen in eine Gruppe alk überführbaren Rest darstellt, oder gegebenenfalls einem Tautomeren und/oder Salz davon alk' in alk überführt und jeweils ein gegebenenfalls verfahrensgemäss erhältliches Isomerengemisch in die Komponenten auftrennt und das Isomere der Formel I isoliert, gewünschtenfalls jeweils eine verfahrensgemäss oder auf andere Weise erhältliche Verbindung der Formel I in eine andere Verbindung der Formel I überführt, jeweils ein gegebenenfalls verfahrensgemäss erhältliches Stereoisomerengemisch in die Stereoisomeren auftrennt und das gewünschte Stereoisomere isoliert und/oder jeweils eine verfahrensgemäss erhältliche freie Verbindung der Formel I in ein Salz überführt oder ein verfahrensgemäss erhältliches Salz in die freie Verbindung der Formel I oder in ein anderes Salz umwandelt und eine jeweils erhaltene Verbindung der Formel

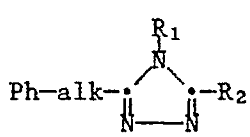 (I),

worin Ph durch $C_1$-$C_7$-Alkyl, Halogen mit einer Atomnummer bis und mit 35 und/oder Trifluormethyl substituiertes Phenyl bedeutet, alk $C_1$-$C_4$-Alkyliden darstellt, $R_1$ Wasserstoff oder $C_1$-$C_7$-Alkyl ist und $R_2$ für gegebenenfalls durch $C_1$-$C_7$-Alkyl oder $C_1$-$C_7$-Alkanoyl substituiertes Carbamyl steht, oder ein gegebenenfalls erhaltenes Tautomeres und/oder ein jeweils erhaltenes pharmazeutisch verwendbares Salz davon mit üblichen pharmazeutischen Hilfsstoffen vermischt.

**15.** Verfahren gemäss Anspruch 14 zur Herstellung eines pharmazeutischen Präparates, dadurch gekennzeichnet, dass man eine gemäss dem in Anspruch 14 beschriebenen Verfahren erhaltene Verbindung der Formel I, worin Ph durch $C_1$-$C_7$-Alkyl, Halogen mit einer Atomnummer bis und mit 35 und/oder Trifluormethyl substituiertes Phenyl bedeutet, alk $C_1$-$C_4$-Alkyliden darstellt, $R_1$ Wasserstoff oder $C_1$-$C_7$-Alkyl ist und $R_2$ für gegebenenfalls durch $C_1$-$C_7$-Alkyl oder $C_1$-$C_7$-Alkanoyl substituiertes Carbamyl steht, in freier Form mit üblichen pharmazeutischen Hilfsstoffen vermischt.

**16.** Verfahren zur Herstellung eines pharmazeutischen Präparates, dadurch gekennzeichnet, dass man eine

Verbindung, erhältlich gemäss einem der Ansprüche 1 bis 13, oder gegebenenfalls ein Tautomeres und/oder ein pharmazeutisch verwendbares Salz davon mit üblichen pharmazeutischen Hilfsstoffen vermischt.

17. Verfahren gemäss Anspruch 16 zur Herstellung eines pharmazeutischen Präparates, dadurch gekennzeichnet, dass man eine Verbindung, erhältlich gemäss Anspruch 13, in freier Form mit üblichen pharmazeutischen Hilfsstoffen vermischt.

18. Verfahren gemäss Anspruch 14 zur Herstellung eines pharmazeutischen Präparates, dadurch gekennzeichnet, dass man eine gemäss dem in Anspruch 14 beschriebenen Verfahren erhaltene Verbindung der Formel I, worin Ph p-Chlorphenyl bedeutet, alk Methylen darstellt, $R_1$ Methyl ist und R für N,N-Diäthylcarbamyl steht, oder ein pharmazeutisch verwendbares Salz davon mit üblichen pharmazeutischen Hilfsstoffen vermischt.

19. Verfahren gemäss Anspruch 14 zur Herstellung eines pharmazeutischen Präparates, dadurch gekennzeichnet, dass man eine gemäss dem in Anspruch 14 beschriebenen Verfahren erhaltene Verbindung der Formel I, worin Ph durch Halogen mit einer Atomnummer bis und mit 35 monosubstituiertes Phenyl bedeutet, alk $C_1$-$C_4$-Alkyliden darstellt, $R_1$ Wasserstoff oder $C_1$-$C_7$-Alkyl ist und $R_2$ N,N-Di-$C_1$-$C_4$-alkylcarbamyl,worin die beiden N-Alkylgruppen gleich oder verschieden sind, bedeutet, oder gegebenenfalls ein Tautomeres und/oder ein pharmazeutisch verwendbares Salz davon mit üblichen pharmazeutischen Hilfsstoffen vermischt.

20. Verfahren gemäss Anspruch 14 zur Herstellung eines pharmazeutischen Präparates, dadurch gekennzeichnet, dass man eine der in Anspruch 18 oder 19 beschriebenen Verbindungen der Formel I in freier Form mit üblichen pharmazeutischen Hilfsstoffen vermischt.

21. Verwendung einer Verbindung der Formel I, erhältlich gemäss einem der Ansprüche 1 bis 13, bzw. Verwendung einer der in einem der Ansprüche 18 bis 20 beschriebenen Verbindungen der Formel I oder jeweils gegebenenfalls eines Tautomeren und/oder eines pharmazeutisch verwendbaren Salzes davon zur Herstellung eines pharmazeutischen Präparates, z.B. eines Antikonvulsivums.

## Claims

**Claims for the following Contracting States : AT, BE, CH, DE, FR, GB, IT, LI, LU, NL, SE**

1. A compound of the formula

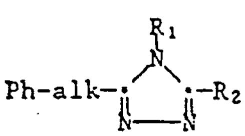

(I)

in which Ph represents phenyl substituted by $C_1$-$C_7$alkyl, halogen having an atomic number of up to and including 35 and/or by trifluoromethyl, alk represents $C_1$-$C_4$-alkylidene, $R_1$ is hydrogen or $C_1$-$C_7$alkyl, and $R_2$ represents carbamoyl that is unsubstituted or substituted by $C_1$-$C_7$alkyl or by $C_1$-$C_7$alkanoyl, with the proviso that, in a compound of the formula I in which $R_1$ represents methyl, $R_2$ represents N,N-diethylcarbamoyl and alk represents methylene, Ph is other than phenyl substituted in the p-position by chlorine, or, as the case may be, a tautomer and/or salt thereof.

2. A compound of the formula I according to claim 1, in which Ph represents phenyl mono-, di- or tri-substituted by $C_1$-$C_7$alkyl, halogen having an atomic number of up to and including 35 and/or by trifluoromethyl, alk represents $C_1$-$C_4$alkylidene, $R_1$ is hydrogen or $C_1$-$C_7$alkyl, and $R_2$ represents carbamoyl, N-$C_1$-$C_7$alkylcarbamoyl, N,N-di-$C_1$-$C_4$alkylcarbamoyl or N-$C_1$-$C_7$alkanoylcarbamoyl, or, as the case may be, a tautomer and/or salt thereof.

3. A compound of the formula I according to claim 1, in which Ph represents phenyl mono-substituted by $C_1$-$C_4$-alkyl, halogen or by trifluoromethyl or di-substituted by halogen, by halogen and $C_1$-$C_4$alkyl, or by halogen and trifluoromethyl, wherein halogen in each case has an atomic number of up to and

including 35, alk represents 1,1- or 2,2-$C_1$-$C_4$alkylidene, $R_1$ is hydrogen or $C_1$-$C_4$-alkyl, and $R_2$ represents carbamoyl, N-$C_1$-$C_4$alkylcarbamoyl, N,N-di-$C_1$-$C_4$alkylcarbamoyl or N-$C_2$-$C_7$alkanoylcarbamoyl, or, as the case may be, a tautomer and/or salt thereof.

**4.** A compound of the formula I according to any one of claims 1 to 3, in which Ph carries at least one of the alkyl and/or halogen substituents in an o-position or a trifluoromethyl substituent in a m-position, or, as the case may be, a tautomer and/or salt thereof.

**5.** A compound of the formula I according to claim 1, in which Ph represents o-$C_1$-$C_4$alkylphenyl, m-trifluoromethylphenyl, o-halophenyl or 2,3-, 2,4-, 2,5- or 2,6-dihalophenyl, wherein halogen in each case has an atomic number of up to and including 35, alk represents 1,1-$C_1$-$C_4$alkylidene, $R_1$ is hydrogen or $C_1$-$C_4$alkyl, and $R_2$ represents carbamoyl or N-$C_1$-$C_4$alkylcarbamoyl, N,N-di-$C_1$-$C_4$alkylcarbamoyl or N-$C_2$-$C_7$alkanoylcarbamoyl, or, as the case may be, a tautomer and/or salt thereof.

**6.** A compound of the formula I according to any one of claims 1 to 5 with the proviso that Ph is other than phenyl mono-substituted by halogen having an atomic number of up to and including 35 when $R_2$ represents N,N-di-$C_1$-$C_4$alkylcarbamoyl in which the two N-alkyl groups are identical or different, or, as the case may be, a tautomer and/or salt thereof.

**7.** A compound of the formula I according to claim 1, in which Ph represents m-trifluoromethylphenyl, o-fluorophenyl or 2,6-difluorophenyl, alk represents methylene, $R_1$ is $C_1$-$C_4$alkyl, and $R_2$ represents carbamoyl, or a salt thereof.

**8.** A compound of the formula I according to claim 1, in which Ph represents m-trifluoromethylphenyl or 2,6-difluorophenyl, alk represents methylene, $R_1$ is $C_1$-$C_4$-alkyl, and $R_2$ represents carbamoyl, or a salt thereof.

**9.** 5-(2,6-difluorobenzyl)-4-methyl-4H-1,2,4-triazole-3-carboxamide or a salt thereof.

**10.** 5-(o-chlorobenzyl)-4-methyl-4H-1,2,4-triazole-3-carboxamide, 4-methyl-5-(m-trifluoromethylbenzyl)4H-1,2,4-triazole-3-carboxamide, 4-ethyl-5-(2,6-difluorobenzyl)-4H-1,2,4-triazole-3-carboxamide or 5-(2,6-difluorobenzyl)-4-methyl-4H-1,2,4-triazole-3-(N-methyl)carboxamide, or a salt thereof in each case.

**11.** 5-(2,6-difluorobenzyl)-4-methyl-4H-1,2,4-triazole-3-(N-ethyl)carboxamide, 5-(o-fluorobenzyl)-4-methyl-4H-1,2,4-triazole-3-carboxamide, 5-(2,6-difluorobenzyl)-4-methyl-4H-1,2,4-triazole-3-(N,N-dimethyl)-carboxamide, 5-(o-chlorobenzyl)-1,2,4-triazole-3-carboxamide, 5-[1-(2,6-difluorophenyl)ethyl]-4-methyl-4H-1,2,4-triazole-3-carboxamide or 5-(2,6-difluorobenzyl)4-methyl-4H-1,2,4-triazole-3-(N-acetyl)-carboxamide, or a salt thereof in each case.

**12.** A compound according to any one of claims 1 to 5 and 9 to 11 in free form.

**13.** A compound of the formula

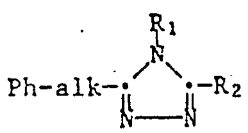

(I),

in which Ph represents phenyl substituted by $C_1$-$C_7$alkyl, halogen having an atomic number of up to and including 35 and/or by trifluoromethyl, alk represents $C_1$-$C_4$-alkylidene, $R_1$ is hydrogen or $C_1$-$C_7$alkyl, and $R_2$ represents carbamoyl that is unsubstituted or substituted by $C_1$-$C_7$alkyl or by $C_1$-$C_7$alkanoyl, or, as the case may be, a tautomer and/or a pharmaceutically acceptable salt thereof, for use in a method for the prophylactic and/or therapeutic treatment of the animal or human body.

**14.** A compound according to claim 13 in free form for use in a method for the prophylactic and/or therapeutic treatment of the animal or human body.

**15.** A compound according to any one of claims 1 to 12, or, as the case may be, a tautomer and/or a pharmaceutically acceptable salt thereof, for use in a method for the prophylactic and/or therapeutic treatment of the animal or human body.

**16.** A compound according to claim 12 in free form for use in a method for the prophylactic and/or therapeutic treatment of the animal or human body.

**17.** A compound of the formula I according to claim 13, in which Ph represents p-chlorophenyl, alk represents methylene, $R_1$ is methyl and $R_2$ represents N,N-diethylcarbamoyl, or a pharmaceutically acceptable salt thereof, for use in a method for the prophylactic and/or therapeutic treatment of the animal or human body.

**18.** A compound of the formula I according to claim 13, in which Ph represents phenyl mono-substituted by halogen having an atomic number of up to and including 35, alk represents $C_1$-$C_4$alkylidene, $R_1$ is hydrogen or $C_1$-$C_7$alkyl, and $R_2$ represents N,N-di-$C_1$-$C_4$alkylcarbamoyl in which the two N-alkyl groups are identical or different, or, as the case may be, a tautomer and/or a pharmaceutically acceptable salt thereof, for use in a method for the prophylactic and/or therapeutic treatment of the animal or human body.

**19.** A compound according to claim 17 or 18 in free form for use in a method for the prophylactic and/or therapeutic treatment of the animal or human body.

**20.** A pharmaceutical composition containing, as active ingredient, a compound according to any one of claims 1 to 19 or, as the case may be, a tautomer and/or a pharmaceutically acceptable salt thereof, optionally together with customary pharmaceutical excipients.

**21.** A pharmaceutical composition containing, as active ingredient, a compound according to any one of claims 12, 14, 16 and 19 in free form, optionally together with customary pharmaceutical excipients.

**22.** A process for the manufacture of a compound of the formula

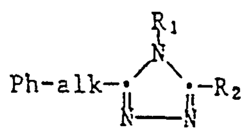

(I)

in which Ph represents phenyl substituted by $C_1$-$C_7$alkyl, halogen having an atomic number of up to and including 35 and/or by trifluoromethyl, alk represents $C_1$-$C_4$-alkylidene, $R_1$ is hydrogen or $C_1$-$C_7$alkyl, and $R_2$ represents carbamoyl that is unsubstituted or substituted by $C_1$-$C_7$alkyl or by $C_1$-$C_7$alkanoyl, with the proviso that, in a compound of the formula I in which $R_1$ represents methyl, $R_2$ represents N,N-diethylcarbamoyl and alk represents methylene, Ph is other than phenyl substituted in the p-position by chlorine, or, as the case may be, a tautomer and/or salt thereof, characterised in that

a) $X_1$ and $X_2$ are eliminated from a compound of the formula

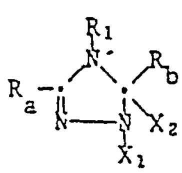

(II),

in which $X_1$ and $X_2$ represent groups that can be eliminated with the formation of an additional bond, and one of the two radicals $R_a$ and $R_b$ is a group Ph-alk and the other is a radical $R_2$, or, as the case may be, from a tautomer and/or salt thereof, or

b) in a compound of the formula

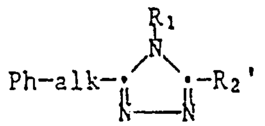

(III),

in which $R_2$' represents a group that can be converted into a radical $R_2$, or, as the case may be, in a tautomer and/or salt thereof, $R_2$' is converted into $R_2$, or

c) for the manufacture of a compound of the formula I in which $R_1$ is other than hydrogen and $R_2$ is especially N,N-di-$C_1$-$C_4$alkylcarbamoyl, the radical of the formula Ph-alk is introduced into a compound of the formula

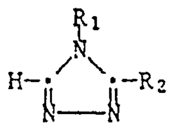

(IV)

or into a salt thereof, or

d) for the manufacture of a compound of the formula I in which $R_1$ is hydrogen, in a compound of the formula

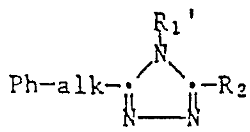

(V),

in which $R_1$' represents a group that can be replaced by hydrogen, or in a salt thereof, $R_1$' is replaced by hydrogen, or

e) for the manufacture of a compound of the formula I in which $R_2$ is N,N-di-$C_1$-$C_4$alkylcarbamoyl, in a compound of the formula

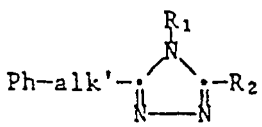

(VI),

in which alk' represents a radical that can be converted into a group alk, or, as the case may be, in a tautomer and/or salt thereof, alk' is converted into alk, and a mixture of isomers which may be obtained in each case in accordance with the process is separated into its components and the isomer of the formula I is isolated, if desired a compound of the formula I obtainable in each case in accordance with the process or by other means is converted into a different compound of the formula I, a mixture of stereoisomers which may be obtained in each case in accordance with the process is separated into the stereoisomers and the desired stereoisomer is isolated and/or a free compound of the formula I obtainable in each case in accordance with the process is converted into a salt, or a salt obtainable in accordance with the process is converted into the free compound of the formula I or into a different salt.

**23.** A process according to claim 22 for the manufacture of a compound of the formula I in which Ph represents phenyl mono-, di- or tri-substituted by $C_1$-$C_7$alkyl, halogen having an atomic number of up to and including 35 and/or by trifluoromethyl, alk represents $C_1$-$C_4$alkylidene, $R_1$ is hydrogen or $C_1$-$C_7$alkyl, and $R_2$ represents carbamoyl, N-$C_1$-$C_7$alkylcarbamoyl, N,N-di-$C_1$-$C_4$alkylcarbamoyl or N-$C_1$-$C_7$alkanoylcarbamoyl, or, as the case may be, a tautomer and/or salt thereof.

**24.** A process according to claim 22 for the manufacture of a compound of the formula I in which Ph represents phenyl mono-substituted by $C_1$-$C_4$alkyl, halogen or by trifluoromethyl or di-substituted by

halogen, by halogen and $C_1$-$C_4$alkyl, or by halogen and trifluoromethyl, wherein halogen in each case has an atomic number of up to and including 35, alk represents 1,1- or 2,2-$C_1$-$C_4$-alkylidene, $R_1$ is hydrogen or $C_1$-$C_4$alkyl, and $R_2$ represents carbamoyl, N-$C_1$-$C_4$alkylcarbamoyl, N,N-di-$C_1$-$C_4$-alkylcarbamoyl or N-$C_2$-$C_7$alkanoylcarbamoyl, or, as the case may be, a tautomer and/or salt thereof.

**25.** A process according to claim 22 for the manufacture of a compound of the formula I in which Ph, alk, $R_1$ and $R_2$ have one of the respective meanings described in any one of claims 22 to 24 and in which Ph carries at least one of the alkyl and/or halogen substituents in an o-position or a trifluoromethyl substituent in a m-position, or, as the case may be, a tautomer and/or salt thereof.

**26.** A process according to claim 22 for the manufacture of a compound of the formula I in which Ph represents o-$C_1$-$C_4$alkylphenyl, m-trifluoromethylphenyl, o-halophenyl or 2,3-, 2,4-, 2,5- or 2,6-dihalophenyl, wherein halogen in each case has an atomic number of up to and including 35, alk represents 1,1-$C_1$-$C_4$alkylidene, $R_1$ is hydrogen or $C_1$-$C_4$alkyl, and $R_2$ represents carbamoyl or N-$C_1$-$C_4$alkylcarbamoyl, N,N-di-$C_1$-$C_4$alkylcarbamoyl or N-$C_2$-$C_7$alkanoylcarbamoyl, or, as the case may be, a tautomer and/or salt thereof.

**27.** A process according to claim 22 for the manufacture of a compound of the formula I in which Ph, alk, $R_1$ and $R_2$ have one of the respective meanings described in any one of claims 22 to 26, with the proviso that Ph is other than phenyl mono-substituted by halogen having an atomic number of up to and including 35 when $R_2$ represents N,N-di-$C_1$-$C_4$alkylcarbamoyl in which the two N-alkyl groups are identical or different, or, as the case may be, a tautomer and/or salt thereof.

**28.** A process according to claim 22 for the manufacture of a compound of the formula I in which Ph represents m-trifluoromethylphenyl, o-fluorophenyl or 2,6-difluorophenyl, alk represents methylene, $R_1$ is $C_1$-$C_4$alkyl, and $R_2$ represents carbamoyl, or a salt thereof.

**29.** A process according to claim 22 for the manufacture of a compound of the formula I in which Ph represents m-trifluoromethylphenyl or 2,6-difluorophenyl, alk represents methylene, $R_1$ is $C_1$-$C_4$alkyl, and $R_2$ represents carbamoyl, or a salt thereof.

**30.** A process according to claim 22 for the manufacture of 5-(2,6-difluorobenzyl)-4-methyl-4H-1,2,4-triazole-3-carboxamide or a salt thereof.

**31.** A process according to claim 22 for the manufacture of 5-(o-chlorobenzyl)-4-methyl-4H-1,2,4-triazole-3-carboxamide, 4-methyl-5-(m-trifluoromethylbenzyl)-4H-1,2,4-triazole-3-carboxamide, 4-ethyl-5-(2,6-difluorobenzyl)-4H-1,2,4-triazole-3-carboxamide or 5-(2,6-difluorobenzyl)-4-methyl-4H-1,2,4-triazole-3-(N-methyl)carboxamide, or a salt thereof in each case.

**32.** A process according to claim 22 for the manufacture of 5-(2,6-difluorobenzyl)-4-methyl-4H-1,2,4-triazole-3-(N-ethyl)carboxamide, 5-(o-fluorobenzyl)-4-methyl-4H-1,2,4-triazole-3-carboxamide, 5-(2,6-difluorobenzyl)-4-methyl-4H-1,2,4-triazole-3-(N,N-dimethyl)carboxamide, 5-(o-chlorobenzyl)-1,2,4-triazole-3-carboxamide, 5-[1-(2,6-difluorophenyl)ethyl]-4-methyl-4H-1,2,4-triazole-3-carboxamide or 5-(2,6-difluorobenzyl)-4-methyl-4H-1,2,4-triazole-3-(N-acetyl)carboxamide, or a salt thereof in each case.

**33.** A process according to claim 22 for the manufacture of a compound of the formula I in which Ph, alk, $R_1$ and $R_2$ have one of the respective meanings described in any one of claims 22 to 26 in free form, or a process according to claim 22 for the manufacture of one of the compounds described in any one of claims 30 to 32 in free form.

**34.** A process for the manufacture of a pharmaceutical composition, characterised in that

a) $X_1$ and $X_2$ are eliminated from a compound of the formula

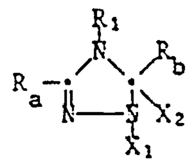

(II),

in which $X_1$ and $X_2$ represent groups that can be eliminated with the formation of an additional bond, and one of the two radicals $R_a$ and $R_b$ is a group Ph-alk and the other is a radical $R_2$, or, as the case may be, from a tautomer and/or salt thereof, or
b) in a compound of the formula

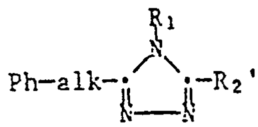

(III),

in which $R_2$' represents a group that can be converted into a radical $R_2$, or, as the case may be, in a tautomer and/or salt thereof, $R_2$' is converted into $R_2$, or
c) for the manufacture of a compound of the formula I in which $R_1$ is other than hydrogen and $R_2$ is especially N,N-di-$C_1$-$C_4$alkylcarbamoyl, the radical of the formula Ph-alk is introduced into a compound of the formula

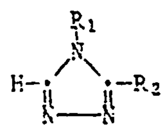

(IV)

or into a salt thereof, or
d) for the manufacture of a compound of the formula I in which $R_1$ is hydrogen, in a compound of the formula

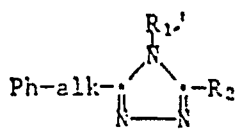

(V),

in which $R_1$' represents a group that can be replaced by hydrogen, or in a salt thereof, $R_1$' is replaced by hydrogen, or
e) for the manufacture of a compound of the formula I in which $R_2$ is N,N-di-$C_1$-$C_4$alkylcarbamoyl, in a compound of the formula

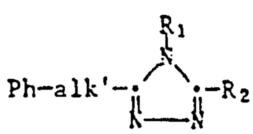

(VI),

in which alk' represents a radical that can be converted into a group alk, or, as the case may be, in a tautomer and/or salt thereof, alk' is converted into alk, and a mixture of isomers which may be obtained in each case in accordance with the process is separated into its components and the isomer of the formula I is isolated, if desired a compound of the formula I obtainable in each case in accordance with the process or by other means is converted into a different compound of the formula I, a mixture of stereoisomers which may be obtained in each case in accordance with the process is separated into the stereoisomers and the desired stereoisomer is isolated, and/or a free

compound of the formula I obtainable in each case in accordance with the process is converted into a salt, or a salt obtainable in accordance with the process is converted into the free compound of the formula I or into a different salt, and a resulting compound of the formula

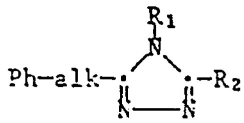

(I),

in which Ph represents phenyl substituted by $C_1$-$C_7$alkyl, halogen having an atomic number of up to and including 35 and/or by trifluoromethyl, alk represents $C_1$-$C_4$alkylidene, $R_1$ is hydrogen or $C_1$-$C_7$alkyl, and $R_2$ represents carbamoyl that is unsubstituted or substituted by $C_1$-$C_7$-alkyl or by $C_1$-$C_7$alkanoyl, or a tautomer that may be obtained and/or in each case a resulting pharmaceutically acceptable salt thereof is mixed with customary pharmaceutical excipients.

**35.** A process according to claim 34 for the manufacture of a pharmaceutical composition, characterised in that a compound of the formula I obtained in accordance with the process described in claim 34, in which Ph represents phenyl substituted by $C_1$-$C_7$alkyl, halogen having an atomic number of up to and including 35 and/or by trifluoromethyl, alk represents $C_1$-$C_4$alkylidene, $R_1$ is hydrogen or $C_1$-$C_7$alkyl, and $R_2$ represents carbamoyl that is unsubstituted or substituted by $C_1$-$C_7$alkyl or by $C_1$-$C_7$alkanoyl, in free form is mixed with customary pharmaceutical excipients.

**36.** A process for the manufacture of a pharmaceutical composition, characterised in that a compound obtainable according to any one of claims 22 to 33, or, as the case may be, a tautomer and/or a pharmaceutically acceptable salt thereof, is mixed with customary pharmaceutical excipients.

**37.** A process according to claim 36 for the manufacture of a pharmaceutical composition, characterised in that a compound obtainable according to claim 33 in free form is mixed with customary pharmaceutical excipients.

**38.** A process according to claim 34 for the manufacture of a pharmaceutical composition, characterised in that a compound of the formula I obtained in accordance with the process described in claim 34, in which Ph represents p-chlorophenyl, alk represents methylene, $R_1$ is methyl and $R_2$ represents N,N-diethylcarbamoyl, or a pharmaceutically acceptable salt thereof, is mixed with customary pharmaceutical excipients.

**39.** A process according to claim 34 for the manufacture of a pharmaceutical composition, characterised in that a compound of the formula I obtained in accordance with the process described in claim 34, in which Ph represents phenyl mono-substituted by halogen having an atomic number of up to and including 35, alk represents $C_1$-$C_4$-alkylidene, $R_1$ is hydrogen or $C_1$-$C_7$alkyl, and $R_2$ represents N,N-di-$C_1$-$C_4$alkylcarbamoyl in which the two N-alkyl groups are identical or different, or, as the case may be, a tautomer and/or a pharmaceutically acceptable salt thereof, is mixed with customary pharmaceutical excipients.

**40.** A process according to claim 34 for the manufacture of a pharmaceutical composition, characterised in that one of the compounds of the formula I described in claim 38 or 39 in free form is mixed with customary pharmaceutical excipients.

**41.** The use of a compound of the formula I obtainable according to any one of claims 22 to 33, or the use of one of the compounds of the formula I described in any one of claims 38 to 40, or the use of a compound of the formula I according to any one of claims 1 to 19, or, as the case may be, of a tautomer and/or a pharmaceutically acceptable salt thereof, for the manufacture of a pharmaceutical composition, for example an anti-convulsant.

**Claims for the following Contracting States : ES, GR**

**1.** A process for the manufacture of a compound of the formula

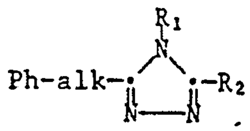

(I)

in which Ph represents phenyl substituted by $C_1$-$C_7$alkyl, halogen having an atomic number of up to and including 35 and/or by trifluoromethyl, alk represents $C_1$-$C_4$-alkylidene, $R_1$ is hydrogen or $C_1$-$C_7$alkyl, and $R_2$ represents carbamoyl that is unsubstituted or substituted by $C_1$-$C_7$alkyl or by $C_1$-$C_7$alkanoyl, with the proviso that, in a compound of the formula I in which $R_1$ represents methyl, $R_2$ represents N,N-diethylcarbamoyl and alk represents methylene, Ph is other than phenyl substituted in the p-position by chlorine, or, as the case may be, a tautomer and/or salt thereof, characterised in that

a) $X_1$ and $X_2$ are eliminated from a compound of the formula

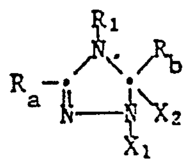

(II),

in which $X_1$ and $X_2$ represent groups that can be eliminated with the formation of an additional bond, and one of the two radicals $R_a$ and $R_b$ is a group Ph-alk and the other is a radical $R_2$, or, as the case may be, from a tautomer and/or salt thereof, or

b) in a compound of the formula

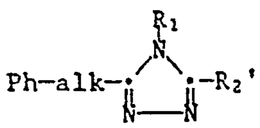

(III),

in which $R_2$' represents a group that can be converted into a radical $R_2$, or, as the case may be, in a tautomer and/or salt thereof, $R_2$' is converted into $R_2$, or

c) for the manufacture of a compound of the formula I in which $R_1$ is other than hydrogen and $R_2$ is especially N,N-di-$C_1$-$C_4$alkylcarbamoyl, the radical of the formula Ph-alk is introduced into a compound of the formula

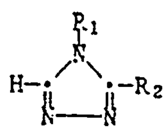

(IV)

or into a salt thereof, or

d) for the manufacture of a compound of the formula I in which $R_1$ is hydrogen, in a compound of the formula

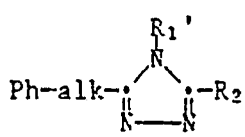

(V),

in which $R_1$' represents a group that can be replaced by hydrogen, or in a salt thereof, $R_1$' is replaced by hydrogen, or

e) for the manufacture of a compound of the formula I in which $R_2$ is N,N-di-$C_1$-$C_4$alkylcarbamoyl, in

a compound of the formula

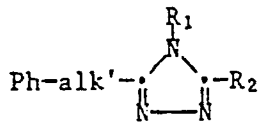 (VI),

in which alk' represents a radical that can be converted into a group alk, or, as the case may be, in a tautomer and/or salt thereof, alk' is converted into alk, and a mixture of isomers which may be obtained in each case in accordance with the process is separated into its components and the isomer of the formula I is isolated, if desired a compound of the formula I obtainable in each case in accordance with the process or by other means is converted into a different compound of the formula I, a mixture of stereoisomers which may be obtained in each case in accordance with the process is separated into the stereoisomers and the desired stereoisomer is isolated and/or a free compound of the formula I obtainable in each case in accordance with the process is converted into a salt, or a salt obtainable in accordance with the process is converted into the free compound of the formula I or into a different salt.

2. A process according to claim 1 for the manufacture of a compound of the formula I in which Ph represents phenyl mono-, di- or tri-substituted by $C_1$-$C_7$alkyl, halogen having an atomic number of up to and including 35 and/or by trifluoromethyl, alk represents $C_1$-$C_4$alkylidene, $R_1$ is hydrogen or $C_1$-$C_7$alkyl, and $R_2$ represents carbamoyl, N-$C_1$-$C_7$alkylcarbamoyl, N,N-di-$C_1$-$C_4$alkylcarbamoyl or N-$C_1$-$C_7$alkanoylcarbamoyl, or, as the case may be, a tautomer and/or salt thereof.

3. A process according to claim 1 for the manufacture of a compound of the formula I in which Ph represents phenyl mono-substituted by $C_1$-$C_4$alkyl, halogen or by trifluoromethyl or di-substituted by halogen, by halogen and $C_1$-$C_4$alkyl, or by halogen and trifluoromethyl, wherein halogen in each case has an atomic number of up to and including 35, alk represents 1,1- or 2,2-$C_1$-$C_4$-alkylidene, $R_1$ is hydrogen or $C_1$-$C_4$alkyl, and $R_2$ represents carbamoyl, N-$C_1$-$C_4$alkylcarbamoyl, N,N-di-$C_1$-$C_4$-alkylcarbamoyl or N-$C_2$-$C_7$alkanoylcarbamoyl, or, as the case may be, a tautomer and/or salt thereof.

4. A process according to claim 1 for the manufacture of a compound of the formula I in which Ph, alk, $R_1$ and $R_2$ have one of the respective meanings described in any one of claims 1 to 3 and in which Ph carries at least one of the alkyl and/or halogen substituents in an o-position or a trifluoromethyl substituent in a m-position, or, as the case may be, a tautomer and/or salt thereof.

5. A process according to claim 1 for the manufacture of a compound of the formula I in which Ph represents o-$C_1$-$C_4$alkylphenyl, m-trifluoromethylphenyl, o-halophenyl or 2,3-, 2,4-, 2,5- or 2,6-dihalophenyl, wherein halogen in each case has an atomic number of up to and including 35, alk represents 1,1-$C_1$-$C_4$alkylidene, $R_1$ is hydrogen or $C_1$-$C_4$alkyl, and $R_2$ represents carbamoyl or N-$C_1$-$C_4$alkylcarbamoyl, N,N-di-$C_1$-$C_4$alkylcarbamoyl or N-$C_2$-$C_7$alkanoylcarbamoyl, or, as the case may be, a tautomer and/or salt thereof.

6. A process according to claim 1 for the manufacture of a compound of the formula I in which Ph, alk, $R_1$ and $R_2$ have one of the respective meanings described in any one of claims 1 to 5, with the proviso that Ph is other than phenyl mono-substituted by halogen having an atomic number of up to and including 35 when $R_2$ represents N,N-di-$C_1$-$C_4$alkylcarbamoyl in which the two N-alkyl groups are identical or different, or, as the case may be, a tautomer and/or salt thereof.

7. A process according to claim 1 for the manufacture of a compound of the formula I in which Ph represents m-trifluoromethylphenyl, o-fluorophenyl or 2,6-difluorophenyl, alk represents methylene, $R_1$ is $C_1$-$C_4$alkyl, and $R_2$ represents carbamoyl, or a salt thereof.

8. A process according to claim 1 for the manufacture of a compound of the formula I in which Ph represents m-trifluoromethylphenyl or 2,6-difluorophenyl, alk represents methylene, $R_1$ is $C_1$-$C_4$alkyl, and $R_2$ represents carbamoyl, or a salt thereof.

**9.** A process according to claim 1 for the manufacture of 5-(2,6-difluorobenzyl)-4-methyl-4H-1,2,4-triazole-3-carboxamide or a salt thereof.

**10.** A process according to claim 1 for the manufacture of 5-(o-chlorobenzyl)-4-methyl-4H-1,2,4-triazole-3-carboxamide, 4-methyl-5-(m-trifluoromethylbenzyl)4H-1,2,4-triazole-3-carboxamide, 4-ethyl-5-(2,6-difluorobenzyl)-4H-1,2,4-triazole-3-carboxamide or 5-(2,6-difluorobenzyl)-4-methyl-4H-1,2,4-triazole-3-(N-methyl)carboxamide, or a salt thereof in each case.

**11.** A process according to claim 1 for the manufacture of 5-(2,6-difluorobenzyl)-4-methyl-4H-1,2,4-triazole-3-(N-ethyl)carboxamide, 5-(o-fluorobenzyl)-4-methyl-4H-1,2,4-triazole-3-carboxamide, 5-(2,6-difluorobenzyl)-4-methyl-4H-1,2,4-triazole-3-(N,N-dimethyl)carboxamide, 5-(o-chlorobenzyl)-1,2,4-triazole-3-carboxamide, 5-[1-(2,6-difluorophenyl)ethyl]-4-methyl-4H-1,2, 4-triazole-3-carboxamide or 5-(2,6-difluorobenzyl)-4-methyl-4H-1,2,4-triazole-3-(N-acetyl)carboxamide, or a salt thereof in each case.

**12.** A process according to claim 1 for the manufacture of a compound of the formula I in which Ph, alk, $R_1$ and $R_2$ have one of the respective meanings described in any one of claims 1 to 8 in free form, or a process according to claim 1 for the manufacture of one of the compounds described in any one of claims 9 to 11 in free form.

**13.** A process according to claim 1 for the manufacture of a compound of the formula I in which Ph, alk $R_1$ and $R_2$ have one of the respective meanings described in any one of claims 1 to 5 in free form, or a process according to claim 1 for the manufacture of one of the compounds described in any one of claims 9 to 11 in free form.

**14.** A process for the manufacture of a pharmaceutical composition, characterised in that
a) $X_1$ and $X_2$ are eliminated from a compound of the formula

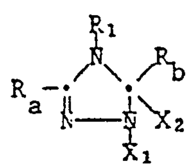

(II),

in which $X_1$ and $X_2$ represent groups that can be eliminated with the formation of an additional bond, and one of the two radicals $R_a$ and $R_b$ is a group Ph-alk and the other is a radical $R_2$, or, as the case may be, from a tautomer and/or salt thereof, or
b) in a compound of the formula

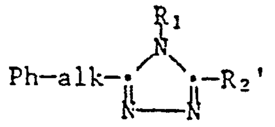

(III),

in which $R_2$' represents a group that can be converted into a radical $R_2$, or, as the case may be, in a tautomer and/or salt thereof, $R_2$' is converted into $R_2$, or
c) for the manufacture of a compound of the formula I in which $R_1$ is other than hydrogen and $R_2$ is especially N,N-di-$C_1$-$C_4$alkylcarbamoyl, the radical of the formula Ph-alk is introduced into a compound of the formula

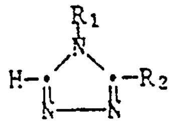

(IV)

or into a salt thereof, or

d) for the manufacture of a compound of the formula I in which $R_1$ is hydrogen, in a compound of the formula

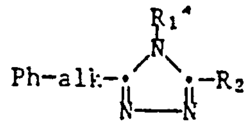

(V),

in which $R_1$' represents a group that can be replaced by hydrogen, or in a salt thereof, $R_1$' is replaced by hydrogen, or

e) for the manufacture of a compound of the formula I in which $R_2$ is N,N-di-$C_1$-$C_4$alkylcarbamoyl, in a compound of the formula

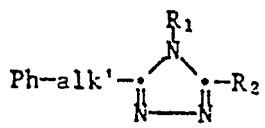

(VI),

in which alk' represents a radical that can be converted into a group alk, or, as the case may be, in a tautomer and/or salt thereof, alk' is converted into alk, and a mixture of isomers which may be obtained in each case in accordance with the process is separated into its components and the isomer of the formula I is isolated, if desired a compound of the formula I obtainable in each case in accordance with the process or by other means is converted into a different compound of the formula I, a mixture of stereoisomers which may be obtained in each case in accordance with the process is separated into the stereoisomers and the desired stereoisomer is isolated, and/or a free compound of the formula I obtainable in each case in accordance with the process is converted into a salt, or a salt obtainable in accordance with the process is converted into the free compound of the formula I or into a different salt, and a resulting compound of the formula

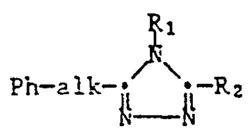

(I),

in which Ph represents phenyl substituted by $C_1$-$C_7$alkyl, halogen having an atomic number of up to and including 35 and/or by trifluoromethyl, alk represents $C_1$-$C_4$alkylidene, $R_1$ is hydrogen or $C_1$-$C_7$alkyl, and $R_2$ represents carbamoyl that is unsubstituted or substituted by $C_1$-$C_7$-alkyl or by $C_1$-$C_7$alkanoyl, or a tautomer that may be obtained and/or a resulting pharmaceutically acceptable salt thereof in each case is mixed with customary pharmaceutical excipients.

**15.** A process according to claim 14 for the manufacture of a pharmaceutical composition, characterised in that a compound of the formula I obtained in accordance with the process described in claim 14, in which Ph represents phenyl substituted by $C_1$-$C_7$alkyl, halogen having an atomic number of up to and including 35 and/or by trifluoromethyl, alk represents $C_1$-$C_4$alkylidene, $R_1$ is hydrogen or $C_1$-$C_7$alkyl, and $R_2$ represents carbamoyl that is unsubstituted or substituted by $C_1$-$C_7$alkyl or by $C_1$-$C_7$alkanoyl, in free form is mixed with customary pharmaceutical excipients.

**16.** A process for the manufacture of a pharmaceutical composition, characterised in that a compound obtainable according to any one of claims 1 to 13, or, as the case may be, a tautomer and/or a pharmaceutically acceptable salt thereof, is mixed with customary pharmaceutical excipients.

**17.** A process according to claim 16 for the manufacture of a pharmaceutical composition, characterised in that a compound obtainable according to claim 13 in free form is mixed with customary pharmaceutical excipients.

18. A process according to claim 14 for the manufacture of a pharmaceutical composition, characterised in that a compound of the formula I obtained in accordance with the process described in claim I4, in which Ph represents p-chlorophenyl, alk represents methylene, $R_1$ is methyl and $R_2$ represents N,N-diethylcarbamoyl, or a pharmaceutically acceptable salt thereof, is mixed with customary pharmaceutical excipients.

19. A process according to claim 14 for the manufacture of a pharmaceutical composition, characterised in that a compound of the formula I obtained in accordance with the process described in claim 14, in which Ph represents phenyl mono-substituted by halogen having an atomic number of up to and including 35, alk represents $C_1$-$C_4$-alkylidene, $R_1$ is hydrogen or $C_1$-$C_7$alkyl, and $R_2$ represents N,N-di-$C_1$-$C_4$alkylcarbamoyl in which the two N-alkyl groups are identical or different, or, as the case may be, a tautomer and/or a pharmaceutically acceptable salt thereof, is mixed with customary pharmaceutical excipients.

20. A process according to claim 14 for the manufacture of a pharmaceutical composition, characterised in that one of the compounds of the formula I described in claim 18 or 19 in free form is mixed with customary pharmaceutical excipients.

21. The use of a compound of the formula I obtainable according to any one of claims 1 to 13, or the use of one of the compounds of the formula I described in any one of claims 18 to 20, or, as the case may be, in each case a tautomer and/or a pharmaceutically acceptable salt thereof, for the manufacture of a pharmaceutical composition, for example an anti-convulsant.

**Revendications**

**Revendications pour les Etats contractants suivants : AT, BE, CH, DE, FR, GB, IT, LI, LU, NL, SE**

1. Un composé de formule

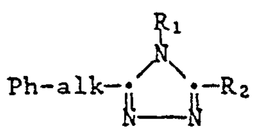 (I),

dans laquelle Ph représente un groupe phényle substitué par des groupes alkyle en C1-C7, des halogènes de numéro atomique allant jusqu'à 35 inclus et/ou des groupes trifluorométhyle, alk représente un groupe alkylidène en C1-C4, $R_1$ représente l'hydrogène ou un groupe alkyle en C1-C7 et $R_2$ représente un groupe carbamyle éventuellement substitué par des groupes alkyle en C1-C7 ou alcanoyle en C1-C, sous réserve que, dans un composé de formule I dans laquelle $R_1$ représente un groupe méthyle, $R_2$ un groupe N,N-diéthylcarbamyle et alk un groupe méthylène, Ph ne peut représenter un groupe phényle substitué par le chlore en position para, ou le cas échéant un tautomère et/ou un sel d'un tel composé.

2. Un composé de formule I selon la revendication 1, dans laquelle Ph représente un groupe phényle mono-, di- ou tri-substitué par des groupes alkyle en C1-C7, des halogènes et/ou des groupes trifluorométhyle, alk représente un groupe alkylidène en C1-C4, $R_1$ représente l'hydrogène ou un groupe alkyle en C1-C7 et $R_2$ un groupe carbamyle, N-(alkyle en C1-C7)-carbamyle, N,N-di-(alkyle en C1-C4)-carbamyle ou N-(alcanoyle en C1-C7)-carbamyle, ou le cas échéant un tautomère et/ou un sel d'un tel composé.

3. Un composé de formule I selon la revendication 1, dans laquelle Ph représente un groupe phényle monosubstitué par un groupe alkyle en C1-C4, un halogène ou un groupe trifluorométhyle ou disubstitué par des halogènes, un halogène et un groupe alkyle en C1-C4 ou un halogène et un groupe trifluorométhyle, les halogènes en question ayant des numéros atomiques allant jusqu'à 35 inclus, alk représente un groupe 1,1- ou 2,2-alkylidène en C1-C4, $R_1$ représente l'hydrogène ou un groupe alkyle en C1-C4 et $R_2$ un groupe carbamyle, N-(alkyle en C1-C4)-carbamyle, N,N-di-(alkyle en C1-C4)-carbamyle ou N-(alcanoyle en C2-C7)-carbamyle, ou le cas échéant un tautomère et/ou un sel d'un tel

composé.

**4.** Un composé de formule I selon l'une des revendications 1 à 3, dans laquelle Ph porte au moins l'un des substituants alkyle et/ou halogéno dans une position ortho ou un substituant trifluorométhyle dans une position méta, ou le cas échéant, un tautomère et/ou un sel d'un tel composé.

**5.** Un composé de formule I selon la revendication 1, dans laquelle Ph représente un groupe o-(alkyle en C1-C4)-phényle, m-trifluorométhylphényle, o-halogénophényle ou 2,3-, 2,4-, 2,5- ou 2,6-dihalogénophényle, les halogènes en question ayant un numéro atomique allant jusqu'à 35 inclus, alk représente un groupe 1,1-alkylidène en C1-C4, $R_1$ représente l'hydrogène ou un groupe alkyle en C1-C4 et $R_2$ un groupe carbamyle ou N-(alkyle en C1-C4)-carbamyle, N,N-di-(alkyle en C1-C4)-carbamyle ou N-(alcanoyle en C2-C7)-carbamyle, ou le cas échéant un tautomère et/ou un sel d'un tel composé.

**6.** Un composé de formule I selon l'une des revendications 1 à 5, avec la restriction que Ph ne peut représenter un groupe phényle monosubstitué par un halogène de numéro atomique allant jusqu'à 35 inclus lorsque $R_2$ représente un groupe N,N-di-(alkyle en C1-C4)-carbamyle dans lequel les deux groupes N-alkyle sont identiques ou différents, ou le cas échéant un tautomère et/ou un sel d'un tel composé.

**7.** Un composé de formule I selon la revendication 1, dans laquelle Ph représente un groupe m-trifluorométhylphényle, o-fluorophényle ou 2,6-difluorophényle, alk représente un groupe méthylène, $R_1$ un groupe alkyle en C1-C4 et $R_2$ un groupe carbamyle, ou un sel d'un tel composé.

**8.** Un composé de formule I selon la revendication 1, dans laquelle Ph représente un groupe m-trifluorométhylphényle ou 2,6-difluorophényle alk représente un groupe méthylène, $R_1$ représente un groupe alkyle en C1-C4 et $R_2$ un groupe carbamyle, ou un sel d'un tel composé.

**9.** Le 5-(2,6-difluorobenzyl)-4-méthyl-4H-1,2,4-triazole-3-carboxamideou un sel de ce composé.

**10.** Le 5-(o-chlorobenzyl)-4-méthyl-4H-1,2,4-triazole-3-carboxamide, le 4-méthyl-5-(m-trifluorométhylbenzyl)-4H-1,2,4-triazole-3-carboxamide, le 4-éthyl-5-(2,6-difluorobenzyl)-4H-1,2,4-triazole-3-carboxamide ou le 5-(2,6-difluorobenzyl)-4-méthyl-4H-1,2,4-triazole-3-(N-méthyl)-carboxamide ou un sel de l'un de ces composés.

**11.** Le 5-(2,6-difluorobenzyl)-4-méthyl-4H-1,2,4-triazole-3-(N-éthyl)-carboxamide, le 5-(o-fluorobenzyl)-4-méthyl-4H-1,2,4-triazole-3-carboxamide, le 5-(2,6-difluorobenzyl)-4-méthyl-4H-1,2,4-triazole-3-(N,N-diméthyl)carboxamide, le 5-(o-chlorobenzyl)-1,2,4-triazole-3-carboxamide, le 5-[1-(2,6-difluorophényl)-éthyl]-4-méthyl-4H-1,2,4-triazole-3-carboxamide ou le 5-(2,6-difluorobenzyl)-4-méthyl-4H-1,2,4-triazole-3-(N-acétyl)-carboxamide ou un sel de l'un de ces composés.

**12.** Un composé selon l'une des revendications 1 à 5 et 9 à 11 à l'état libre.

**13.** Un composé de formule

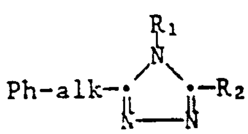

(I),

dans laquelle Ph représente un groupe phényle substitué par des groupes alkyle en C1-C7, des halogènes de numéro atomique allant jusqu'à 35 inclus et/ou des groupes trifluorométhyle, alk représente un groupe alkylidène en C1-C4, $R_1$ représente l'hydrogène ou un groupe alkyle en C1-C7 et $R_2$ un groupe carbamyle éventuellement substitué par des groupes alkyle en C1-C7 ou alcanoyle en C1-C7, ou le cas échéant un tautomère et/ou un sel acceptable pour l'usage pharmaceutique de ce composé, pour l'utilisation dans un procédé pour le traitement prophylactique et/ou thérapeutique de l'organisme humain ou animal.

**14.** Un composé selon la revendication 13, à l'état libre, pour l'utilisation dans un procédé pour le traitement prophylactique et/ou thérapeutique de l'organisme humain ou animal.

**15.** Un composé selon l'une des revendications 1 à 12 ou le cas échéant un tautomère et/ou un sel acceptable pour l'usage pharmaceutique d'un tel composé pour l'utilisation dans un procédé pour le traitement prophylactique et/ou thérapeutique de l'organisme humain ou animal.

**16.** Un composé selon la revendication 12, à l'état libre, pour l'utilisation dans un procédé pour le traitement prophylactique et/ou thérapeutique de l'organisme humain ou animal.

**17.** Un composé de formule I de la revendication 13, dans laquelle Ph représente un groupe' p-chlorophényle, alk représente un groupe méthylène, $R_1$ un groupe méthyle et $R_2$ un groupe N,N-diéthylcarbamyle, ou un sel acceptable pour l'usage pharmaceutique d'un tel composé, pour l'utilisation dans un procédé pour le traitement prophylactique et/ou thérapeutique de l'organisme humain ou animal.

**18.** Un composé de formule I de la revendication 13, dans laquelle Ph représente un groupe phényle monosubstitué par un halogène de numéro atomique allant jusqu'à 35 inclus, alk représente un groupe alkylidène en C1-C4, $R_1$ l'hydrogène ou un groupe alkyle en C1-C7 et $R_2$ un groupe N,N-di-(alkyle en C1-C4)-carbamyle dans lequel les deux groupes N-alkyle sont identiques ou différents, ou le cas échéant un tautomère et/ou un sel acceptable pour l'usage pharmaceutique d'un tel composé, pour l'utilisation dans un procédé pour le traitement prophylactique et/ou thérapeutique de l'organisme humain ou animal.

**19.** Un composé selon la revendication 17 ou 18 à l'état libre pour l'utilisation dans un procédé pour le traitement prophylactique et/ou thérapeutique de l'organisme humain ou animal.

**20.** Une composition pharmaceutique contenant en tant que substance active un composé selon l'une des revendications 1 à 19 ou le cas échéant un tautomère et/ou un sel acceptable pour l'usage pharmaceutique d'un tel composé, éventuellement avec des produits auxiliaires pharmaceutiques usuels.

**21.** Une composition pharmaceutique contenant en tant que substance active un composé selon l'une des revendications 12, 14, 16 et 19 à l'état libre, éventuellement avec des produits auxiliaires pharmaceutiques usuels.

**22.** Procédé de préparation d'un composé de formule

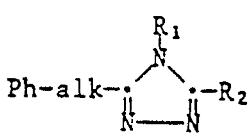 (I),

dans laquelle Ph représente un groupe phényle substitué par des groupes alkyle en C1-C7, des halogènes de numéro atomique allant jusqu'à 35 inclus ou des groupes trifluorométhyle, alk représente un groupe alkylidène en C1-C4, $R_1$ l'hydrogène ou un groupe alkyle en C1-C7 et $R_2$ un groupe carbamyle éventuellement substitué par des groupes alkyle en C1-C7 ou alcanoyle en C1-C7, sous réserve que, dans un composé de formule I dans laquelle $R_1$ représente un groupe méthyle, $R_2$ un groupe N,N-diéthylcarbamyle et alk un groupe méthylène, Ph ne peut représenter un groupe phényle substitué par le chlore en position para, ou le cas échéant d'un tautomère et/ou d'un sel d'un tel composé, caractérisé en ce que

a) dans un composé de formule

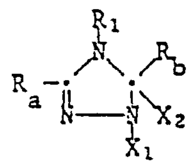

(II),

dans laquelle $X_1$ et $X_2$ représentent des groupes éliminables avec formation d'une liaison supplémentaire, et l'un des symboles $R_a$ et $R_b$ représente un groupe Ph-alk et l'autre un groupe $R_2$, ou le cas échéant dans un tautomère et/ou un sel d'un tel composé, on élimine $X_1$ et $X_2$, ou bien

b) dans un composé de formule

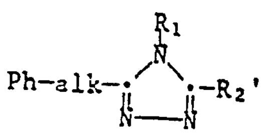

(III),

dans laquelle $R'_2$ représente un groupe convertible en un groupe $R_2$, ou le cas échéant dans un tautomère et/ou un sel d'un tel composé, on convertit $R'_2$ en $R_2$, ou bien,

c) pour préparer un composé de formule I dans laquelle $R_1$ a une signification autre que l'hydrogène et $R_2$ représente en particulier un groupe N,N-di-(alkyle en C1-C4)-carbamyle, on introduit le groupe de formule Ph-alk dans un composé de formule

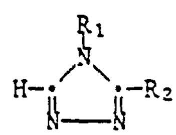

(IV)

ou dans un sel d'un tel composé, ou bien

d) pour préparer un composé de formule I dans laquelle $R_1$ représente l'hydrogène, partant d'un composé de formule

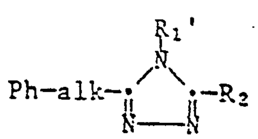

(V),

dans laquelle $R'_1$ représente un groupe remplaçable par l'hydrogène, ou d'un sel d'un tel composé, on remplace $R'_1$ par l'hydrogène, ou bien

e) pour préparer un composé de formule I dans laquelle $R_2$ représente un groupe N,N-di-(alkyle en C1-C4)-carbamyle, partant d'un ccmposé de formule

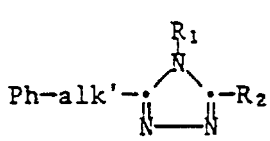

(VI),

dans laquelle alk' représente un groupe convertible en un groupe alk, ou le cas échéant d'un tautomère et/ou d'un sel d'un tel composé, on convertit alk' en alk, et dans chaque cas, on sépare un mélange d'isomères éventuellement obtenu par ce procédé en les composants et on isole l'isomère de formule I, et si on le désire, dans chaque cas, on convertit un composé de formule I obtenu par le procédé ou de toute autre manière, en un autre composé de formule I, et dans chaque

cas on sépare un mélange de stéréoisomères éventuellement obtenu par le procédé en les stéréoisomères et on isole le stéréoisomère recherché, et/ou dans chaque cas, on convertit un composé de formule I obtenu dans le procédé à l'état libre en un sel, ou un sel obtenu par le procédé en le composé libre de formule I ou en un autre sel.

**23.** Procédé selon la revendication 22, pour préparer un composé de formule I dans laquelle Ph représente un groupe phényle mono-, di- ou tri-substitué par des groupes alkyle en C1-C7, des halogènes de numéro atomique allant jusqu'à 35 inclus et/ou des groupes trifluorométhyle, alk représente un groupe alkylidène en C1-C4, $R_1$ l'hydrogène ou un groupe alkyle en C1-C7 et $R_2$ représente un groupe carbamyle, N-(alkyle en C1-C7)-carbamyle, N,N-di-(alkyle en C1-C4)-carbamyle ou N-(alcanoyle en C1-C7)-carbamyle, ou le cas échéant un tautomère et/ou un sel d'un tel composé.

**24.** Procédé selon la revendication 22 pour préparer un composé de formule I dans laquelle Ph représente un groupe phényle monosubstitué par un groupe alkyle en C1-C4, un halogène ou un groupe trifluorométhyle ou disubstitué par des halogènes, un halogène et un groupe alkyle en C1-C4 ou un halogène et un groupe trifluorométhyle, les halogènes en question ayant dans chaque cas un numéro atomique allant jusqu'à 35 inclus, alk représente un groupe 1,1- ou 2,2-alkylidène en C1-C4, $R_1$ représente l'hydrogène ou un groupe alkyle en C1-C4 et $R_2$ un groupe carbamyle, N-(alkyle en C1-C4)-carbamyle, N,N-di-(alkyle en C1-C4)-carbamyle ou N(alcanoyle en C2-C7)-carbamyle, ou le cas échéant un tautomère et/ou un sel d'un tel composé.

**25.** Procédé selon la revendication 22 pour préparer un composé de formule I dans laquelle Ph, alk, $R_1$ et $R_2$ ont l'une quelconque des significations indiquées dans l'une des revendications 22 à 24 et Ph porte au moins l'un des substituants alkyle et/ou halogéno dans une position ortho ou un substituant trifluorométhyle dans une position méta, ou le cas échéant un tautomère et/ou un sel d'un tel composé.

**26.** Procédé selon la revendication 22 pour préparer un composé de formule I dans laquelle Ph représente un groupe o-(alkyle en C1-C4)-phényle, m-trifluorométhylphényle, o-halogénophényle ou 2,3-, 2,4-, 2,5- ou 2,6-dihalogénophényle, les halogènes en question ayant dans tous les cas un numéro atomique allant jusqu'à 35 inclus, alk représente un groupe 1,1-alkylidène en C1-C4, $R_1$ l'hydrogène ou un groupe alkyle en C1-C4 et $R_2$ un groupe carbamyle ou N-(alkyle en C1-C4)-carbamyle, N,N-di-(alkyle en C1-C4)-carbamyle ou N-(alcanoyle en C2-C7)-carbamyle, ou le cas échéant un tautomère et/ou un sel d'un tel composé.

**27.** Procédé selon la revendication 22 pour préparer un composé de formule I dans laquelle Ph, alk, $R_1$ et $R_2$ ont l'une des significations indiquées dans l'une des revendications 22 à 26, sous réserve que Ph ne peut représenter un groupe phényle monosubstitué par un halogène de numéro atomique allant jusqu'à 35 inclus lorsque $R_2$ représente un groupe N,N-di-(alkyle en C1-C4)-carbamyle dans lequel les deux groupes N-alkyle sont identiques ou différents, ou le cas échéant un tautomère et/ou un sel d'un tel composé.

**28.** Procédé selon la revendication 22 pour préparer un composé de formule I dans laquelle Ph représente un groupe m-trifluorométhylphényle, o-fluorophényle ou 2,6-difluorophényle, alk représente un groupe méthylène, $R_1$ un groupe alkyle en C1-C4 et $R_2$ un groupe carbamyle, ou un sel d'un tel composé.

**29.** Procédé selon la revendication 22 pour préparer un composé de formule I dans laquelle ph représente un groupe m-trifluorométhylphényle ou 2,6-difluorophényle, alk représente un groupe méthylène, $R_1$ un groupe alkyle en C1-C4 et $R_2$ un groupe carbamyle, ou un sel d'un tel composé.

**30.** Procédé selon la revendication 22 pour préparer le 5-(2,6-difluorobenzyl)-4-méthyl-4H-1,2,4-triazole-3-carboxamideou l'un de ses sels.

**31.** Procédé selon la revendication 22 pour préparer le 5-(o-chlorobenzyl)-4-méthyl-4H-1,2,4-triazole-3-carboxamide, le 4-méthyl-5-(m-trifluorométhylbenzyl)-4H-1,2,4-triazole-3-carboxamide, le 4-éthyl-5-(2,6-difluorobenzyl)-4H-1,2,4-triazole-3-carboxamide ou le 5-(2,6-difluorobenzyl)-4-méthyl-4H-1,2,4-triazole-3-(N-méthyl)-carboxamide ou un sel de l'un de ces composés.

**32.** Procédé selon la revendication 22 pour préparer le 5-(2,6-difluorobenzyl)-4-méthyl-4H-1,2,4-triazole-3-(N-éthyl)-carboxamide, le 5-(o-fluorobenzyl)-4-méthyl-4H-1,2,4-triazole-3-carboxamide, le 5-(2,6-difluorobenzyl)-4-méthyl-4H-1,2,4-triazole-3-(N,N-diméthyl)-carboxamide, le 5-(o-chlorobenzyl)-1,2,4-triazole-3-carboxamide, le 5-[1-(2,6-difluorophényl)-éthyl)-4-méthyl-4H-1,2,4-triazole-3-carboxamide ou le 5-(2,6-difluorobenzyl)-4-méthyl-4H-1,2,4-triazole-3-(N-acétyl)-carboxamide ou un sel de l'un de ces composés.

**33.** Procédé selon la revendication 22 pour préparer un composé de formule I dans laquelle Ph, alk, $R_1$ et $R_2$ ont l'une quelconque des significations indiquées dans l'une des revendications 22 à 26, à l'état libre, et procédé selon la revendication 22 pour préparer l'un des composés décrits dans l'une des revendications 30 à 32 à l'état libre.

**34.** Procédé de préparation d'une composition pharmaceutique, caractérisé en ce que

a) dans un composé de formule

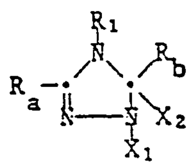

(II),

dans laquelle $X_1$ et $X_2$ représentent des groupes éliminables avec formation d'une liaison supplémentaire, et l'un des symboles $R_a$ et $R_b$ représente un groupe Ph-alk et l'autre un groupe $R_2$, ou le cas échéant dans un tautomère et/ou un sel d'un tel composé, on élimine $X_1$ et $X_2$, ou bien

b) dans un composé de formule

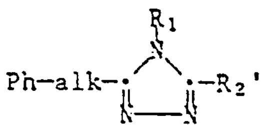

(III),

dans laquelle $R'_2$ représente un groupe convertible en un groupe $R_2$, ou le cas échéant dans un tautomère et/ou un sel d'un tel composé, on convertit $R'_2$ en $R_2$, ou bien

c) pour préparer un composé de formule I dans laquelle $R_1$ a une signification autre que l'hydrogène et $R_2$ représente en particulier un groupe N,N-di-(alkyle en C1-C4)-carbamyle, on introduit le groupe de formule Ph-alk dans un composé de formule

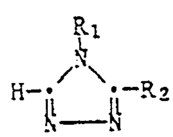

(IV)

ou dans un sel d'un tel composé, ou bien

d) pour préparer un composé de formule I dans laquelle $R_1$ représente l'hydrogène, partant d'un composé de formule

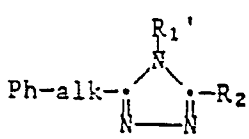

(V),

dans laquelle $R'_1$ représente un groupe remplaçable par l'hydrogène, ou d'un sel d'un tel composé, on remplace $R'_1$ par l'hydrogène, ou bien

e) pour préparer un composé de formule I dans laquelle $R_2$ représente un groupe N,N-di-(alkyle en

C1-C4)-carbamyle, partant d'un composé de formule

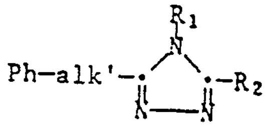

(VI),

dans laquelle alk' représente un groupe convertible en un groupe alk, ou le cas échéant d'un tautomère et/ou d'un sel d'un tel composé, on convertit alk' en alk, et dans chaque cas, on sépare un mélange d'isomères éventuellement obtenu par ce procédé en les composants et on isole l'isomère de formule I, et si on le désire, dans chaque cas, on convertit un composé de formule I obtenu par le procédé ou de toute autre manière, en un autre composé de formule I, et dans chaque cas on sépare un mélange de stéréoisomères éventuellement obtenu par le procédé en les stéréoisomères et on isole le stéréoisomère recherché, et/ou dans chaque cas, on convertit un composé de formule I obtenu dans le procédé à l'état libre en un sel, ou un sel obtenu par le procédé en le composé libre de formule I ou en un autre sel, et on mélange dans chaque cas un composé ainsi obtenu répondant à la formule

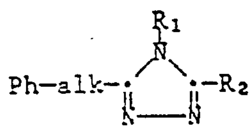

(I),

dans laquelle Ph représente un groupe phényle substitué par des groupes alkyle en C1-C7, des halogènes de numéro atomique allant jusqu'à 35 inclus et/ou des groupes trifluorométhyle, alk représente un groupe alkylidène en C1-C4, $R_1$ l'hydrogène ou un groupe alkyle en C1-C7 et $R_2$ un groupe carbamyle éventuellement substitué par des groupes alkyle en C1-C7 ou alcanoyle en C1-C7, ou le cas échéant un tautomère ainsi obtenu et/ou un sel acceptable pour l'usage pharmaceutique d'un tel composé, ainsi obtenu, avec des produits auxiliaires pharmaceutiques usuels.

**35.** Procédé selon la revendication 34, pour préparer une composition pharmaceutique, caractérisé en ce que l'on mélange un composé obtenu par le procédé décrit dans la revendication 34, répondant à la formule I dans laquelle Ph représente un groupe phényle substitué par des groupes alkyle en C1-C7, des halogènes de numéro atomique allant jusqu'à 35 inclus et/ou des groupes trifluorométhyle, alk représente un groupe alkylidène en C1-C4, $R_1$ l'hydrogène ou un groupe alkyle en C1-C7 et $R_2$ un groupe carbamyle éventuellement substitué par des groupes alkyle en C1-C7 ou alcanoyle en C1-C7, à l'état libre, avec des produits auxiliaires pharmaceutiques usuels.

**36.** Procédé de préparation d'une composition pharmaceutique caractérisé en ce que l'on mélange un composé obtenu selon l'une des revendications 22 à 23 ou le cas échéant un tautomère et/ou un sel acceptable pour l'usage pharmaceutique d'un tel composé, avec des produits auxiliaires pharmaceutiques usuels.

**37.** Procédé selon la revendication 36, pour préparer une composition pharmaceutique, caractérisé en ce que l'on mélange un composé obtenu selon la revendication 33, à l'état libre, avec des produits auxiliaires pharmaceutiques usuels.

**38.** Procédé selon la revendication 34, pour préparer une composition pharmaceutique, caractérisé en ce que l'on mélange un composé obtenu par le procédé décrit dans la revendication 34, répondant à la formule I dans laquelle Ph représente un groupe p-chlorophényle, alk un groupe méthylène, $R_1$ un groupe méthyle et $R_2$ un groupe N,N-diéthylcarbamyle, ou un sel acceptable pour l'usage pharmaceutique d'un tel composé, avec des produits auxiliaires pharmaceutiques usuels.

**39.** Procédé selon la revendication 34, pour préparer une composition pharmaceutique, caractérisé en ce que l'on mélange un composé obtenu par le procédé décrit dans la revendication 34, répondant à la

formule I dans laquelle Ph représente un groupe phényle monosubstitué par un halogène de numéro atomique allant jusqu'à 35 inclus, alk représente un groupe alkylidène en C1-C4, $R_1$ l'hydrogène ou un groupe alkyle en C1-C7 et $R_2$ un groupe N,N-di-(alkyle en C1-C4)-carbamyle dans lequel les deux groupes N-alkyle sont identiques ou différents, ou le cas échéant un tautomère et/ou un sel acceptable pour l'usage pharmaceutique d'un tel composé, avec des produits auxiliaires pharmaceutiques usuels.

**40.** Procédé selon la revendication 34, pour préparer une composition pharmaceutique, caractérisé en ce que l'on mélange l'un des composés de formule I décrit dans les revendications 38 ou 39, à l'état libre, avec des produits auxiliaires pharmaceutiques usuels.

**41.** Utilisation d'un composé de formule I, obtenu selon l'une des revendications 22 à 33, ou utilisation de l'un des composés décrits dans l'une des revendications 38 à 40 et répondant à la formule I, ou utilisation d'un composé de formule I selon l'une des revendications 1 à 19 ou le cas échéant d'un tautomère et/ou d'un sel acceptable pour l'usage pharmaceutique, par exemple un médicament anticonvulsif.

**Revendications pour les Etats contractants suivants : ES, GR**

**1.** Procédé de préparation d'un composé de formule

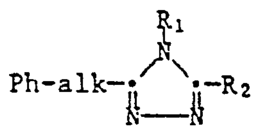

(I),

dans laquelle Ph représente un groupe phényle substitué par des groupes alkyle en C1-C7, des halogènes de numéro atomique allant jusqu'à 35 inclus ou des groupes trifluorométhyle, alk représente un groupe alkylidène en C1-C4, $R_1$ l'hydrogène ou un groupe alkyle en C1-C7 et $R_2$ un groupe carbamyle éventuellement substitué par des groupes alkyle en C1-C7 ou alcanoyle en C1-C7, sous réserve que dans un composé de formule I dans laquelle $R_1$ représente un groupe méthyle, $R_2$ un groupe N,N-diéthylcarbamyle et alk un groupe méthylène, Ph ne peut représenter un groupe phényle substitué par le chlore en position para, ou le cas échéant d'un tautomère et/ou d'un sel d'un tel composé, caractérisé en ce que

a) dans un composé de formule

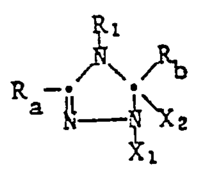

(II),

dans laquelle $X_1$ et $X_2$ représentent des groupes éliminables avec formation d'une liaison supplémentaire, et l'un des symboles $R_a$ et $R_b$ représente un groupe Ph-alk et l'autre un groupe $R_2$, ou le cas échéant dans un tautomère et/ou un sel d'un tel composé, on élimine $X_1$ et $X_2$, ou bien

b) dans un composé de formule

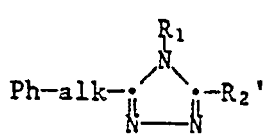

(III),

dans laquelle $R'_2$ représente un groupe convertible en un groupe $R_2$, ou le cas échéant dans un tautomère et/ou un sel d'un tel composé, on convertit $R'_2$ en $R_2$, ou bien,

c) pour préparer un composé de formule I dans laquelle $R_1$ a une signification autre que l'hydrogène

et $R_2$ représente en particulier un groupe N,N-di-(alkyle en C1-C4)-carbamyle, on introduit le groupe de formule Ph-alk dans un composé de formule

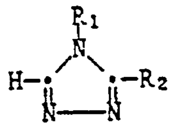

(IV)

ou dans un sel d'un tel composé, ou bien

d) pour préparer un composé de formule I dans laquelle $R_1$ représente l'hydrogène, partant d'un composé de formule

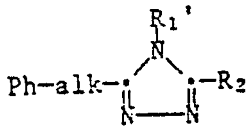

(V),

dans laquelle $R'_1$ représente un groupe remplaçable par l'hydrogène, ou d'un sel d'un tel composé, on remplace $R'_1$ par l'hydrogène, ou bien

e) pour préparer un composé de formule I dans laquelle $R_2$ représente un groupe N,N-di-(alkyle en C1-C4)-carbamyle, partant d'un composé de formule

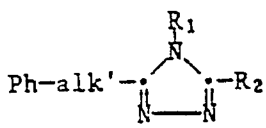

(VI),

dans laquelle alk' représente un groupe convertible en un groupe alk, ou le cas échéant d'un tautomère et/ou d'un sel d'un tel composé, on convertit alk' en alk, et dans chaque cas, on sépare un mélange d'isomères éventuellement obtenu par ce procédé en les composants et on isole l'isomère de formule I, et si on le désire, dans chaque cas, on convertit un composé de formule I obtenu par le procédé ou de toute autre manière, en un autre composé de formule I, et dans chaque cas on sépare un mélange de stéréoisomères éventuellement obtenu par le procédé en les stéréoisomères et on isole le stéréoisomère recherché, et/ou dans chaque cas, on convertit un composé de formule I obtenu dans le procédé à l'état libre en un sel, ou un sel obtenu par le procédé en le composé libre de formule I ou en un autre sel.

**2.** Procédé selon la revendication 1 pour préparer un composé de formule I dans laquelle Ph représente un groupe phényle mono-, di- ou tri-substitué par des groupes alkyle en C1-C7, des halogènes de numéro atomique allant jusqu'à 35 inclus et/ou des groupes trifluorométhyle, alk représente un groupe alkylidène en C1-C4, $R_1$ l'hydrogène ou un groupe alkyle en C1-C7 et $R_2$ représente un groupe carbamyle, N-(alkyle en C1-C7)-carbamyle, N,N-di-(alkyle en C1-C4)-carbamyle ou N-(alcanoyle en C1-C7)-carbamyle, ou le cas échéant un tautomère et/ou un sel d'un tel composé.

**3.** Procédé selon la revendication 1 pour préparer un composé de formule I dans laquelle Ph représente un groupe phényle monosubstitué par un groupe alkyle en C1-C4, un halogène ou un groupe trifluorométhyle ou disubstitué par des halogènes, un halogène et un groupe alkyle en C1-C4 ou un halogène et un groupe trifluorométhyle, les halogènes en question ayant dans chaque cas un numéro atomique allant jusqu'à 35 inclus, alk représente un groupe 1,1- ou 2,2-alkylidène en C1-C4, $R_1$ représente l'hydrogène ou un groupe alkyle en C1-C4 et $R_2$ un groupe carbamyle, N-(alkyle en C1-C4)-carbamyle, N,N-di-(alkyle en C1-C4)-carbamyle ou N-(alcanoyle en C2-C7)-carbamyle, ou le cas échéant un tautomère et/ou un sel d'un tel composé.

**4.** Procédé selon la revendication 1 pour préparer un composé de formule I dans laquelle Ph, alk, $R_1$ et

$R_2$ ont l'une quelconque des significations indiquées dans l'une des revendications 1 à 3 et Ph porte au moins l'un des substituants alkyle et/ou halogéno dans une position ortho ou un substituant trifluorométhyle dans une position méta, ou le cas échéant un tautomère et/ou un sel d'un tel composé.

5. Procédé selon la revendication 1 pour préparer un composé de formule I dans laquelle Ph représente un groupe o-(alkyle en C1-C4)-phényle, m-trifluorométhylphényle, o-halogénophényle ou 2,3-, 2,4-, 2,5- ou 2,6-dihalogénophényle, les halogènes en question ayant dans tous les cas un numéro atomique allant jusqu'à 35 inclus, alk représente un groupe 1,1-alkylidène en C1-C4, $R_1$ l'hydrogène ou un groupe alkyle en C1-C4 et $R_2$ un groupe carbamyle ou N-(alkyle en C1-C4)-carbamyle, N,N-di-(alkyle en C1-C4)-carbamyle ou N-(alcanoyle en C2-C7)-carbamyle, ou le cas échéant un tautomère et/ou un sel d'un tel composé.

6. Procédé selon la revendication 1 pour préparer un composé de formule I dans laquelle Ph, alk, $R_1$ et $R_2$ ont l'une des significations indiquées dans l'une des revendications 1 à 5, sous réserve que Ph ne peut représenter un groupe phényle monosubstitué par un halogène de numéro atomique allant jusqu'à 35 inclus lorsque $R_2$ représente un groupe N,N-di-(alkyle en C1-C4)-carbamyle dans lequel les deux groupes N-alkyle sont identiques ou différents, ou le cas échéant un tautomère et/ou un sel d'un tel composé.

7. Procédé selon la revendication 1 pour préparer un composé de formule I dans laquelle Ph représente un groupe m-trifluorométhylphényle, o-fluorophényle ou 2,6-difluorophényle, alk représente un groupe méthylène, $R_1$ un groupe alkyle en C1-C4 et $R_2$ un groupe carbamyle, ou un sel d'un tel composé.

8. Procédé selon la revendication 1 pour préparer un composé de formule I dans laquelle Ph représente un groupe m-trifluorométhylphényle ou 2,6-difluorophényle, alk représente un groupe méthylène, $R_1$ un groupe alkyle en C1-C4 et $R_2$ un groupe carbamyle, ou un sel d'un tel composé.

9. Procédé selon la revendication 1 pour préparer le 5-(2,6-difluorobenzyl)-4-méthyl-4H-1,2,4-triazole-3-carboxamideou l'un de ses sels.

10. Procédé selon la revendication 1 pour préparer le 5-(o-chlorobenzyl)-4-méthyl-4H-1,2,4-triazole-3-carboxamide, le 4-méthyl-5-(m-trifluorométhylbenzyl)-4H-1,2,4-triazole-3-carboxamide, le 4-éthyl-5-(2,6-difluorobenzyl)-4H-1,2,4-triazole-3-carboxamide ou le 5-(2,6-difluorobenzyl)-4-méthyl-4H-1,2,4-triazole-3-(N-méthyl)-carboxamide ou un sel de l'un de ces composés.

11. Procédé selon la revendication 1 pour préparer le 5-(2,6-difluorobenzyl)-4-méthyl-4H-1,2,4-triazole-3-(N-éthyl)-carboxamide, le 5-(o-fluorobenzyl)-4-méthyl-4H-1,2,4-triazole-3-carboxamide, le 5-(2,6-difluorobenzyl)-4-méthyl-4H-1,2,4-triazole-3-(N,N-diméthyl)-carboxamide, le 5-(o-chlorobenzyl)-1,2,4-triazole-3-carboxamide, le 5-[1(2,6-difluorophényl)-éthyl]-4-méthyl-4H-1,2,4-triazole-3-carboxamide ou le 5-(2,6-difluorobenzyl)-4-méthyl-4H-1,2,4-triazole-3-(N-acétyl)-carboxamide ou un sel de l'un de ces composes.

12. Procédé selon la revendication 1 pour préparer un composé de formule I dans laquelle Ph, alk, $R_1$ et $R_2$ ont l'une des significations indiquées dans l'une des revendications 1 à 8, à l'état libre, ou procédé selon la revendication 1 pour préparer l'un des composés décrit dans l'une des revendications 9 à 11, à l'état libre.

13. Procédé selon la revendication 1 pour préparer un composé de formule I dans laquelle Ph, alk, $R_1$ et $R_2$ ont l'une des significations indiquées dans l'une des revendications 1 à 5, à l'état libre, ou procédé selon la revendication 1 pour préparer l'un des composés décrit dans l'une des revendications 9 à 11, à l'état libre.

14. Procédé de préparation d'une composition pharmaceutique, caractérisé en ce que
a) dans un composé de formule

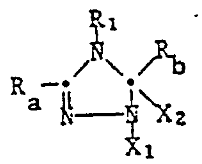 (II),

dans laquelle $X_1$ et $X_2$ représentent des groupes éliminables avec formation d'une liaison supplémentaire, et l'un des symboles $R_a$ et $R_b$ représente un groupe Ph-alk et l'autre un groupe $R_2$, ou le cas échéant dans un tautomère et/ou un sel d'un tel composé, on élimine $X_1$ et $X_2$, ou bien

b) dans un composé de formule

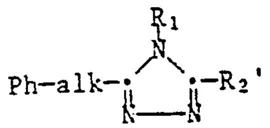 (III),

dans laquelle $R'_2$ représente un groupe convertible en un groupe $R_2$, ou le cas échéant dans un tautomère et/ou un sel d'un tel composé, on convertit $R'_2$ en $R_2$, ou bien

c) pour préparer un composé de formule I dans laquelle $R_1$ a une signification autre que l'hydrogène et $R_2$ représente en particulier un groupe N,N-di-(alkyle en C1-C4)-carbamyle, on introduit le groupe de formule Ph-alk dans un composé de formule

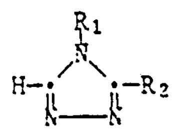 (IV)

ou dans un sel d'un tel composé, ou bien

d) pour préparer un composé de formule I dans laquelle $R_1$ représente l'hydrogène, partant d'un composé de formule

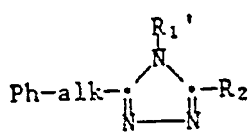 (V),

dans laquelle $R'_1$ représente un groupe remplaçable par l'hydrogène, ou d'un sel d'un tel composé, on remplace $R'_1$ par l'hydrogène, ou bien

e) pour préparer un composé de formule I dans laquelle $R_2$ représente un groupe N,N-di-(alkyle en C1-C4)-carbamyle, partant d'un composé de formule

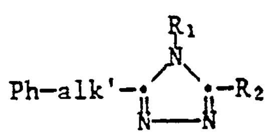 (VI),

dans laquelle alk' représente un groupe convertible en un groupe alk, ou le cas échéant d'un tautomère et/ou d'un sel d'un tel composé, on convertit alk' en alk, et dans chaque cas, on sépare un mélange d'isomères éventuellement obtenu par ce procédé en les composants et on isole l'isomère de formule I, et si on le désire, dans chaque cas, on convertit un composé de formule I obtenu par le procédé ou de toute autre manière, en un autre composé de formule I, et dans chaque

cas, on sépare un mélange de stéréoisomères éventuellement obtenu par le procédé en les stéréoisomères et on isole le stéréoisomère recherché, et/ou dans chaque cas, on convertit un composé de formule I obtenu dans le procédé à l'état libre en un sel, ou un sel obtenu par le procédé en le composé libre de formule I ou en un autre sel, et on mélange dans chaque cas un composé ainsi obtenu répondant à la formule

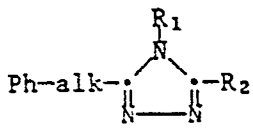

(I),

dans laquelle Ph représente un groupe phényle substitué par des groupes alkyle en C1-C7, des halogènes de numéro atomique allant jusqu'à 35 inclus et/ou des groupes trifluorométhyle, alk représente un groupe alkylidène en C1-C4, $R_1$ l'hydrogène ou un groupe alkyle en C1-C7 et $R_2$ un groupe carbamyle éventuellement substitué par des groupes alkyle en C1-C7 ou alcanoyle en C1-C7, ou le cas échéant un tautomère ainsi obtenu et/ou un sel acceptable pour l'usage pharmaceutique d'un tel composé, ainsi obtenu, avec des produits auxiliaires pharmaceutiques usuels.

**15.** Procédé selon la revendication 14, pour préparer une composition pharmaceutique, caractérisé en ce que l'on mélange un composé obtenu par le procédé décrit dans la revendication 14, répondant à la formule I dans laquelle Ph représente un groupe phényle substitué par des groupes alkyle en C1-C7, des halogènes de numéro atomique allant jusqu'à 35 inclus et/ou des groupes trifluorométhyle, alk représente un groupe alkylidène en C1-C4, $R_1$ l'hydrogène ou un groupe alkyle en C1-C7 et $R_2$ un groupe carbamyle éventuellement substitué par des groupes alkyle en C1-C7 ou alcanoyle en C1-C7, à l'état libre, avec des produits auxiliaires pharmaceutiques usuels.

**16.** Procédé de préparation d'une composition pharmaceutique caractérisé en ce que l'on mélange un composé obtenu selon l'une des revendications 1 à 13 ou le cas échéant un tautomère et/ou un sel acceptable pour l'usage pharmaceutique d'un tel composé, avec des produits auxiliaires pharmaceutiques usuels.

**17.** Procédé selon la revendication 16, pour préparer une composition pharmaceutique, caractérisé en ce que l'on mélange un composé obtenu selon la revendication 13, à l'état libre, avec des produits auxiliaires pharmaceutiques usuels.

**18.** Procédé selon la revendication 14 pour préparer une composition pharmaceutique, caractérisé en ce que l'on mélange un composé obtenu par le procédé décrit dans la revendication 14, répondant à la formule I dans laquelle Ph représente un groupe p-chlorophényle, alk un groupe méthylène, $R_1$ un groupe méthyle et $R_1$ un groupe N,N-diéthylcarbamyle, ou un sel acceptable pour l'usage pharmaceutique d'un tel composé, avec des produits auxiliaires pharmaceutiques usuels.

**19.** Procédé selon la revendication 14 pour préparer une composition pharmaceutique, caractérisé en ce que l'on mélange un composé obtenu par le procédé décrit dans la revendication 14, répondant à la formule I dans laquelle Ph représente un groupe phényle monosubstitué par un halogène de numéro atomique allant jusqu'à 35 inclus, alk représente un groupe alkylidène en C1-C4, $R_1$ l'hydrogène ou un groupe alkyle en C1-C7 et $R_2$ un groupe N,N-di-(alkyle en C1-C4)-carbamyle dans lequel les deux groupes N-alkyle sont identiques ou différents, ou le cas échéant un tautomère et/ou un sel acceptable pour l'usage pharmaceutique d'un tel composé, avec des produits auxiliaires pharmaceutiques usuels.

**20.** Procédé selon la revendication 14 pour préparer une composition pharmaceutique, caractérisé en ce que l'on mélange l'un des composés de formule I décrit dans les revendications 18 ou 19, à l'état libre, avec des produits auxiliaires pharmaceutiques usuels.

**21.** Utilisation d'un composé de formule I obtenu selon l'une des revendications 1 à 13 ou utilisation de l'un des composés décrits dans l'une des revendications 18 à 20 et répondant à la formule I ou le cas échéant d'un tautomère et/ou d'un sel acceptable pour l'usage pharmaceutique d'un tel composé pour préparer une composition pharmaceutique, par exemple un médicament anticonvulsif.